(19) Europäisches Patentamt
European Patent Office
Office européen des brevets

(11) Publication number: **0 135 332**
**B1**

(12) **EUROPEAN PATENT SPECIFICATION**

(45) Date of publication of patent specification: **07.12.88**

(21) Application number: **84305305.9**

(22) Date of filing: **03.08.84**

(51) Int. Cl.[4]: **C 07 D 401/12,**
C 07 D 403/12,
C 07 D 413/12,
C 07 D 417/12,
C 07 D 491/048,
C 07 D 491/052,
C 07 D 251/14,
C 07 D 249/14,
C 07 D 239/42, A 01 N 47/36

(54) **Herbicidal benzenesulfonamides, benzylsulfonamides and benzenesulfamates.**

(30) Priority: **05.08.83 US 520801**
**12.07.84 US 628259**
**19.09.83 US 533771**
**13.07.84 US 628939**
**08.12.83 US 559372**
**29.06.84 US 624843**

(43) Date of publication of application:
**27.03.85 Bulletin 85/13**

(45) Publication of the grant of the patent:
**07.12.88 Bulletin 88/49**

(84) Designated Contracting States:
**AT BE CH DE FR GB IT LI LU NL SE**

(56) References cited:
**EP-A-0 007 687**
**EP-A-0 023 141**
**EP-A-0 035 893**
**EP-A-0 044 807**
**EP-A-0 073 627**
**EP-A-0 083 975**
**EP-A-0 085 236**
**EP-A-0 111 442**
**US-A-4 369 058**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Hanagan, Mary Ann**
**17 Barclay Court**
**Blue Bell, PA 19422 (US)**
Inventor: **Hay, James Volney**
**36 Stature Drive**
**Newark, DE 19713 (US)**
Inventor: **Tseng, Chi Ping**
**1103 Artwin Road**
**Wilmington, DE 19803 (US)**

(74) Representative: **Hildyard, Edward Martin et al**
**Frank B. Dehn & Co. Imperial House**
**15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 135 332 B1**

**Description**

Background of the Invention

This invention relates to novel 2-sulfamoyl benzenesulfonamides and novel 2,6-disubstituted benzenesulfonamides, agriculturally suitable compositions thereof and a method of their use as preemergent or postemergent herbicides or plant growth regulants.

Herbicidal sulfonamides of the type described herein are generally referred to in the art as "sulfonylurea" herbicides.

The "sulfonylurea" herbicides are an extremely potent class of herbicides discovered within the last few years. This class of herbicides generally consists of a sulfonylurea bridge, $—SO_2NHCOHN—$, linking two aromatic or heteroaromatic rings.

New compounds effective for controlling the growth of undesired vegetation are in constant demand. In the most common situation, such compounds are sought to selectively control the growth of undesired vegetation to permit the growth of useful crops such as cotton, rice, corn, wheat and soybeans, to name a few. Unchecked weed growth in such useful crops can cause significant losses, reducing profit to the farmer and increasing costs to the consumer. In other situations, herbicides are desired which will control all plant growth. Examples of areas in which complete control of all vegetation is desired are areas around fuel storage tanks, ammunition depots and industrial storage areas. There are many products commercially available for these purposes, but the search continues for products which are more effective, less costly and environmentally safe.

EP—A—7,767, discloses herbicidal sulfonylureas bearing *ortho*-carboxylic acid ester groups.

EP—A—44,211, discloses herbicidal sulfonylureas bearing *ortho*-phenyl groups.

EP—A—44,209, discloses herbicidal sulfonylureas bearing $—CH_2OR$ and $CH_2CO_2R_8$ *ortho*-groups.

EP—A—35,893, discloses herbicidal sulfonylureas bearing an *ortho*-sulfonyl group.

EP—A—44,212, discloses herbicidal sulfonylureas bearing *ortho*-sulfonate groups.

U.S. 4,369,058, issued January 18, 1983 discloses herbicidal sulfonylureas bearing *ortho*-amino substituents.

EP—A—83,975, discloses herbicidal sulfonylureas bearing *ortho*-heterocyclic substituents.

EP—A—85,476, discloses herbicidal sulfonylureas bearing *ortho*-pyridyl, pyrimidyl, triazinyl and furanyl groups.

South African Patent Application 83/0127 discloses herbicidal N-arylsulfonyl-N'-pyrimidinylureas of formula

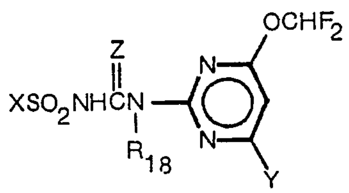

where

X is optionally substituted phenyl or naphthyl.

South African Patent Application 83/0441 discloses herbicidal ureas of formula

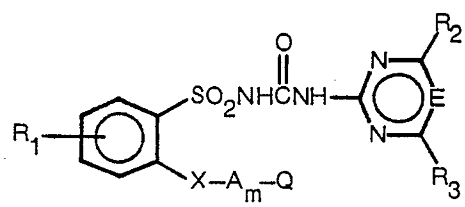

where

X is O, S, SO or $SO_2$;

m is 0 or 1; and

Q is any of a wide variety of moities.

South African Patent Application 83/8416 discloses herbicidal sulfonamides of formula

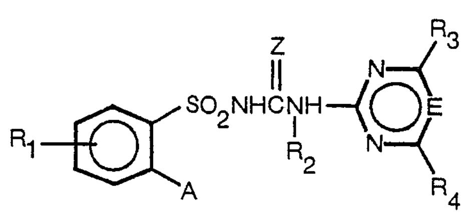

where

A is an unsaturated or only partially saturated 5- or 6-membered heterocyclic ring system which is

bonded through a carbon atom and contains 1, 2 or 3 heteroatoms and which may be substituted; and

$R_1$ is H, halo, $NO_2$, $C_1$—$C_4$ alkyl, $C_1$—$C_4$ haloalkyl, $C_1$—$C_4$ alkoxy, $C_1$—$C_4$ haloalkoxy, $C_1$—$C_4$ alkoxycarbonyl, $C_1$—$C_4$ alkythio, $C_1$—$C_4$ alkylsulfinyl, $C_1$—$C_4$ alkylsulfonyl or $C_2$—$C_5$ alkoxyalkoxy.

U.S. Patent 4,310,346 issued January 12, 1982 to Levitt et al discloses herbicidal sulfonylurea compounds of the following structure.

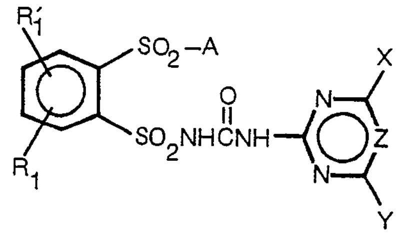

wherein

among other substituents, A can be $NR_2R_3$; R can be H or $C_1$—$C_6$ alkyl;

$R_3$ can be $C_1$—$C_4$ alkyl; and $NR_2R_3$ taken together can be

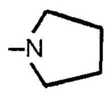 or 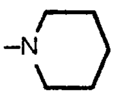

Other specifications disclosing herbicidal sulfonylurea compounds include: EP—A—30,433, EP—A—46,677, EP—A—101,407, US—A—4,302,241 and EP—A—73,562.

Moreover the following Specifications, also disclosing herbicidal sulfonylurea compounds, were published after the relevant priority date of this Application: EP—A—101,308, US—A—4,515,624, EP—A—103,537, EP—A—125,205, EP—A—101,670, EP—A—107,624, EP—A—111,442 and EP—A—94,260.

## Summary of the Invention

Novel compounds, suitable agricultural compositions thereof and a method for their use as preemergent or postemergent herbicides or plant growth regulants have been found. The novel compounds of this invention are of the formula

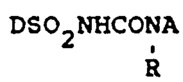

I

wherein

D is 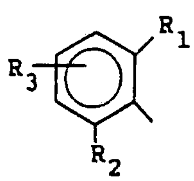 or 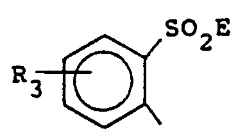 ;

D-1 D-4

R is H or $CH_3$;

$R_1$ is $S(O)_nR_8$, $CF_3$, $NR_9R_{10}$, $CO_2R_{11}$,

$SO_2NR_{12}R_{13}$, 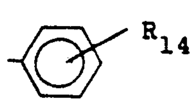 , 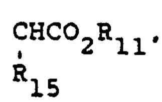,

$OSO_2R_{16}$, $CH_2OR_{17}$ or Q;

$R_2$ is $S(O)_mR_8$, $CF_3$, $NR_9R_{10}$, $CO_2R_{11}$,

$SO_2NR_{12}R_{13}$, 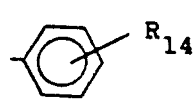 , 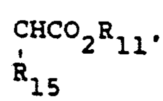,

$OSO_2R_{16}$, $CH_2OR_{17}$ or Q;

$R_3$ is H, Cl, F, Br, $CH_3$, $OCH_3$ or $CF_3$;
E is

$NR_{22}R_{23}$, 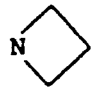, 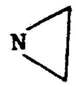 or 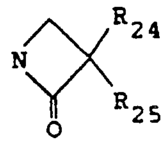;

$R_8$ is $C_1$—$C_3$ alkyl;
$R_9$ and $R_{10}$ are independently $C_1$—$C_2$ alkyl;
$R_{11}$ is $C_1$—$C_3$ alkyl, $CH_2CH{=}CH_2$, $CH_2CH_2Cl$ or $CH_2CH_2OCH_3$;
$R_{12}$ is $CH_3$ or $OCH_3$;
$R_{13}$ is $C_1$—$C_3$ alkyl;
$R_{14}$ is H, Cl, Br, F, $CH_3$ or $OCH_3$;
$R_{15}$ is H or $CH_3$;
$R_{16}$ is $C_1$—$C_3$ alkyl or $CF_3$;
$R_{17}$ is $C_1$—$C_3$ alkyl or $CF_2H$;
n is 0, 1 or 2;
m is 0 or 1;
Q is

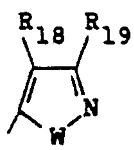,

Q-1

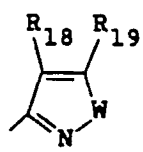,

Q-2

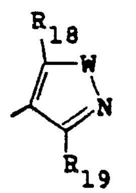,

Q-3

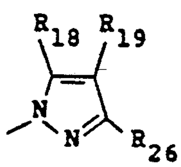,

Q-4

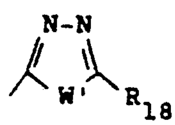,

Q-5

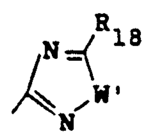,

Q-6

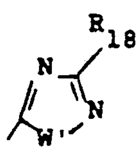,

Q-7

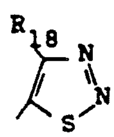,

Q-8

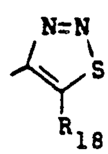,

Q-9

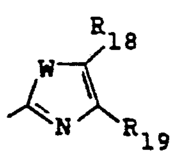,

Q-10

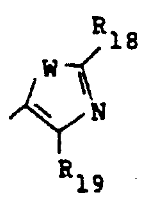,

Q-11

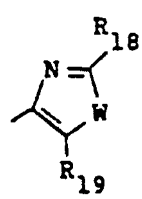,

Q-12

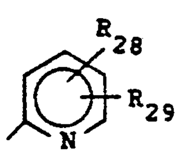,

Q-13

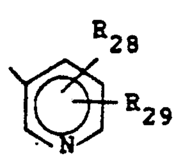,

Q-14

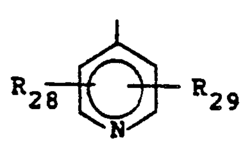,

Q-15

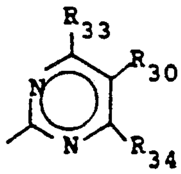 , 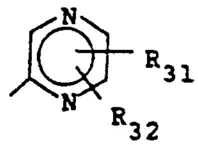 , 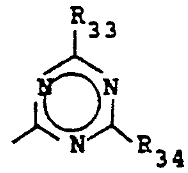 ,

Q-16 Q-17 Q-18

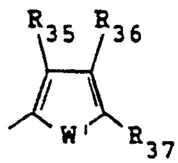 or 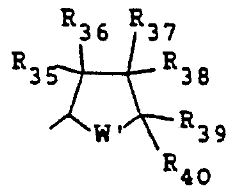 ;

Q-19 Q-20

W is O, S or $NR_{27}$;
W' is O or S;
$R_{18}$, $R_{19}$, $R_{24}$, $R_{25}$, $R_{26}$, $R_{27}$, $R_{28}$, $R_{29}$, $R_{30}$, $R_{31}$, $R_{32}$, $R_{35}$, $R_{36}$, $R_{37}$, $R_{38}$, $R_{39}$ and $R_{40}$ are independently H or $CH_3$;
$R_{33}$ and $R_{34}$ are independently H, $CH_3$ or $OCH_3$;
$R_{22}$ is $C(O)R_{41}$, $C(O)NR_{42}R_{43}$, $CO_2R_{44}$, $C(O)NHR_{45}$ or $CF_2H$;
$R_{23}$ is H or $C_1$—$C_3$ alkyl;
$R_{41}$ is $CF_3$ or phenyl optionally substituted with Cl, $CH_3$, $CF_3$, $NO_2$ or $OCH_3$;
$R_{42}$ is H, $C_1$—$C_4$ alkyl or phenyl optionally substituted with Cl, $CH_3$, $CF_3$, $NO_2$ or $OCH_3$;
$R_{43}$ is H or $C_1$—$C_4$ alkyl;
$R_{42}$ and $R_{43}$ may be taken together to be $—(CH_2)_4—$, $—(CH_2)_5—$ or $—CH_2CH_2OCH_2CH_2—$;
$R_{44}$ is $C_1$—$C_4$ alkyl;
$R_{45}$ is A—1;
A is

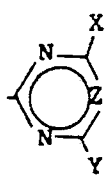 , 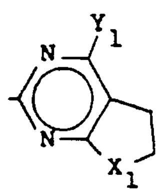 , 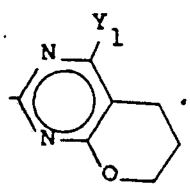 ,

A-1 A-2 A-3

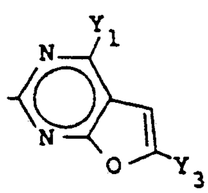 , 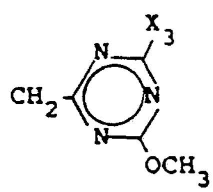 or 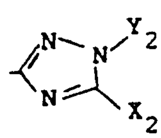 ;

A-4 A-5 A-6

X is $CH_3$, $OCH_3$, $OCF_2H$ or Cl;
Y is $CH_3$, $C_2H_5$, $OCH_3$, $OC_2H_5$, $CH_2OCH_3$, $OCH_2CF_3$, $OCF_2H$, $NHCH_3$, $N(CH_3)_2$ or $SCH_3$;
$X_1$ is $CH_2$ or O;
$Y_1$ is $CH_3$ or $OCH_3$;
$X_2$ is $CH_3$, $OCH_3$ or $SCH_3$;
$Y_2$ is $CH_3$, $C_2H_5$ or $CH_2CF_3$;
$X_3$ is $CH_3$ or $OCH_3$; and
$Y_3$ is H or $CH_3$;
Z is CH or N;
and their agriculturally suitable salts;
provided that

a) when either one of $R_1$ or $R_2$ is $CF_3$, then the other is also $CF_3$, X is other than Cl when Y is $NHCH_3$; and X and Y are other than $OCF_2H$;

b) $R_1$ and $R_2$ are not simultaneously $CO_2R_{11}$;

c) when either one of $R_1$ or $R_2$ is $SO_2NR_{12}R_{13}$, then the other must not be $S(O)_nR_8$, $S(O)_mR_8$, $CH_2OR_{17}$ or $NR_9R_{10}$;

d) when $R_{12}$ is $OCH_3$, then $R_{13}$ is $CH_3$;

e) when either one of $R_1$ or $R_2$ is $CO_2R_{11}$, then the other must not be $S(O)_nR_8$, $S(O)_mR_8$ or $NR_9R_{10}$;

f) the total number of carbon atoms in $R_{35}$ to $R_{40}$ combined, is equal to or less than four;

g) when X is Cl, then Z is CH and Y is $NHCH_3$, $N(CH_3)_2$, $OCH_3$ or $OC_2H_5$;

h) when $R_1$ is $CO_2R_{11}$ and $R_2$ is $OSO_2R_{16}$, then A is not A—1;

j) when $R_3$ is H, A is A—1 and either one of $R_1$ or $R_2$ is Q and Q is not Q—4 or Q—20; then the other of $R_1$ or $R_2$ must not be $S(O)_nR_8$, $S(O)_mR_8$, $CF_3$ or $CO_2R_{11}$;

k) when A is A—6, then $R_1$ is

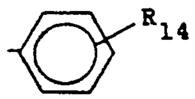, $\underset{R_{15}}{CHCO_2R_{11}}$, $CH_2OR_{17}$ or Q;

l) when $R_{22}$ is $C(O)NHR_{45}$, then A must be A—1 and the values of X, Y and Z must be identical for both A and $R_{45}$;

m) when D is D—4, then R is H; and

n) when $R_1$ and $R_2$ are $CF_3$, then A is other than A—5.

The preferred compounds of the invention for their higher herbicidal activity, greater plant growth regulant activity and/or more favorable ease of synthesis are:

1) Compounds of Formula I where A is A—1; R is H; $R_1$ and $R_2$ are not simultaneously Q; D is D—1, or D—4; and when D is other than D—1 then $R_3$ is H.

2) Compounds of Preferred 1 where D is D—1 and Y is $CH_3$, $CH_2OCH_3$, $OCH_3$, $OCH_2CF_3$ or $OCF_2H$.

3) Compounds of Preferred 2 where $R_3$ is in either the 3- or 5-position.

4) Compounds of Preferred 3 where $R_1$ and $R_2$ are not simultaneously $SO_2NR_{12}R_{13}$ or $NR_9R_{10}$.

5) Compounds of Preferred 4 where $R_3$ is H and at least one of $R_1$ or $R_2$ is

$S(O)_nR_8$, $S(O)_mR_8$, $CF_3$, $NR_9R_{10}$, $SO_2NR_{12}R_{13}$, 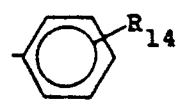, $OSO_2R_{16}$ or $CH_2OR_{17}$.

6) Compounds of Preferred 5 where $R_8$ is $CH_3$ or $C_2H_5$; $R_9$ and $R_{10}$ are both $CH_3$; $R_{12}$ is $CH_3$; $R_{14}$ is H; $R_{16}$ is $CH_3$ or $C_2H_5$; and $R_{17}$ is $CH_3$.

7) Compounds of Preferred 6 where X is $CH_3$, $OCH_3$ or Cl and Y is $CH_3$, $OCH_3$ or $CH_2OCH_3$.

8) Compounds of Preferred 1 where D is D—4 and Y is $CH_3$, $C_2H_5$, $OCH_3$, $OC_2H_5$ or $CH_2OCH_3$.

9) Compounds of Preferred 8 where E is

$NR_{22}R_{23}$ or 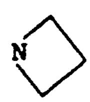 ;

and $R_{23}$ is H or $CH_3$.

10) Compounds of Preferred 9 where E is

$NR_{22}R_{23}$ or 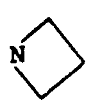 ;

and X is $CH_3$, $OCH_3$ or Cl.

The most preferred compounds of the invention for their highest herbicidal activity, greatest plant growth regulating activity and/or more favorable ease of synthesis are:

N-[(4,6-dimethoxypyrimidin-2-yl)aminocarbonyl]-3-methylsulfonyl-1,1'-biphenyl-2-sulfonamide;

N-[(4,6-dimethoxy-1,3,5-triazin-2-yl)aminocarbonyl]-2,6-bis(methylthio)benzenesulfonamide;

2-(azetidin-1-ylsulfonyl)-N-[(4,6-dimethoxy-1,3,5-triazin-2-yl)aminocarbonyl]benzenesulfonamide;

2-(azetidin-1-ylsulfonyl)-N-[(4-methoxy-6-methylpyrimidin-2-yl)aminocarbonyl]benzenesulfonamide;

N,N'-bis-[(4,6-dimethoxypyrimidin-2-yl)aminocarbonyl]-1,2-benzenedisulfonamide; and

2-(azetidin-1-ylsulfonyl)-N-[(4-methoxy-6-methyl-1,3,5-triazin-2-yl)aminocarbonyl]benzenesulfonamide.

## Detailed Description of the Invention

### Synthesis

The compounds of Formula (Ia) where D is D—1, or D—4 may be prepared by one or more of the methods described below in Equations 1 to 4. The method of choice depends on the acid or base lability of the substituents and would be obvious to one skilled in the art.

As shown in Equation 1 below, compounds of Formula (Ia) can be prepared by the reaction of an appropriately substituted sulfonamide of Formula (II) with the appropriate methyl carbamate of Formula (III) in the presence of an equimolar amount of trimethylaluminum. The reactions are best carried out at 25° to 85° in an inert solvent such as methylene chloride or 1,2-dichloroethane under an inert atmosphere.

Equation 1

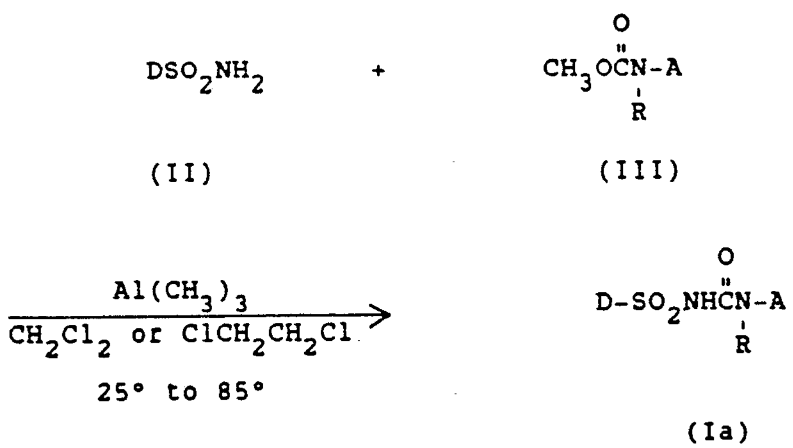


(II) (III) (Ia)

where

D is D–1 or D–4;

$R_1$ and $R_2$ are not $CO_2R_{11}$; and R is H.

Compounds of Formula (Ia) can also be prepared by reacting a sulfonylcarbamate of Formula (IV) with an appropriate amine of Formula (V) as shown in Equation 2. The reaction is carried out at 50° to 100° in a solvent such as dioxane for 0.5 to 24 hours as taught in EP—A—44807, published January 27, 1982. The required carbamates are prepared by reacting the preformed sodium salt of II with diphenylcarbonate.

Equation 2

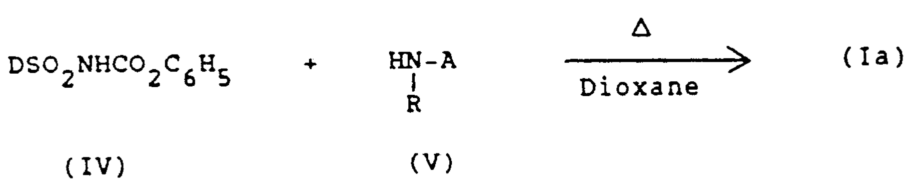


(IV) (V)

wherein

D is D–1 or D–4;

$R_1$ or $R_2$ are not $CO_2R_{11}$ or $OSO_2R_{16}$;

and

R is H or $CH_3$.

Some of the compounds of Formula (Ia) can also be prepared as shown in Equations 3a and 3b.

Equation 3a

$$\text{(II)} \quad + \quad C_6H_5O\overset{O}{\overset{\|}{C}}NHA \quad \xrightarrow[H^+]{DBU} \quad \text{(Ia)}$$

$$\text{(VI)}$$

where

D is D—1 or D—4.

The reaction of Equation 3a can be carried out by contacting equimolar amounts of a sulfonamide of Formula (II) with a heterocyclic phenyl carbamate of Formula (VI) in the presence of an equimolar amount of 1,8-diazabicyclo[5.4.0]undec-7-ene (DBU), by methods analogous to those described in South African Patent Application 830441. The phenyl carbamates of Formula (VI) can be prepared by methods, or modifications thereof known to those skilled in the art, described in South African Patent Application 825671 and South African Patent Application 825045.

Rarely a sulfonamide (II) may not be of sufficient stability to be useful as a starting material in Equations 1—3a. In this case, as well as others, the sulfonyl isocyanate (VIII), can be made as an unisolated intermediate by treating the corresponding sulfonyl chloride (VII), where D is D—1, or D—4, with isocyanate anion in the presence of the heterocyclic amine (V). The amine reacts with the sulfonyl isocyanate as it is formed to give the desired compound of Formula (Ia).

Equation 3b

$$DSO_2Cl \quad + \quad NCO^{\ominus} \quad + \quad \text{(V)} \quad \longrightarrow \quad \text{(Ia)}$$

$$\text{(VII)}$$

where

D is D—1 or D—4.

The reaction is best carried out by adding over one to six hours a solution of at least one equivalent of a tetraalkylammonium isocyanate, such as tetra-*n*-butylammonium isocyanate, in a suitable aprotic organic solvent, such as dichloromethane or tetrahydrofuran, to a well-stirred mixture of one equivalent of sulfonyl chloride (VII) and at least one equivalent of heterocyclic amine (V) in a similar suitable organic solvent at 20—40°C. The reaction mixture is then diluted with dichloromethane, washed with 1 *N* sulfuric acid, and dried over sodium sulfate. Rotary evaporation of the solvent leaves the product of Formula (Ia).

The compounds of Formula (I) may be prepared as shown below in Equation 4 by the reaction of an appropriate benzenesulfonyl isocyanate (VIII) with an appropriate aminoheterocycle (V).

Equation 4

$$DSO_2NCO \quad + \quad \underset{\underset{R}{|}}{HN}\text{-}A \quad \longrightarrow \quad \text{(I)}$$

$$\text{(VIII)} \qquad\qquad \text{(V)}$$

The reaction of Equation 4 is best carried out in an inert aprotic solvent such as methylene chloride, tetrahydrofuran or acetonitrile at a temperature between 20 and 80°. A catalytic amount of 1,4-diazabicyclo[2,2,2]octane (DABCO) may be used to accelerate the reaction. In cases in which the products are insoluble in the reaction solvent, they may be isolated by simple filtration. When the products are soluble, they may be isolated by evaporation of the solvent and trituration of the residue with solvents such as 1-chlorobutane, diethyl ether or methanol and filtration. Impure products may be purified by column chromatography on silica gel.

The benzenesulfonyl isocyanates of Formula (VIIIa), where D is D—1 or D—4, may be prepared as shown in Equation 5, by phosgenation of the sulfonamides (II) in the presence of butyl isocyanate.

Equation 5

$$(II) \xrightarrow[COCl_2/xylene]{n\text{-}C_4H_9NCO} (VIIIa)$$

where

D is D—1 or D—4.

The above reaction is carried out by heating a mixture of the appropriate sulfonamide (II), an alkyl isocyanate such as butyl isocyanate and a catalytic amount of a tertiary amine such as 1,4-diazabicyclo[2,2,2]octane (DABCO) in xylene, or other inert solvent of boiling point ≥135°, to approximately 135°. Phosgene is then added to the mixture over a 1—6 hour period at 125—135° until an excess of phosgene is present as indicated by a permanent drop in the boiling point to less than 130°. The mixture is cooled and filtered to remove a small amount of insoluble by-products. The solvent and the alkyl isocyanate are distilled off *in vacuo* leaving a residue of the crude, sulfonyl isocyanate (VIIIa) which can be used without further purification. This method is described in U.S. 4,238,621.

Alternatively, the sulfonyl isocyanates (VIIIa) can be prepared, as shown in Equation 6, in a two-step procedure. This method is similar to a procedure taught by Ulrich and Sayigh. *New Methods of Preparative Organic Chemistry,* Vol. VI, p. 223—241, Academic Press, New York and London, W. Forest Ed.

Equation 6

$$(II) \xrightarrow[K_2CO_3/MEK]{n\text{-}C_4H_9NCO} DSO_2NH\overset{O}{\overset{\|}{C}}NH\text{-}n\text{-}C_4H_9$$

(IX)

$$(IX) \xrightarrow[toluene,\ \Delta]{COCl_2/DABCO} (VIIIa)$$

wherein D is D—1 or D—4.

The compounds of Formula (IX) are conveniently prepared by stirring a mixture of sulfonamides (II) anhydrous potassium carbonate, and *n*-butyl isocyanate in a solvent such as acetone or methyl ethyl ketone at 25—80° until all of the isocyanate has reacted. The products are isolated by quenching in dilute mineral acid and recrystallizing the solid product. The compounds (IX) are treated with phosgene and a catalytic amount of DABCO in refluxing toluene or a similar solvent in a manner analogous to that described in Equation 5.

Sulfonyl isocyanates of Formula (VIIIa) may also be prepared by the procedure shown below in Equation 7 starting from the appropriate sulfonamides.

Equation 7

$$(II) \xrightarrow[\text{reflux}]{SOCl_2} DSO_2NSO$$

10-50 hours

(X)

$$(X) \xrightarrow[\text{pyridine (cat.)}]{COCl_2} (VIIIa)$$

toluene, Δ

wherein D is D—1 or D—4.

The reactions of Equation 7 are best carried out according to the procedure of Ulrich et al., in *J. Org. Chem., 34,* 3200 (1969). The sulfonamide is boiled under reflux with an excess of thionyl chloride which functions as both a reactant and solvent. When the sulfonamide protons are no longer detectable by proton NMR (15—20 hrs. on the average), the thionyl chloride is removed under reduced pressure and the residue is dissolved in an inert solvent such as toluene, benzene, xylenes. A catalytic amount of pyridine is added. The mixture is treated with at least one equivalent of phosgene and heated to 60°—140° with 80°—100°C preferred. Conversion to the isocyanate is substantially complete within about ¼ to 3 hours. The mixture containing the sulfonyl isocyanate can be used directly or the sulfonyl isocyanate can be isolated in pure form by filtration and evaporation of the filtrate followed by vacuum distillation if necessary.

As shown in Equation 9 below, intermediate sulfonamides for Formula (IIa) where D is D—1 can be prepared from *t*-butyl sulfonamides (XII). Of necessity, the reactions are limited to those cases in which $R_1$ is inert to lithium reagents under the conditions employed. During metallation, esters can be protected as amides and alcohols protected as tetrahydrofuran ethers. For a general review of metallation with lithium reagents, see H. W. Gschwend and R. Rodriguez, *Org. Reactions, 26,* 1 (1979).

Equation 9

a)

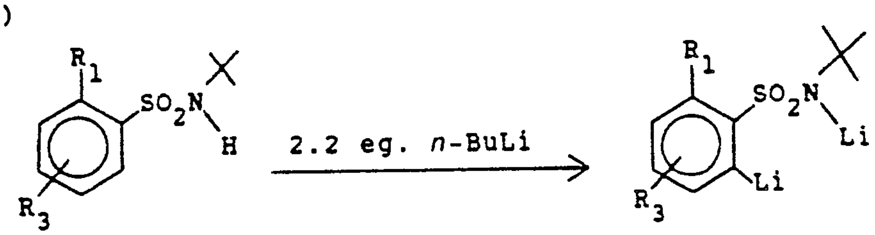


(XII) (XIIa)

where $R_1$ is not $CH_2CO_2R_{11}$, $CO_2R_{11}$, $OSO_2R_{16}$ or $CH_2OR_{17}$;

b)

(XIIa)

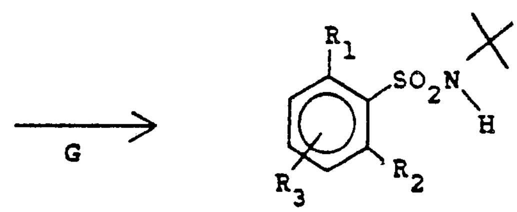


(XIIIa)

where $R_1$ is as defined above;

G is $R_8SSR_8$, $CO_2$, $ClC(O)OR_{11}$, $R_{11}OC(O)OR_{11}$, $CH_2O$, $CH_3I$, or $CH_3CH_2I$; and

$R_2$ is $SR_{11}$, $CO_2H$, $CO_2R_{11}$, $CH_2OH$, $CH_3$ or $CH_2CH_3$.

c)

(XIIa)

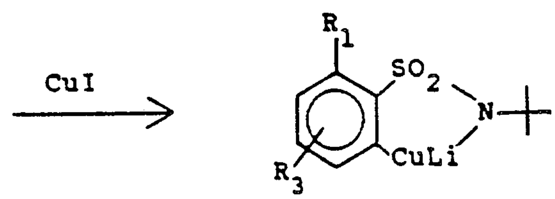


(XIIb)

where $R_1$ is as defined above.

d)

(XIIb) 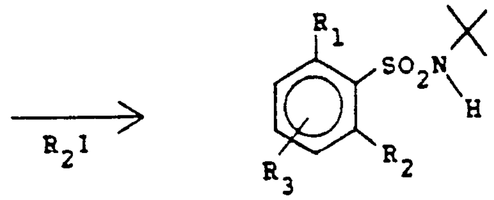


(XIIIb)

where $R_1$ is as defined above; and

$R_2$ is $C_6H_4R_{14}$ or Q.

e)

(XIII) TFA $DSO_2NH_2$

(IIa)

where D is D-1;

$R_1$ is $SR_8$, $CF_3$, $NR_9R_{10}$, $CO_2N(CH_3)_2$, $SO_2NR_{12}R_{13}$, $C_6H_4R_{14}$, OH, $CH_2OH$, Q or $\underset{R_{15}}{CHCO_2R_{11}}$; and

$R_2$ is $SR_8$, $CO_2H$, $CO_2R_{11}$ or $CH_2OH$.

Reaction step (9a) is best carried out under nitrogen in an inert anhydrous aprotic solvent such as tetrahydrofuran, diethyl ether or hexane at temperatures between −78 and 40°C in a manner similar to that of J. G. Lombardino [*J. Org. Chem., 36,* 1843 (1971)].

The preferred mode of addition is to add at least two equivalents of the alkyllithium or phenyllithium reagent to a −45° solution of the sulfonamide (XII) in tetrahydrofuran. After 15 minutes to 3 hours at room temperature, the lithium salt XIIa is obtained.

In Equation 9b, the reaction mixture is cooled to between 0 to −78° and a disulfide, carbon dioxide, chloroformate, dialkyl carbonate, formaldehyde, methyl iodide or ethyl iodide added to the lithium salt. The mixture is then allowed to warm and is stirred at ambient to reflux temperature for 6 to 20 hours. The addition of dilute acetic acid or ammonium chloride removes inorganic salts from the product contained in the organic phase to provide sulfonamides (XIIIa).

In reaction 9c, XIIa is reacted with cuprous iodide at temperatures below 0°C, presumably forming XIIb. After 10 minutes to 1 hour, the aryl iodide or heterocyclic iodide is added and the mixture heated at reflux for 12 to 24 hours. After quenching the reaction with a dilute acid such as hydrochloric acid, ammonium hydroxide can be added in the presence of air to facilitate removal of the copper salts. The product may be extracted with solvents such as ethyl acetate or ether. The residue may be purified by chromatography or triturated using solvents such as hexane/chloroform or hexane/ether.

Several studies on the competition of substituents for ortho-direction capabilities indicates that for substituted sulfonamides, lithiation is directed to the position adjacent to the sulfonamide group:

D. W. Slocum and C. A. Jennings *J. Org. Chem., 41,* 3653 (1976);

A. I. Meyers and K. Lutomski *J. Org. Chem., 44,* 4464 (1979);

P. Beak and R. A. Brown *J. Org. Chem., 47,* 34 (1982); and

P. Beak and V. Snieckus *Acc. Chem. Res., 15,* 306 (1982).

For example, the metallation of (XIIc) was found to occur in the 6-position. The absence of metallation at the 3 position or on the thiomethyl group attests to the powerful directing influence of the sulfonamide group and the thermodynamic stability of the resulting anion.

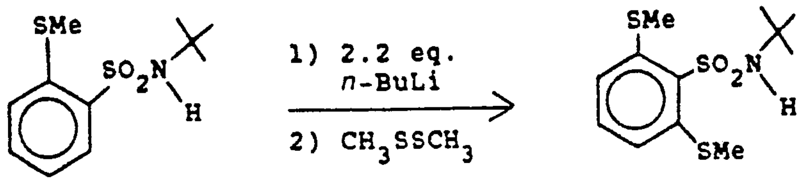


(XIIc)

Reaction step (9e) is best carried out in trifluoroacetic acid or ethanolic hydrogen chloride between ambient and reflux temperatures for 18 to 48 hours according to the method of J. G. Lombardino *[J. Org. Chem., 36,* 1843 (1971)].

The deprotection appears to be concentration dependent. The yield of sulfonamide (IIa) can usually be increased by using a large excess of the acid as a solvent or by stripping off the by-products after 18 hours and redissolving or resuspending the residue in the acid of choice and stirring an additional 20 to 30 hours. The solvent is distilled off in vacuo and the residue can be triturated and recrystallized with solvents such as 1-chlorobutane or chloroform/hexane. The products can also be purified by chromatography.

Some sulfonamides (XIIIc) are best prepared from (XIId) in a "one pot" sequence, an example of which is shown in Equation 9f.

Equation 9f

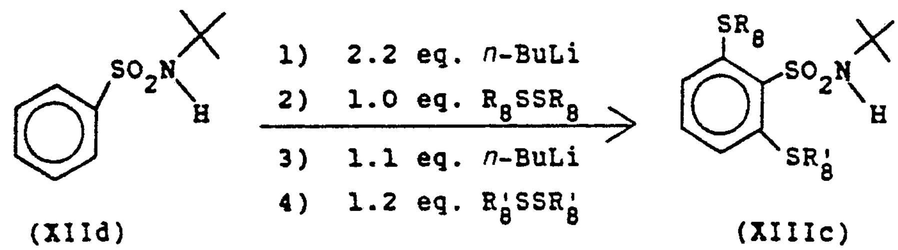


(XIId) (XIIIc)

where

$R_8$ and $R_8'$ are independently $C_1$-$C_3$ alkyl.

This reaction is best carried out in an inert atmosphere in an anhydrous aprotic solvent such as tetrahydrofuran, dimethylethylether or diethyl ether at temperatures between −78 and 40°C in a manner similar to that described for Equation 9a.

The method of choice is to treat a −45°C solution of sulfonamide (XIId) in tetrahydrofuran sequentially with 1) 2.2 equivalents of alkyllithium at −45°C followed by warming to room temperature and stirring for 2 hours, 2) 1.0 equivalent of dialkyl disulfide at 0°C followed by warming to ambient temperature and stirring for 2 hours, 3) 1.1 equivalent of alkyllithium at −45°C followed by warming to room temperature and stirring for 2 hours, and 4) 1.2 equivalents of dialkyl disulfide at 0°C followed by stirring at ambient temperature for 18 hours.

The sulfonamides of Formula (XIIIe) can be prepared from compounds of Formula (XIIId) as shown in Equation 9g.

Equation 9g

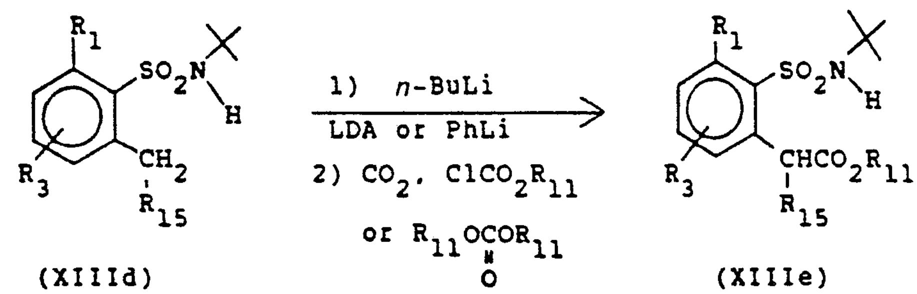


(XIIId) (XIIIe)

wherein

$R_1$ is as described in Equation 9a;

$R_{15}$ is H or $CH_3$; and

$R_{11}$ is H, $CH_3$, $CH_2CH_3$ or $CH_2CH_2CH_3$.

The above reaction is best carried out at −45 to 0° under conditions described for Equations 9a and 9b. The lithiation of *o*-toluenesulfonamides and their subsequent reactions is described by H. Watanabe and C. R. Hauser [*J. Org. Chem., 33,* 4278 (1968)].

The sulfonamides of Formula (XIIId) can be prepared as described in Equation 9b.

Many sulfonamides of Formula (XII) can be prepared from the corresponding sulfonyl chlorides in a manner similar to that taught by J. G. Lombardino [*J. Org. Chem.*, *36*, 1843 (1971)] as shown below.

Equation 9h

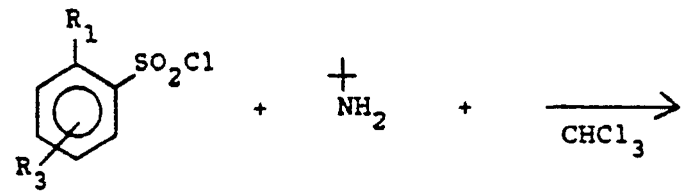


(XII)

(XIV)

wherein

$R_1$ and $R_3$ are as previously defined.

The reaction of Equation 9h is accomplished by treating a 0°C solution of at least two equivalents of *t*-butyl amine in a solvent such as chloroform with the sulfonyl chloride (XIV). The temperature is kept at 0 to 15°C for 1 hour then raised to 50 to 80°C and held at that temperature for 1 to 3 hours. This mixture is then poured into cold acid such as hydrochloric acid and extracted into a solvent such as methylene chloride or ethyl ether. Products are isolated by evaporation of the solvent and trituration of the solid residues with solvents such as hexane, chloroform or 1-chlorobutane.

The intermediate sulfonamides of Formula (IId) where D is D—4 and $R_{23}$ is H, can be prepared by reacting a 1,2-benzenedisulfonylazide of Formula (XXII) with a reducing agent such as sodium borohydride as shown in Equation 10.

Equation 10

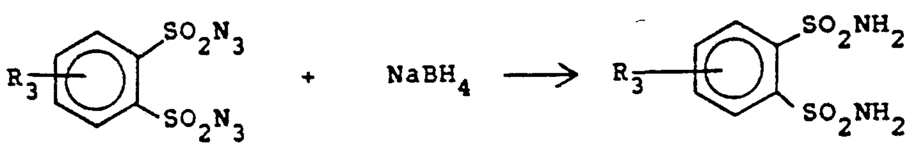


(XXII) (IId)

The reaction is carried out at −20 to 70°C in a solvent such as ethanol for 10 to 96 hours.

The required 1,2-benzenedisulfonylazide (XXII) can be prepared by reacting a *o*-fluorosulfonylbenzenesulfonyl chloride of Formula (XXIII) with sodium azide as shown in Equation 11.

Equation 11

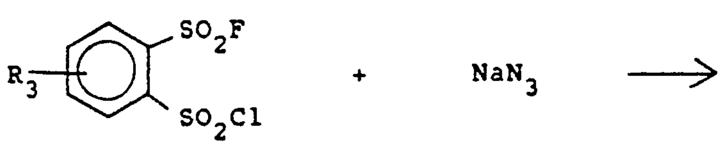


(XXII)

(XXIII)

The reaction is carried out at 0° to 60°C in a solvent such as water or methanol for 10 to 96 hours.

Alternatively, compounds of Formula (XXII) can be prepared by reacting a 1,2-benzenedisulfonyl chloride of Formula (XXIV) with sodium azide as shown in Equation 12.

Equation 12

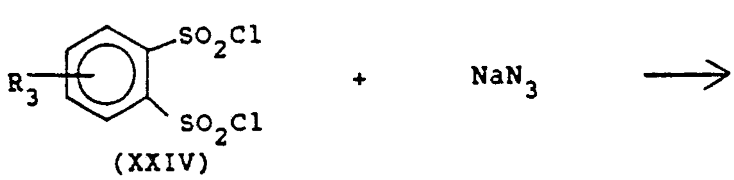


(XXII)

(XXIV)

The reaction is carried out at 0°C to 50°C in a solvent such as methanol or water for 0.2 to 96 hours according to the teaching of R. A. Abramovitch and G. N. Knaus, *J. Org. Chem., 40,* 883 (1975).

The intermediate sulfonamides of Formula (IIe), where D is D—4 and $R_{23}$ is $C_1$—$C_3$ alkyl, can be prepared by reacting a 2-(alkylamino)sulfonylbenzenesulfonylazide of Formula (XXV) with a reducing agent such as sodium borohydride as shown in Equation 13.

Equation 13

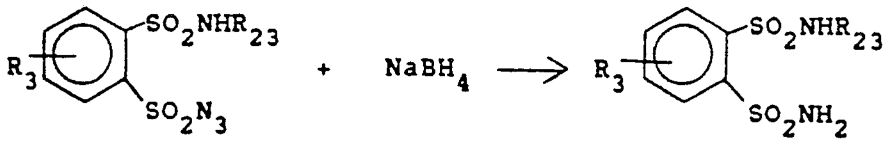


(XXV) (IIe)

where $R_{23}$ is $C_1$-$C_3$ alkyl.

The reaction is carried out at −20°C to 60°C in a protic solvent such as ethanol for 10 to 96 hours under an inert atmosphere.

The required 2-(alkylamino)sulfonylbenzenesulfonylazide (XXV) can be prepared by reacting a N-alkyl-*o*-fluorosulfonylbenzenesulfonamide of Formula (XXVI) with sodium azide as shown in Equation 14.

Equation 14

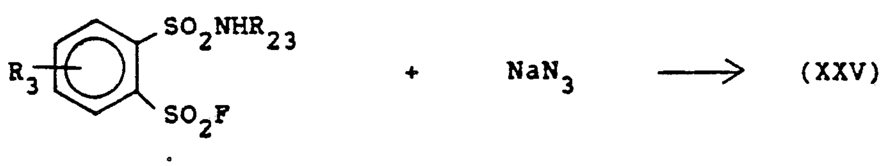


(XXVI)

where $R_{23}$ is $C_1$-$C_3$ alkyl.

The reaction is carried out at −20° to 80°C in a protic solvent such as water or methanol for 0.5 to 90 hours.

The required N-alkyl-*o*-fluorosulfonylbenzenesulfonamide (XXVI) can be prepared by reacting a *o*-fluorosulfonylbenzenesulfonyl chloride (XXIII) with two equivalents of an appropriate alkylamine as shown in Equation 15.

Equation 15

(XXIII) + $H_2NR_{23}$

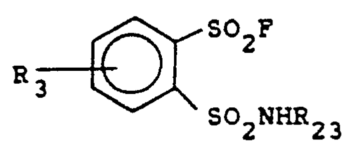

(XXVI)

where $R_{23}$ is $C_1$-$C_3$ alkyl.

The reaction is carried out at −20° to 60°C in an inert protic solvent such as tetrahydrofuran for 0.5 to 96 hours.

The intermediate sulfonamides of Formula (IIf) where D is D—4 and E is

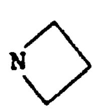 or 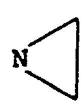

can be prepared by reacting a sulfonyl fluoride of Formula (XXVII) with ammonia as shown in Equation 16.

Equation 16

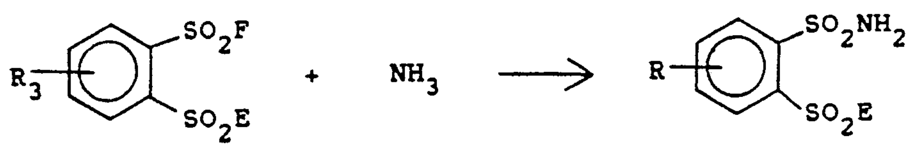

(XXVII) (IIf)

The reaction is carried out at 20—80°C in an inert protic solvent such as tetrahydrofuran for 2—90 hours. The required sulfonyl fluoride (XXVII) is prepared by reacting a *o*-fluorosulfonylbenzenesulfonyl chloride of Formula (XXII) with azetidine or aziridine in the presence of a base such as triethylamine as shown in Equation 17 where E is either

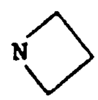 or 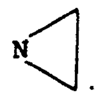.

Equation 17

$$(XXIII) + HE \xrightarrow[CH_3CN]{N(Et)_3} (XXVII)$$

The reaction is carried out at −20°C to 60°C in an inert solvent such as acetonitrile for 2—90 hours.

The intermediate sulfonamides of Formula (IIg), where D is D—4 and E is $NR_{23}C(O)NR_{42}R_{43}$ with $R_{42}$ and $R_{43}$ being other than H, can be prepared by reacting the sulfonamides of Formula (IId) or (IIe) with a carbamyl chloride of Formula (XXVIII) in the presence of a base such as triethylamine as shown in Equation 18.

Equation 18

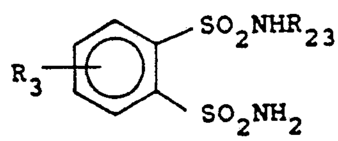

+

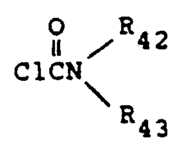

(IId) or (IIe) (XXVIII)

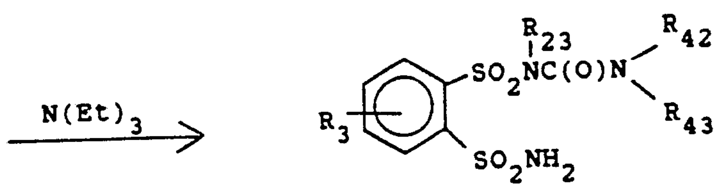


(IIg)

The reaction is carried out at 25—110°C in a solvent such as diglyme for 10 to 296 hours.

Alternatively, the intermediate sulfonamides of Formula (IIg) can be prepared by reacting the sulfonamides of Formula (IId) or (IIe) with a carbamate of Formula (XXIX) in the presence of trimethylaluminum as shown in Equation 19.

Equation 19

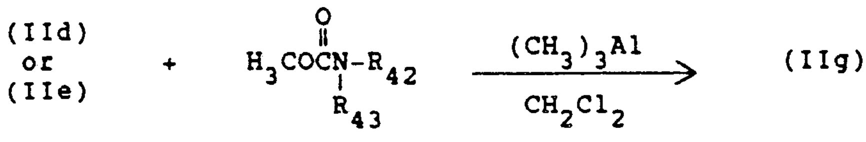


(XXIX)

The reaction is carried out at 25° to 80°C in a solvent such as methylene chloride or 1,2-dichloroethane for 10 to 96 hours under an inert atmosphere.

The intermediate sulfonamides of Formula (IIh) where D is D—4 and E is —$N(R_{23})C(O)R_{41}$ can be prepared by reacting the sulfonamides of Formula (IId) or (IIe) with an acid chloride of Formula (XXX) in the presence of a base such as triethylamine as shown in Equation 20

Equation 20

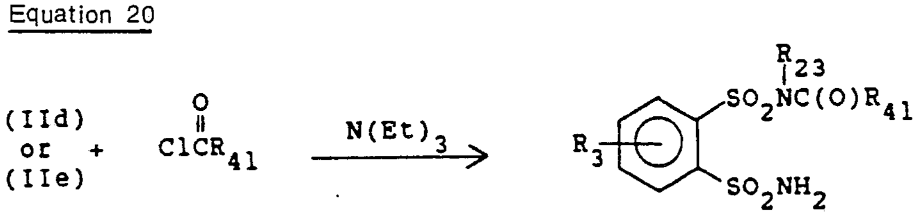


(XXX) (IIh)

The reaction is carried out 25—110°C in a solvent such as diglyme for 10 to 296 hours.

Alternatively, the intermediate sulfonamides of Formula (IIh) can be prepared by reacting the sulfonamides of Formula (IId) or (IIe) with a methyl ester of Formula XXXI in the presence of trimethylaluminum as shown in Equation 21.

Equation 21

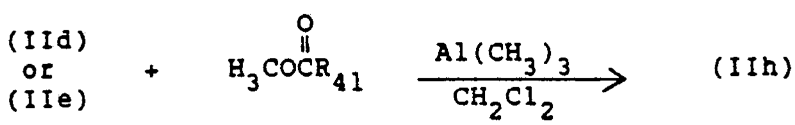


(XXXI)

The reaction is carried out at 25° to 80°C in a solvent such as methylene chloride or 1,2-dichloroethane for 10 to 96 hours under an inert atmosphere.

The intermediate sulfonamides of Formula (IIi) where D is D—4 and E is $N(R_{23})CO_2R_{44}$ can be prepared by reacting the sulfonamides of Formula (IId) or (IIe) with a chloroformate of Formula (XXXII) in the presence of a base such as triethylamine as shown in Equation 22

Equation 22

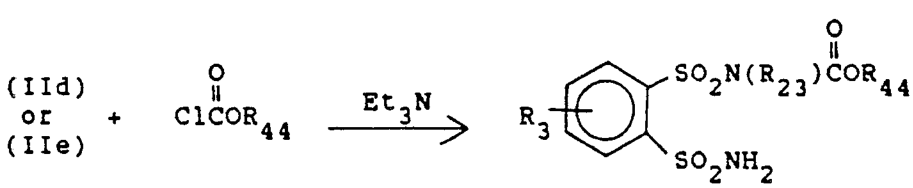


(XXXII) (IIi)

The reaction is carried out at 25—110°C in a solvent such as diglyme for 10 to 296 hours under an inert atmosphere.

The intermediate sulfonamides of Formula (IIj) where D is D—4 and E is $N(R_{23})C(O)NHR_{45}$ can be

prepared by reacting the sulfonamide of Formula (IId) or (IIe) with the isocyanate of Formula (XXXIII) in the presence of a base such as 4-N,N-dimethylaminopyridine as shown in Equation 23.

Equation 23

(IId) or (IIe) + $OCN-R_{45}$

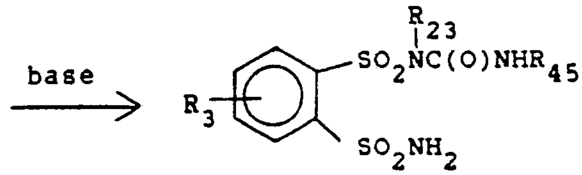


(XXXIII) (IIj)

This reaction is carried out at 25—110°C in a solvent such as diglyme for 10 to 296 hours. The intermediate sulfonamides (IIk) where D is D—4 and $E_1$ is

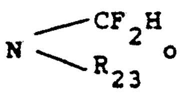
or
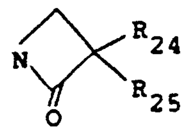

can be prepared by reacting a sulfonyl chloride of Formula (XXXIV) with ammonia as shown in Equation 24.

Equation 24

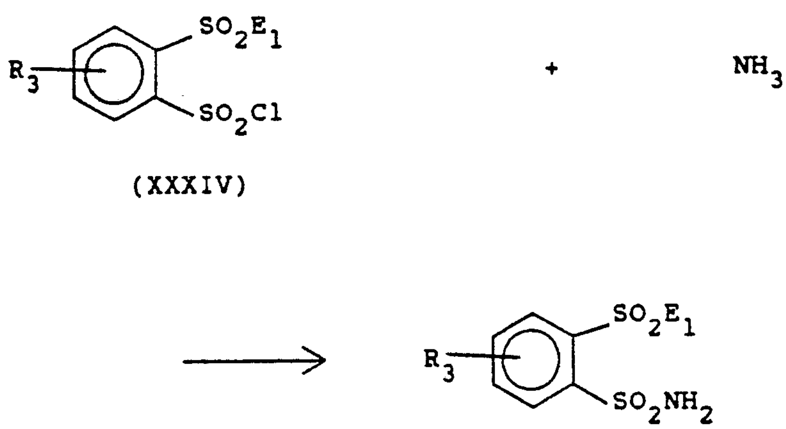

(XXXIV) + $NH_3$

(IIk);

where

$E_1$ is as defined above.

The reaction is carried out at 20—80°C in an inert aprotic solvent such as tetrahydrofuran for 2—90 hours. The required sulfonyl chloride (XXXIV) can be prepared from the corresponding chlorides of Formula (XXXV) by the two step sequence shown in Equation 25.

Equation 25

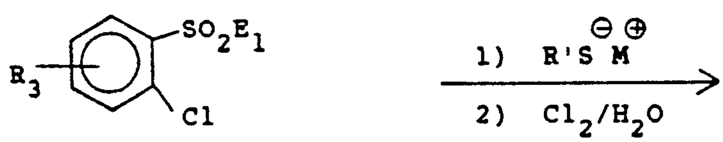

(XXXIV)

(XXXV)

The first step involves nucleophilic displacement of the chlorine atom with an alkyl or benzyl mercaptide to give an intermediate sulfide. The reaction can be carried out at 25° to 80°C in a polar solvent such as DMF for 0.5 to 24 hours. The sulfide is then oxidatively chlorinated to the desired sulfonyl chloride by the addition of molecular chlorine or a chlorine equivalent to the sulfide in the presence of water at 15 to 80°C in an aliphatic carboxylic acid solvent such as acetic acid or an inert organic solvent such as dichloroethane for 1 to 24 hours. The required chlorides of Formula (XXXVa) can be prepared by reacting a *o*-chlorosulfonyl isocyanate of Formula (XXXVI) with an alkene of Formula (XXXVII) as shown in Equation 26.

Equation 26

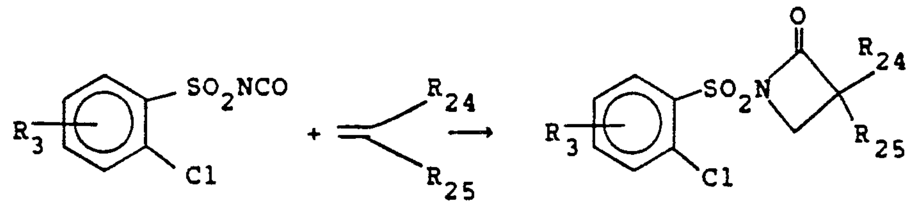

(XXXVI) (XXXVII) (XXXVa)

The reaction is carried out at 25—120°C in a solvent such as nitromethane for 2—90 hours under an inert atmosphere.

The required chlorides of Formula (XXXVb) can be prepared by reacting a o-chlorobenzenesulfonamide of Formula (XXXVIII) with $ClCF_2H$ in the presence of a base such as sodium hydroxide, potassium hydroxide or potassium carbonate as shown in Equation 27.

Equation 27

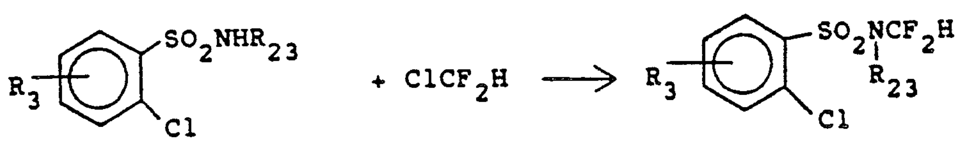


(XXXVIII) (XXXVb)

The reaction is carried out at 25—170°C in a solvent such as DMF or dioxane for 2—90 hours under an inert atmosphere.

Some sulfonamides (III) can be prepared from the corresponding nitro compounds as shown in Equation 28.

Equation 28

a)

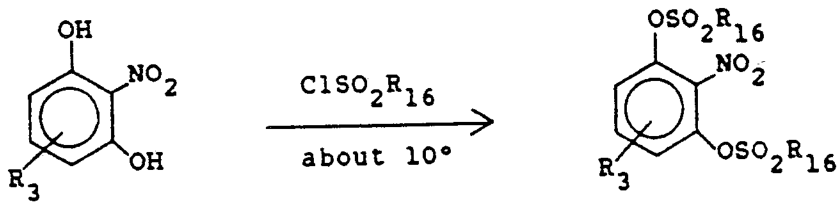


(XXXIX)

wherein

$R_3$ is as previously defined; and

$R_{16}$ is $C_1$-$C_3$ alkyl or $CF_3$.

b)

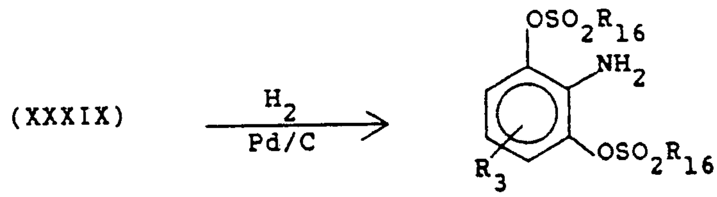


(XXXX)

c)

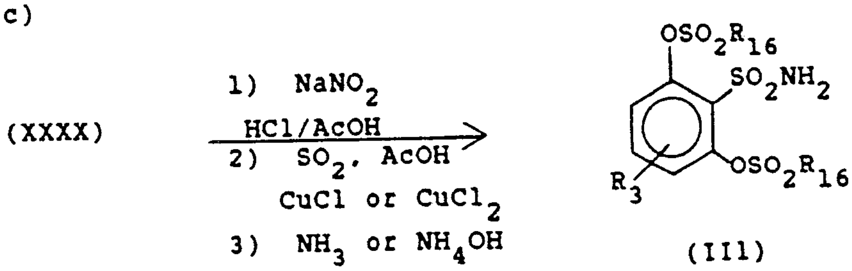


(III)

Reaction step 28a is best carried out in a solvent which will also act as an acid scavenger such as pyridine at temperatures below 10°C during the addition of alkylsulfonyl chloride. After 2 to 20 hours at ambient temperature, the products can be isolated by pouring the mixture into a cold solution of acid such as hydrochloric acid and washing the resulting precipitate with dilute acid.

The reduction in Equation 28b can be run by any of several methods in the literature. For example, the reaction can be run using catalytic reduction with 10% palladium-on-charcoal in an inert solvent such as methanol or tetrahydrofuran at 25° to 45°C at 1 to 3 atmospheres of hydrogen. For details refer to a similar procedure described by M. Vincent, et al., *Bull. Soc. Chim. France*, 1580 (1962).

The reaction of Equation 28c is accomplished by treating a solution of amine such as (XXXX) in a mixture of concentrated hydrochloric acid and glacial acetic acid with a solution of sodium nitrite in water at −5° to 5°C. After stirring for 10—30 minutes at about 0°C to insure complete diazotization, the solution is added to a mixture of an excess of sulfur dioxide and a catalytic amount of cuprous chloride or cupric chloride in glacial acetic acid at about 10°C. The temperature is kept at about 10°C for 1/4 to 1 hour, then raised to 20° to 30°C and held at that temperature for 2 to about 24 hours. This solution is then poured into a large excess of ice water. The sulfonyl chloride can be isolated by filtration or by extraction into a solvent such as ethyl ether, methylene chloride or preferably, 1-chlorobutane, followed by evaporation of the solvent.

The amination is conveniently carried out as described for Equation 10c to provide sulfonamide (II1).

The heterocyclic amines of Formula (V) in Equations 2 and 4 are also important intermediates for the preparation of the compounds of this invention and may be prepared by the following methods.

The pyrimidines and triazines of Formula (Va) to (Vd) below are either known or can be prepared by obvious methods by one skilled in the art. For instance, the synthesis of pyrimidines and triazines of the general formula (Va) has been reviewed in "The Chemistry of Heterocyclic Compounds", a series published by Interscience Publishers, Inc., New York and London. 2-Aminopyrimidines are described by D. J. Brown in "The Pyrimidines", Vol. 16 of this series. 2-Amino-1,3,5-triazines are reviewed by E. M. Smolin and L. Rapaport in "*s*-Triazines and Derivatives", Vol. 13 of the same series. The synthesis of triazines is also described by F. C. Schaefer, U.S. 3,154,547 and by K. R. Huffman and F. C. Schaefer, *J. Org. Chem., 28*, 1812 (1963). The synthesis of the bicyclic amines (Vc) and (Vd) are described in EP—A—15,683, and that of (Vb) in EP—A—46,677.

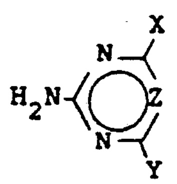

(Va)

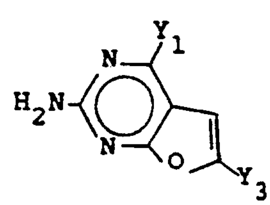

(Vb)

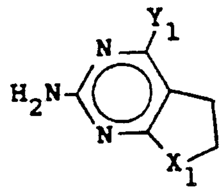

(Vc)

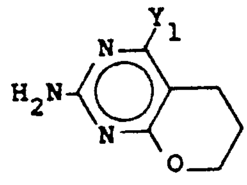

(Vd)

wherein X, $X_1$, Y, $Y_1$, $X_3$ and Z are as originally defined.

Heating equimolar amounts of ethyl propionimidate hydrochloride and N-aminoguanidine nitrate in pyridine gives 3-amino-5-ethyltriazole; *German Patent* 1,073,499 (1960); *Berichte, 96,* 1064 (1963).

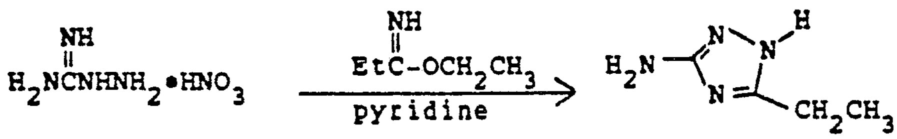


Condensation of hydrazine with ethyl N-cyanoacetimidate yields 3-amino-5-methyltriazole; *Journal of Organic Chemistry, 28,* 1816 (1963).

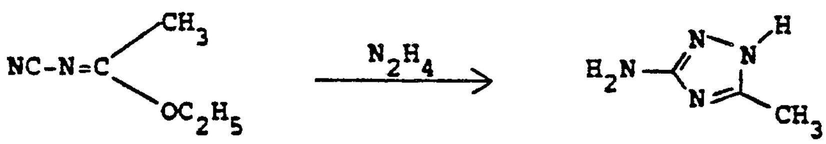


*British Patent* 736,568 (1955) describes the synthesis of 3-amino-5-mercaptotriazole.

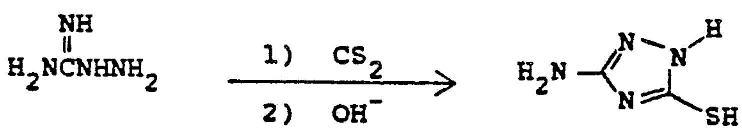


Condensing hydrazine with dimethyl cyanodithioimidocarbonate in acetonitrile gives 3-amino-5-methylthio-1,2,4-triazole while reaction of hydrazine with dimethyl N-cyanoimidocarbonate produces 3-amino-5-methoxy-1,2,4-triazole; *Journal of Organic Chemistry, 39,* 1522 (1974).

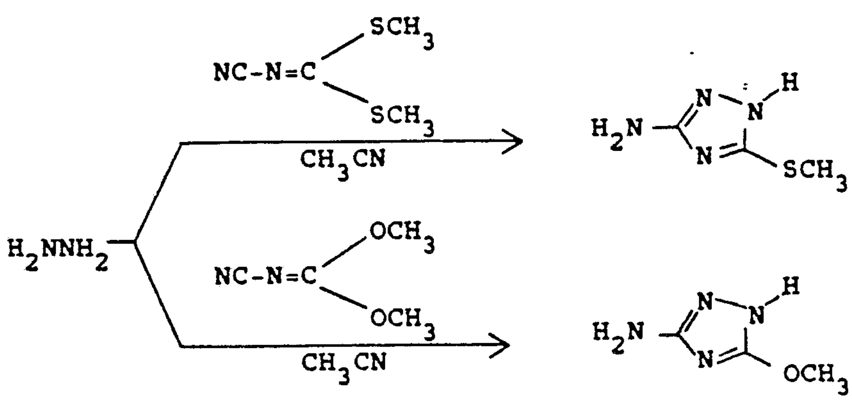


Reaction of substituted hydrazines with N-cyanothioimidocarbonates (prepared according to the procedure given in D. M. Wieland, Ph.D. Thesis, 1971, pp. 123—124) yields disubstituted aminotriazoles as shown below.

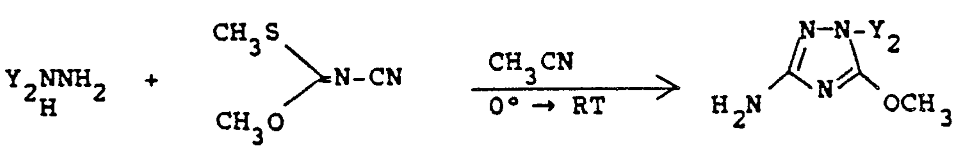

Many of the aminoheterocyclic intermediates of Formula (V) where $R_4$ is methyl may be prepared by a two-step procedure as described for (Ve) in Equation 29.

Equation 29

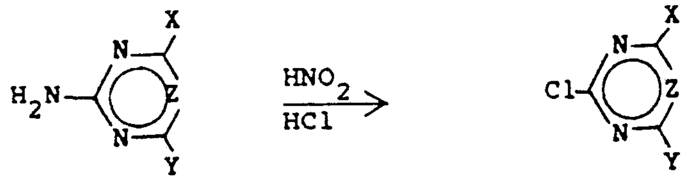


(Va) (XXXXI)

(XXXXI)

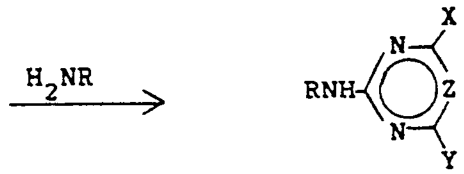


(Ve)

wherein

X, Y and Z are as originally define

R is $CH_3$.

A solution of the amine (Va) in concentrated hydrochloric acid is treated with sodium nitrite solution and the chloro compound (XXXXI) is isolated in the usual manner by filtration of the acidic solution. A representative procedure is described by Bee and Rose in *J. Chem. Soc. C*, 2031 (1966), for the case in which Z = CH, and X = Y = $OCH_3$. Displacement of the chlorine of (XXXXI) may be accomplished by heating with an excess of methylamine in water to obtain the methylamino heterocycle (Ve).

The triazine amines of Formula (Vf) where $X_3$ is $CH_3$ or $OCH_3$ can be prepared according to the teachings of EP—A—94,260, published November 16, 1983.

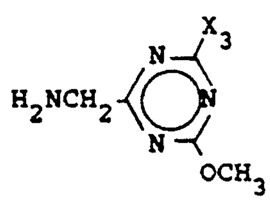

(Vf)

Agriculturally suitable salts of compounds of Formula (I) are also useful herbicides and can be prepared in a number of ways known to the art. For example, metal salts can be made by treating compounds of Formula (I) with a solution of alkali or alkaline earth metal salt having a sufficiently basic anion (e.g., hydroxide, alkoxide, carbonate or hydride). Quaternary amine salts can be made by similar techniques.

Salts of compounds of Formula (I) can also be prepared by exchange of one cation to another. Cationic exchange can be effected by direct treatment of an aqueous solution of a salt of a compound of Formula (I) (e.g., alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula (I) (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula (I) with a suitable acid, e.g., *p*-toluenesulfonic acid or trichloroacetic acid.

The preparation of the compounds of this invention is further illustrated by the following specific examples. Unless otherwise indicated, temperatures are in degrees centigrade.

Example 1

N-[1,1-(Dimethylethyl)]-2-(methylthio)benzenesulfonamide

To a mixture of 21.3 g (0.10 mol) of N-[1,1-(dimethylethyl)]benzenesulfonamide in 400 ml anhydrous tetrahydrofuran (THF) under nitrogen atmosphere was added 137 ml (0.22 mol) 1.6 M butyllithium in hexanes dropwise at −40°. The mixture was then warmed to room temperature for 2 hours then recooled to −20° and treated with 10.8 ml (0.12 mol) methyl disulfide in 15 ml THF. After stirring overnight at ambient temperature, 80 ml of 10% hydrochloric acid was added and the layers separated. The aqueous phase was extracted with ether and the combined organic extracts washed with brine, dried, and evaporated to a solid. The residue was triturated with ether/hexanes and collected to afford 23.5 g of the title compound, m.p. 138—140°.

NMR ($CDCl_3$): δ 1.20 (s, 9H); 2.58 (s, 3H); 5.39 (s, NH); 7.2—7.6 (m, 4H); and 8.02—8.27 (m, 1H) ppm.

Example 2

N-(1,1-Dimethylethyl)-2-(methylthio)-6-phenylbenzenesulfonamide

A solution of 11.3 g (0.0436 mol) N-(1,1-dimethylethyl)-2-(methylthio)benzenesulfonamide in 300 ml tetrahydrofuran (THF) was treated with 60.5 ml (0.096 mol) 2 M *n*-butyllithium in hexanes at −30° under a nitrogen atmosphere. The mixture was stirred for 1 hour at ambient temperature then recooled to −20° and treated with 8.3 g (0.0436 mol) of cuprous iodide (anhydrous). After 10 minutes at −15°, 4.9 ml (0.0438 mol) iodobenzene was added and the mixture was then heated to reflux overnight. Acetic acid (10 ml) was added after cooling the mixture to 15°, then 200 ml concentrated ammonium hydroxide plus 200 ml ethyl acetate was introduced and the mixture was stirred vigorously in the presence of air to facilitate removal of the copper salts. The aqueous phase was separated, extracted with more ethyl acetate and the combined organic extracts were washed successively with concentrated ammonium hydroxide, water and brine, then dried ($MgSO_4$) and evaporated to an oil. The product crystallized from ether/hexanes to afford 10 g of material, m.p. 162—165°.

NMR ($CDCl_3$): δ 1.1 (s, 9H); 2.65 (s, 3H); 5.2 (s, NH); 7.2 (t, J=4Hz, 1H); and 7.5 (m, 7H) ppm.

Example 3

2,6-*Bis*(methylthio)-N-(1,1-dimethylethyl)benzenesulfonamide

Under nitrogen, a homogeneous solution of 80 g of N-(1,1-dimethylethyl)benzenesulfonamide in 1100 ml dry THF was cooled to −40° and 516 ml of 1.6 M *n*-butyllithium added dropwise at this temperature. After the addition was complete, the reaction mixture was allowed to warm to room temperature and stirred for 2 hours. The resulting thick ppt. was cooled to 0° and 38 ml of methyl disulfide added dropwise. After warming to room temperature, the suspension was stirred for 1.5 hour. It was then cooled to −40° and an additional 285 ml *n*-butyllithium added dropwise. The suspension was allowed to warm to 25°C and stirred at this temperature for 2 hours. Cooling to 0° and the dropwise addition of 41 ml of methyl disulfide gave a bright orange suspension. After stirring at room temperature for 18 hours, the white suspension was cooled to 0° and 20 ml of saturated ammonium chloride added dropwise. The mixture was poured into 1 l of water and extracted with ether. An insoluble precipitate remained which was collected by filtration and air dried to provide 42.3 g of 2,6-*bis*(methylthio)-N-(1,1-dimethylethyl)benzenesulfonamide as a white powder, m.p. 182—185°.

The combined ether extracts were washed twice with water, once with brine and dried over sodium sulfate. Concentration gave a pale yellow solid which was washed with *n*-butyl chloride, providing an additional 34.5 g of the title compound, m.p. 174—184°.

NMR (DMSO—$d_6$): δ 1.11 (s, 9, *t*-Bu); 2.48 (s, 6, $SCH_3$); and 7.12—7.65 (m, 4).

IR (nujol): 3.05 (NH).

Example 4

2-(Methylthio)-6-phenylbenzenesulfonamide

In a mixture of 75 ml trifluoroacetic acid plus 2 ml water was dissolved 10 g (.030 mol) of N-(1,1-dimethylethyl)-2-(methylthio)-6-phenylbenzenesulfonamide and heated to 85°. After 15 minutes the mixture was cooled and evaporated, then diluted with water and extracted with methylene chloride. The methylene chloride was washed with saturated sodium bicarbonate solution, dried ($MgSO_4$), evaporated *in vacuo* and crystallized from ether/hexanes to afford 6.5 g of the title compound, m.p. 167—168°.

NMR ($CDCl_3$): δ 2.55 (s, 3H); 6.62 (2, $NH_2$); 7.07 (m, 1H); and 7.35—7.55 (m, 7H) ppm.

Example 5

2-(Methylsulfonyl)-6-phenylbenzenesulfonamide

A solution of 6.5 g (.023 mol) 2-(methylthio)-6-phenylbenzenesulfonamide in 100 ml methylene chloride plus 20 ml ethyl acetate was treated with 10 g (.046 mol) 80% *m*-chloroperoxybenzoic acid. The mixture was subsequently stirred at ambient temperature for 3 days then filtered. The solids were rinsed with saturated sodium bicarbonate, water and more methylene chloride. A second crop was obtained from

the combined bicarbonate washed methylene chloride filtrates to afford 6.9 g of the title compound, m.p. 258—259°.

NMR ($CDCl_3$/DMSO—$d_6$): δ 3.50 (s, 3H); 6.95 (s, $NH_2$); 7.47 (s, 5H); 7.5—7.98 (m, 3H); and 8.42 (m, 1H) ppm.

Example 6

N-[(4-Methoxy-6-methylpyrimidin-2-yl)aminocarbonyl]-3-(methylsulfonyl)-1,1′-biphenyl-2-sulfonamide

To a stirred suspension of 1.2 g (3.86 mmol) 2-(methylsulfonyl)-6-phenylbenzenesulfonamide in 35 ml methylene chloride under nitrogen atmosphere was added 2.5 ml (5 mmol) of 2 M trimethylaluminum in toluene via syringe at ambient temperature. Solid methyl N-(4-methoxy-6-methylpyrimidin-2-yl)carbamate (0.95 g, 4.8 mmol) was then added and the mixture was heated to reflux for 1 day. Aqueous 5% hydrochloric acid (50 ml) was added to the cooled mixture followed by ether. The solid product which separated was collected and rinsed with ether and water, then chromatographed on silica gel with 10% ethyl acetate in methylene chloride as the eluant. Trituration of the solid product with ether/hexanes afforded 0.32 g of the title compound, m.p. 206—208°.

NMR ($CDCl_3$/DMSO—$d_6$): δ 2.30 (s, 3H); 3.60 (s, 3H); 3.88 (s, 3H); 6.27 (s, 1H); 7.15—7.9 (m, 7H); 8.4 (sbr, NH); 8.45 (m, 1H); and 12.3 (sbr, NH) ppm.

IR (nujol): 1700, 3250 $cm^{-1}$.

Example 7

2,6-Bis(methylthio)benzenesulfonamide

A brown solution of 70 g of 2,6-*bis*(methylthio)-N-(1,1-dimethylethyl)benzenesulfonamide in 200 ml trifluoroacetic acid was stirred at room temperature. After 10 minutes, a heavy precipitate formed, the suspension was stirred for 18 hours. The resulting solid was collected by filtration and dissolved in 10% NaOH, filtered and the filtrate acidified with 10% HCl. The precipitated solid was washed with water and air dried to provide 48 g of 2,6-*bis*(methylthio)benzenesulfonamide, m.p. 197—199°.

NMR (DMSO—$d_6$): δ 2.48 (s, 6, $SCH_3$); and 7.12—7.62 (m, 5).

IR (nujol): 2.90 and 3.00 ($NH_2$).

Example 8

2,6-*Bis*(Methylthio)-N-(butylaminocarbonyl)benzenesulfonamide

A stirred suspension of 5.0 g (.02 mol) of 2,6-*bis*(methylthio)benzenesulfonamide, 8.3 g (.06 mol) potassium carbonate and 3.4 ml (.03 mol) *n*-butyl isocyanate was heated at reflux for four hours. After stirring at 25° overnight, the mixture was poured into ice and 30 ml 10% HCl. The resulting precipitate was collected, washed several times with water and air dried to provide 6.5 g of the title compound, m.p. 151—154°.

NMR (DMSO—$d_6$): δ 0.50—1.00 (m, 3); 1.00—1.47 (m, 4); 2.43 (s, 6); 2.67—3.15 (m, 2); 6.18—6.56 (m, 1); and 7.10—7.75 (m, 3).

IR (nujol): 3.00 (NH); and 5.95 (C=O).

Example 9

2,6-*Bis*(methylthio)-N-[(4-chloro-6-methoxypyrimidin-2-yl)aminocarbonyl]benzenesulfonamide

A suspension of 2.3 g (6.7 mmol) of 2,6-*bis*(methylthio)-N-(butylaminocarbonyl)benzenesulfonamide and .07 g (.67 mmol) of DABCO in 30 ml xylene was heated to 140°. To the resulting solution, phosgene (1.9 ml, 16 mmol) was added dropwise at 135 to 140°C. The mixture was heated at reflux for 2 hours, cooled and the solvent removed *in vacuo*. The resulting solid was dissolved in 50 ml methylene chloride under $N_2$ and 1.37 g (8.6 mmol) 2-amino-4-chloro-6-methylpyrimidine added with .01 g (.1 mmol) of DABCO. The mixture was stirred overnight and the solvent removed *in vacuo*.

The residue was triturated with 1-chlorobutane. The resulting solid was dissolved in cold 1% NaOH, filtered and precipitated with 10% HCl washed with water and air dried to provide .46 g of the title compound, m.p. 179—180°C.

NMR (DMSO—$d_6$): δ 2.48 (s, 6, $SCH_3$); 4.00 (s, 3, $OCH_3$); 6.90 (s, 1, ArH); 7.18—7.79 (m, 3, ArH); 10.88 (brs, 1, NH); and 12.48 (brs, 1, NH).

IR (nujol): 5.80 (C=O).

Example 10

N,N′-Bis[(4,6-dimethoxypyrimidin-2-yl)aminocarbonyl]-1,2-benzenedisulfonamide

To a suspension of 0.5 g of 1,2-benzenedisulfonamide and 2.5 ml of trimethylaluminum in 50 ml of anhydrous methylene chloride was added 1 g of 4,6-dimethoxypyrimidin-2-ylcarbamic acid methyl ester under $N_2$ with stirring. After addition, the mixture was stirred at room temperature for 30 minutes and was then heated to reflux for 12 hours. The reaction mixture was then cooled down to room temperature. To this mixture was added 50 ml of methylene chloride, 100 ml of water, 10 ml of acetic acid and 5 drops of concentrated hydrochloric acid. The mixture was then stirred at room temperature for 20 minutes and filtered. The organic layer was separated, washed with water, dried over $MgSO_4$ and concentrated. The residue was stirred in *n*-butyl chloride for 2 hours and filtered. The solid was washed with copious amount

of ethyl acetate and then dried to yield 0.18 g of N,N'-bis[(4,6-dimethoxypyrimidin-2-yl)aminocarbonyl]-1,2-benzenedisulfonamide, m.p. 201—203°C.

NMR (DMSO—$d_6$) δ: 3.92 (12H, s); 5.95 (2H, s); 7.8—8.6 (4H); 10.2 (2H, s); 12.2 (2H, bs).

Example 11

1,2-Benzenedisulfonamide

One gram of 1,2-benzenedisulfonylazide was added portionwise to 0.4 gm sodium borohydride in 15 ml of ethyl alcohol with stirring and cooling. After addition, the mixture was stirred with cooling (ice/acetone bath) for 2 hours and filtered. The solid was washed with cold ethyl alcohol and ether to yield 0.8 g of 1,2-benzenedisulfonamide, m.p. 250°C. Mass spec. (M/e) 236.

Example 12

2-(Azetidin-1-ylsulfonyl)-N-[(4,6-dimethoxy-1,3,5-triazin-2-yl)aminocarbonyl]benzenesulfonamide

In a dry flask under nitrogen was placed 1.20 gram of 2-(azetidin-1-ylsulfonyl)benzenesulfonamide, 50 ml of dry dichloromethane and 2.48 ml of 2 M trimethylaluminum in toluene. To this was added 0.885 g of 4,6-dimethoxy-1,3,5-triazin-2-ylcarbamic acid methyl ester and the resultant solution was heated to reflux for 12 hours. The solution was washed with 50 ml of a water/acetic acid/1N HCl solution (40:10:0.4). The organic fraction was concentrated and chromatographed on silica gel (ethyl acetate/methylene chloride (15:85)). The slower moving portions were collected, treated with ethyl acetate and dried to give 0.45 g of a solid, m.p. 175—177°C.

HNMR ($CDCl_3/CF_3CO_2H$, 90 MHz) δ: 2.0—2.35 (m, 3H); 4.05 (t, 4H, J 8Hz); 4.15 (s, 6H); 7.7—8.7 (m, 4H).

IR (nujol) 1700 $cm^{-1}$.

Example 13

2-(Azetidin-1-ylsulfonyl)benzenesulfonamide

In a 1-l flask was placed 9.8 g of 2-(azetidin-1-ylsulfonyl)benzenesulfonyl fluoride and 500 ml of THF. Then 9 ml of ammonia was added and the mixture was refluxed for two days. Four 25 ml portions of liquid ammonia were then added to the refluxing solution over a 48 hour period. The reaction mixture was concentrated and the residue chromatographed on silica gel (dichloroethane). The desired product was collected to give 4.9 g of a solid m.p. 121—123°C.

NMR ($CDCl_3$/DMSO—$d_6$, 90 mHz) δ: 1.9—2.30 (m, 2H); 3.95 (t, 4H); 6.90 (bs, 2H); 7.65—8.35 (m, 4H).

IR (nujol) $cm^{-1}$ 3420 and 3300 ($NH_2$), 1340 and 1170 ($SO_2$).

Example 14

2-(1-Azetidinylsulfonyl)benzenesulfonyl fluoride

In a flask was placed 9.24 g of 2-fluorosulfonylbenzenesulfonyl chloride and 200 ml of dry acetonitrile. The resultant solution was cooled to 0°C. An addition funnel was charged with 2 g of azetidine, 4.95 ml of triethylamine and 50 ml of acetonitrile. This solution was added dropwise to the first solution. After stirring overnight the solvent was evaporated under reduced pressure. The residue was partitioned between dichloromethane and 1 N hydrochloric acid. The organic layer was separated, dried and concentrated to give 9.80 g of a solid, m.p. 93°C.

NMR ($CDCl_3$, 90 MHz) δ: 2.20 (m, 2H); 4.10 (t, 4H); 7.6—8.50 (m, 4H).

IR (nujol) $cm^{-1}$ 1360 (b); 1165 (b) ($SO_2$).

Example 15

N-Difluoromethyl-N-methyl-2-chlorobenzenesulfonamide

A mixture of 260 ml of dioxane, 260 ml of 50% sodium hydroxide aqueous solution and 55 g (0.2683 mole) of N-methyl-2-chlorobenzenesulfonamide was heated to 70°—75°C. To this mixture was then added 37.6 ml of Freon 22® ($CF_2HCl$) dropwise over a period of 1 hour. The solution was heated at 70—75°C for an additional 12 hours and was then allowed to cool to room temperature while stirring. The reaction mixture was then extracted with one portion of ether and one portion of ethyl acetate. The combined organic layers were washed with water, 1N HCl solution and brine. The organic layer was then concentrated and the residue was chromatographed on silica gel (ethyl acetate/Hexane 1:1) to yield 29.95 g of N-difluoromethyl-N-methyl-2-chlorobenzenesulfonamide as an oil.

NMR ($CDCL_3$) δ: 2.86 (3H, s); 7.08 (1H, t); 7.4—8.2 (4H, m).

Example 16

N-Difluoromethyl-N-methyl-2-(n-propylthio)benzenesulfonamide

To a mixture of 14.62 ml of n-propylmercaptan, 16.3 g of potassium t-butoxide and 400 ml of DMF at 0°C was added 27.45 g (0.1076 mole) of N-difluoromethyl-N-methyl-2-chlorobenzenesulfonamide in 100 ml of DMF. The reaction mixture was stirred at room temperature for 2 days and then at 60°C for 4 hours. The mixture was then concentrated and the residue was partitioned between 1N HCl solution and methylene chloride. The organic layer was separated, dried over $MgSO_4$, and concentrated. The residue was chromatographed on silica gel (Hexane/chloroform 3:2) to yield 24.63 g of N-difluoromethyl-N-methyl-2-(n-propylthio)benzenesulfonamide as an oil.

NMR ($CDCl_3$) δ: 1.05 (3H, t); 1.50—1.80 (2H, m); 2.81 (3H, s); 2.98 (2H, t); 7.18 (1H, t); 7.2—8.05 (4H, m).

Example 17

N-Difluoromethyl-N-methyl-1,2-benzenedisulfonamide

To a mixture of 23.08 g (0.078 mole) of N-difluoromethyl-N-methyl-2-(n-propylthio)benzenesulfonamide, 160 ml of acetic acid and 3.0 ml of distilled water at 0°C was added 16.59 ml of chlorine dropwise over 15 minutes. The reaction mixture was allowed to warm to ambient temperature and was stirred at ambient temperature for 2 hours. The reaction mixture was then poured into ice water with a solid precipitating. The solid was washed with water and small amount of pentane to give 20.6 g of material (m.p. 89—91°C) after being air dried. The filtrate was extracted with 500 ml of $CH_2Cl_2$. The $CH_2Cl_2$ layer was separated, washed with brine, dried over $MgSO_4$, and concentrated to give 10.16 g of a light yellow oil. The solid and the oil were then combined and dissolved in 300 ml of anhydrous THF. To this solution was added 50 ml of ammonium hydroxide dropwise. The reaction mixture was heated to reflux. After refluxing for 4 hours, an additioanl 30 ml of ammonium hydroxide was added. The reaction was refluxed for another 12 hours. To this mixture was then added another 50 ml of ammonium hydroxide. The solution was then refluxed for another 12 hours. To the reaction mixture was then added 50 ml of ethyl acetate. The resulting solution was washed with water, dried over $MgSO_4$ and concentrated. The residue was then triturated with $CH_2Cl_2$/acetone (1:1) with a solid precipitating. The solid was then collected by filtration to yield 3.93 g of N-difluoromethyl-N-methyl-1,2-benzenedisulfonamide, m.p. 128°C. The filtrate was concentrated and the residue was chromatographed on silica gel to yield another 8.5 g of the title compound.

NMR (DMSO—$d_6$) δ: 2.85 (3H, bs); 7.26 (1H, t); 7.5 (2H, bs); 7.75—830 (4H, m).

Following the procedures described above, the compounds in the Tables hereinafter can be prepared. In some of these tables, some variables are defined twice, there being a different definition when Z = CH and when Z = N. In these instances, a single table entry represents two or more compounds. However, when a row is followed by a superscript, those substituent definitions apply only when Z has the definition denoted by the superscript.

Table 1

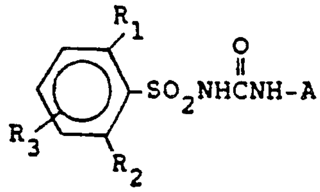

| $R_2$ | $R_1$ | $R_3$ | A | $X_1$ | $Y_1$ | $X_3$ | $Y_2$ | $X_2$ | $Y_3$ |
|---|---|---|---|---|---|---|---|---|---|
| $SO_2CH_3$ | $C_6H_5$ | H | A-2 | O | $CH_3$ | - | - | - | - |
| $SO_2CH_3$ | $C_6H_5$ | H | A-2 | O | $OCH_3$ | - | - | - | - |
| $SO_2CH_3$ | $C_6H_5$ | H | A-2 | $CH_2$ | $CH_3$ | - | - | - | - |
| $SO_2CH_3$ | $C_6H_5$ | H | A-2 | $CH_2$ | $OCH_3$ | - | - | - | - |
| $SCH_3$ | $SCH_3$ | H | A-2 | O | $CH_3$ | - | - | - | - |
| $SCH_3$ | $SCH_3$ | H | A-2 | O | $OCH_3$ | - | - | - | - |
| $SO_2CH_3$ | $C_6H_5$ | H | A-2 | O | Cl | - | - | - | - |
| $SO_2CH_3$ | $C_6H_5$ | H | A-2 | O | $OCF_2H$ | - | - | - | - |
| $SO_2CH_3$ | $C_6H_5$ | H | A-3 | - | $CH_3$ | - | - | - | - |
| $SO_2CH_3$ | $C_6H_5$ | H | A-3 | - | $OCH_3$ | - | - | - | - |
| $SO_2CH_3$ | $C_6H_5$ | H | A-3 | - | Cl | - | - | - | - |
| $SO_2CH_3$ | $C_6H_5$ | H | A-3 | - | $OCF_2H$ | - | - | - | - |
| $SCH_3$ | $SCH_3$ | H | A-3 | - | $OCH_3$ | - | - | - | - |
| $SCH_3$ | $SCH_3$ | H | A-3 | - | $CH_3$ | - | - | - | - |
| $SO_2CH_3$ | $C_6H_5$ | H | A-4 | - | $CH_3$ | - | - | - | $CH_3$ |
| $SO_2CH_3$ | $C_6H_5$ | H | A-4 | - | $OCH_3$ | - | - | - | $CH_3$ |
| $SO_2CH_3$ | $C_6H_5$ | H | A-4 | - | Cl | - | - | - | H |
| $SO_2CH_3$ | $C_6H_5$ | H | A-4 | - | $OCF_2H$ | - | - | - | $CH_3$ |
| $SCH_3$ | $SCH_3$ | H | A-4 | - | $OCH_3$ | - | - | - | $CH_3$ |
| $SCH_3$ | $SCH_3$ | H | A-4 | - | $CH_3$ | - | - | - | H |
| $SO_2CH_3$ | $C_6H_5$ | H | A-5 | - | - | $CH_3$ | - | - | - |
| $SO_2CH_3$ | $C_6H_5$ | H | A-5 | - | - | $OCH_3$ | - | - | - |
| $SCH_3$ | $CH_2OR_{17}$ | H | A-5 | - | - | $CH_3$ | - | - | - |
| $SCH_3$ | $CH_2OR_{17}$ | H | A-5 | - | - | $OCH_3$ | - | - | - |
| $SO_2CH_3$ | $C_6H_5$ | H | A-6 | - | - | - | $CH_3$ | $CH_3$ | - |
| $SO_2CH_3$ | $C_6H_5$ | H | A-6 | - | - | - | $CH_3$ | $OCH_3$ | - |

Table 1 (continued)

| $R_2$ | $R_1$ | $R_3$ | A | $X_1$ | $Y_1$ | $X_3$ | $Y_2$ | $X_2$ | $Y_3$ |
|---|---|---|---|---|---|---|---|---|---|
| $SO_2CH_3$ | $C_6H_5$ | H | A-6 | - | - | - | $CH_3$ | $SCH_3$ | - |
| $SO_2CH_3$ | $C_6H_5$ | H | A-6 | - | - | - | $CH_2CH_3$ | $CH_3$ | - |
| $SO_2CH_3$ | $C_6H_5$ | H | A-6 | - | - | - | $CH_2CH_3$ | $OCH_3$ | - |
| $SO_2CH_3$ | $C_6H_5$ | H | A-6 | - | - | - | $CH_2CH_3$ | $SCH_3$ | - |
| $SO_2CH_3$ | $C_6H_5$ | H | A-6 | - | - | - | $CH_2CF_3$ | $CH_3$ | - |
| $SO_2CH_3$ | $C_6H_5$ | H | A-6 | - | - | - | $CH_2CF_3$ | $OCH_3$ | - |

Table 2a

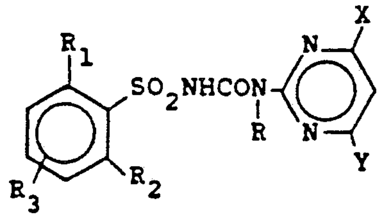

| $R_1$ | $R_2$ | $R_3$ | X | Y | m.p. (°C) |
|---|---|---|---|---|---|
| $SCH_3$ | $SCH_2CH_3$ | H | $CH_3$ | $OCH_3$ | |
| $SCH_2CH_3$ | $SCH_2CH_3$ | 3-Cl | $CH_3$ | $OCH_3$ | |
| $SCH_2CH_3$ | $SCH_2CH_2CH_3$ | H | $OCH_3$ | $OCH_3$ | |
| $SCH_2CH_2CH_3$ | $SCH_2CH_2CH_3$ | H | $CH_3$ | $OCH_3$ | |
| $S(O)CH_3$ | $SCH_3$ | H | $CH_3$ | $CH_3$ | |
| $S(O)_2CH_3$ | $SCH_3$ | H | $CH_3$ | $OCH_3$ | |
| $S(O)CH_3$ | $S(O)CH_3$ | H | $OCH_3$ | $OCH_3$ | |
| $S(O)_2CH_3$ | $S(O)CH_3$ | H | Cl | $OCH_3$ | |
| $SCH_3$ | $N(CH_3)_2$ | H | $CH_3$ | $OCH_3$ | |
| $S(O)CH_3$ | $N(CH_3)_2$ | H | $OCH_3$ | $OCH_3$ | |
| $S(O)_2CH_3$ | $N(CH_3)_2$ | H | Cl | $OCH_3$ | |
| $SCH_3$ | $N(CH_3)CH_2CH_3$ | 3-F | $CH_3$ | $OCH_3$ | |
| $SCH_3$ | $N(CH_2CH_3)_2$ | H | $OCH_3$ | $OCH_3$ | |
| $CF_3$ | $CF_3$ | H | $CH_3$ | $OCH_3$ | |
| $CF_3$ | $CF_3$ | 4-Br | $CH_3$ | $CH_3$ | |
| $N(CH_3)_2$ | $N(CH_3)_2$ | H | $CH_3$ | $OCH_3$ | |
| $N(CH_3)CH_2CH_3$ | $N(CH_3)_2$ | H | $OCH_3$ | $OCH_3$ | |
| $N(CH_2CH_3)_2$ | $N(CH_3)_2$ | 4-$CH_3$ | $CH_3$ | $OCH_3$ | |
| $N(CH_3)CH_2CH_3$ | $N(CH_3)CH_2CH_3$ | H | $OCH_3$ | $OCH_3$ | |
| $N(CH_2CH_3)_2$ | $N(CH_2CH_3)_2$ | H | Cl | $OCH_3$ | |
| $N(CH_3)_2$ | $C_6H_5$ | H | $CH_3$ | $OCH_3$ | |
| $N(CH_3)_2$ | 2'-$C_6H_4Cl$ | H | Cl | $OCH_3$ | |
| $N(CH_3)_2$ | $CH_2CO_2CH_3$ | H | $CH_3$ | $CH_3$ | |
| $N(CH_3)_2$ | $CH_2CO_2CH_2CH_3$ | H | $CH_3$ | $OCH_3$ | |

Table 2a (continued)

| $R_1$ | $R_2$ | $R_3$ | X | Y | m.p. (°C) |
|---|---|---|---|---|---|
| $N(CH_3)_2$ | $CH_2CO_2CH_2CH_2CH_3$ | H | Cl | $OCH_3$ | |
| $N(CH_3)_2$ | $CH_2CO_2CH_2CH=CH_2$ | H | $OCH_3$ | $OCH_3$ | |
| $N(CH_3)_2$ | $CH_2CO_2CH_2CH_2Cl$ | 5-$OCH_3$ | $CH_3$ | $CH_3$ | |
| $N(CH_3)_2$ | $CH_2CO_2CH_2CH_2OCH_3$ | H | $CH_3$ | $CH_3$ | |
| $N(CH_3)_2$ | $CHCO_2CH_3$ (with $CH_3$ on CH) | H | $CH_3$ | $OCH_3$ | |
| $N(CH_3)_2$ | $CHCO_2CH_2CH=CH_2$ (with $CH_3$ on CH) | H | $OCH_3$ | $OCH_3$ | |
| $N(CH_3)_2$ | $OSO_2CH_3$ | H | $CH_3$ | $OCH_3$ | |
| $N(CH_3)_2$ | $OSO_2CH_2CH_3$ | 3-$CF_3$ | Cl | $OCH_3$ | |
| $N(CH_3)_2$ | $OSO_2CH_2CH_2CH_3$ | H | $CH_3$ | $OCH_3$ | |
| $N(CH_3)_2$ | $OSO_2CF_3$ | H | $OCH_3$ | $OCH_3$ | |
| $N(CH_3)_2$ | $CH_2OCH_3$ | H | $CH_3$ | $OCH_3$ | |
| $N(CH_3)_2$ | $CH_2OCH_2CH_3$ | H | $CH_3$ | $CH_3$ | |
| $N(CH_3)_2$ | $CH_2OCH_2CH_2CH_3$ | H | $OCH_3$ | $OCH_3$ | |
| $N(CH_3)_2$ | $CH_2OCF_2H$ | H | $CH_3$ | $OCH_3$ | |
| $CO_2CH_3$ | $SO_2N(CH_3)_2$ | H | $CH_3$ | $OCH_3$ | |
| $CO_2CH_3$ | $SO_2N(CH_3)OCH_3$ | H | $CH_3$ | $CH_3$ | |
| $CO_2CH_2CH_2Cl$ | $SO_2N(CH_3)_2$ | H | $OCH_3$ | $OCH_3$ | |
| $CO_2CH_3$ | $C_6H_5$ | H | $CH_3$ | $OCH_3$ | |
| $CO_2CH_3$ | 2'-$C_6H_4OCH_3$ | H | $OCH_3$ | $OCH_3$ | |
| $CO_2CH_2CH_2CH_3$ | 3'-$C_6H_4CH_3$ | H | $CH_3$ | $CH_3$ | |
| $CO_2CH_3$ | $CH_2CO_2CH_3$ | H | $CH_3$ | $OCH_3$ | |
| $CO_2CH_2CH_2Cl$ | $CHCO_2CH_3$ (with $CH_3$ on CH) | H | $OCH_3$ | $OCH_3$ | |
| $CO_2CH_3$ | $CH_2OCH_3$ | H | $CH_3$ | $OCH_3$ | |
| $CO_2CH_3$ | $CH_2OCF_2H$ | H | $OCH_3$ | $OCH_3$ | |
| $SO_2N(CH_3)_2$ | $SO_2N(CH_3)_2$ | H | $CH_3$ | $CH_3$ | |
| $SO_2N(CH_3)OCH_3$ | $SO_2N(CH_3)_3$ | H | $CH_3$ | $OCH_3$ | |
| $SO_2N(CH_3)OCH_3$ | $SO_2N(CH_3)OCH_3$ | H | $CH_3$ | $CH_3$ | |
| $SO_2N(CH_3)_2$ | $C_6H_5$ | H | $OCH_3$ | $OCH_3$ | |
| $SO_2N(CH_3)_2$ | 4'-$C_6H_4Cl$ | H | $CH_3$ | $OCH_3$ | |

Table 2a (continued)

| $R_1$ | $R_2$ | $R_3$ | X | Y | m.p. (°C) |
|---|---|---|---|---|---|
| $SO_2N(CH_3)_2$ | 2'-$C_6H_4OCH_3$ | H | $OCH_3$ | $OCH_3$ | |
| $SO_2N(CH_3)_2$ | $CH_2CO_2CH_3$ | H | $CH_3$ | $OCH_3$ | |
| $SO_2N(CH_3)_2$ | $CH(CH_3)CO_2CH_3$ | H | $CH_3$ | $CH_3$ | |
| $SO_2N(CH_3)_2$ | $OSO_2CH_3$ | H | $CH_3$ | $OCH_3$ | |
| $SO_2N(CH_3)_2$ | $OSO_2CF_3$ | H | $OCH_3$ | $OCH_3$ | |
| $C_6H_5$ | $C_6H_5$ | H | $CH_3$ | $OCH_3$ | |
| $C_6H_5$ | 2'-$C_6H_4Cl$ | H | $OCH_3$ | $OCH_3$ | |
| $C_6H_5$ | 3'-$C_6H_4Cl$ | H | $CH_3$ | $OCH_3$ | |
| 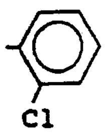 | 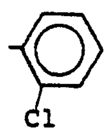 | H | $OCH_3$ | $OCH_3$ | |
| 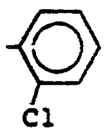 | 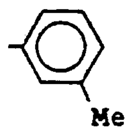 | H | $CH_3$ | $CH_3$ | |
| $C_6H_5$ | $CH_2CO_2CH_3$ | H | $CH_3$ | $OCH_3$ | |
| $C_6H_5$ | $-CH(CH_3)CO_2CH_3$ | H | $OCH_3$ | $OCH_3$ | |
| 2'-$C_6H_4CH_3$ | $CH_2CO_2CH_3$ | H | Cl | $OCH_3$ | |
| $C_6H_5$ | $OSO_2CH_3$ | H | $CH_3$ | $OCH_3$ | |
| $C_6H_5$ | $OSO_2CF_3$ | H | $CH_3$ | $OCH_3$ | |
| 2'-$C_6H_4F$ | $OSO_2CH_2CH_3$ | H | $OCH_3$ | $OCH_3$ | |
| $C_6H_5$ | $CH_2OCH_3$ | H | $CH_3$ | $OCH_3$ | |
| 4'-$C_6H_4Cl$ | $CH_2OCF_2H$ | H | $CH_3$ | $OCH_3$ | |
| $CH_2CO_2CH_3$ | $CH_2CO_2CH_3$ | 3-$CH_3$ | $CH_3$ | $OCH_3$ | |
| $CH_2CO_2CH_3$ | $CH_2CO_2CH_2CH_2Cl$ | H | $CH_3$ | $OCH_3$ | |
| $CH_2CO_2CH_2CH_2OCH_3$ | $CH_2CO_2CH_2CH{=}CH_2$ | H | $CH_3$ | $OCH_3$ | |
| $CH(CH_3)CO_2CH_3$ | $CH_2CO_2CH_3$ | H | $OCH_3$ | $OCH_3$ | |
| $CH(CH_3)CO_2CH_2CH_2Cl$ | $CH_2CO_2CH_3$ | H | $CH_3$ | $CH_3$ | |

Table 2a (continued)

| $R_1$ | $R_2$ | $R_3$ | X | Y | m.p.(°C) |
|---|---|---|---|---|---|
| $CH(CH_3)CO_2CH_3$ | $CH(CH_3)CO_2CH_3$ | H | $OCH_3$ | $CH_3$ | |
| $CH_2CO_2CH_3$ | $OSO_2CH_3$ | H | $OCH_3$ | $OCH_3$ | |
| $CH(CH_3)CO_2CH_3$ | $OSO_2CF_3$ | H | $CH_3$ | $OCH_3$ | |
| $CH_2CO_2CH_3$ | $CH_2OCH_3$ | H | $CH_3$ | $OCH_3$ | |
| $CH(CH_3)CO_2CH_3$ | $CH_2OCF_2H$ | H | $CH_3$ | $CH_3$ | |
| $OSO_2CH_3$ | $OSO_2CH_3$ | H | $CH_3$ | $OCH_3$ | |
| $OSO_2CF_3$ | $OSO_2CH_3$ | H | $CH_3$ | $CH_3$ | |
| $OSO_2CF_3$ | $OSO_2CF_3$ | H | $CH_3$ | $CH_3$ | |
| $OSO_2CH_3$ | $CH_2OCH_3$ | 4-$CF_3$ | $CH_3$ | $OCH_3$ | |
| $OSO_2CF_3$ | $CH_2OCH_3$ | H | $OCH_3$ | $OCH_3$ | |
| $OSO_2CH_3$ | $CH_2OCF_2H$ | H | $CH_3$ | $OCH_3$ | |
| $OSO_2CF_3$ | $CH_2OCF_2H$ | 5-Cl | $CH_3$ | $CH_3$ | |
| $CH_2OCH_3$ | $CH_2OCH_3$ | H | $CH_3$ | $OCH_3$ | |
| $CH_2OCF_2H$ | $CH_2OCH_3$ | H | $OCH_3$ | $OCH_3$ | |
| $CH_2OCF_2H$ | $CH_2OCF_2H$ | H | $CH_3$ | $CH_3$ | |
| $SO_2CH_3$ | $C_6H_5$ | H | $CH_3$ | $CH_3$ | 228-230°d |
| $SO_2CH_3$ | $C_6H_5$ | H | $CH_3$ | $OCH_3$ | 206-208°d |
| $SO_2CH_3$ | $C_6H_5$ | H | $CH_3$ | $CH_2OCH_3$ | |
| $SO_2CH_3$ | $C_6H_5$ | 3-Cl | $CH_3$ | $CH_2OCH_3$ | |
| $SCH_3$ | $SCH_3$ | H | $CH_3$ | $CH_3$ | 188-194° |
| $SCH_3$ | $SCH_3$ | H | $CH_3$ | $OCH_3$ | 206-208° |
| $SCH_3$ | $SCH_3$ | H | $CH_3$ | $NHCH_3$ | |
| $SCH_3$ | $SCH_3$ | H | $CH_3$ | $N(CH_3)_2$ | |
| $SCH_3$ | $SCH_3$ | H | $CH_3$ | $CH_2CH_3$ | |
| $SCH_3$ | $SCH_3$ | H | $CH_3$ | $OCH_2CH_3$ | |
| $SCH_3$ | $SCH_3$ | 5-$CF_3$ | $CH_3$ | $OCH_2CH_3$ | |
| $SCH_3$ | $SCH_3$ | H | $CH_3$ | $SCH_3$ | |

Table 2a (continued)

| $R_1$ | $R_2$ | $R_3$ | X | Y | m.p.(°C) |
|---|---|---|---|---|---|
| $SCH_3$ | $SCH_3$ | 3-Cl | $CH_3$ | $OCH_3$ | |
| $SCH_3$ | $SCH_3$ | 3-$CH_3$ | $CH_3$ | $OCH_3$ | |
| $SO_2CH_3$ | 2'-$C_6H_4Cl$ | H | $CH_3$ | $OCH_3$ | |
| $SO_2CH_3$ | $C_6H_5$ | 3-Cl | $CH_3$ | $OCH_3$ | |
| $SO_2CH_3$ | $C_6H_5$ | 5-Cl | $CH_3$ | $OCH_3$ | |
| $SO_2CH_3$ | $C_6H_5$ | H | $OCH_3$ | $CH_2OCH_3$ | |
| $SO_2CH_3$ | $C_6H_5$ | H | $OCH_3$ | $OCH_3$ | 212-214° |
| $SO_2CH_3$ | $C_6H_5$ | H | $OCH_3$ | $OCH_2CF_3$ | |
| $SCH_3$ | $SCH_3$ | H | $OCH_3$ | $OCH_3$ | 220-225° |
| $SCH_3$ | $SCH_3$ | H | $OCH_3$ | $CH_2OCH_3$ | |
| $SCH_3$ | $SCH_3$ | 3-Cl | $CH_3$ | $OCH_3$ | |
| $SCH_3$ | $SCH_3$ | 3-$OCH_3$ | $OCH_3$ | $OCH_3$ | |
| $SCH_3$ | $SCH_3$ | H | $OCH_3$ | $N(CH_3)_2$ | |
| $SCH_3$ | $SCH_3$ | H | $OCH_3$ | $CH_2CH_3$ | |
| $SCH_3$ | $SCH_3$ | H | $OCH_3$ | $SCH_3$ | |
| $SO_2CH_3$ | $C_6H_5$ | H | Cl | $NHCH_3$ | |
| $SO_2CH_3$ | $C_6H_5$ | H | Cl | $N(CH_3)_2$ | |
| $SO_2CH_3$ | $C_6H_5$ | H | Cl | $OCH_3$ | |
| $SCH_3$ | $SCH_3$ | H | Cl | $OCH_3$ | 179-186° |
| $SCH_3$ | $SCH_3$ | H | Cl | $N(CH_3)_2$ | |
| $SCH_3$ | $SCH_3$ | 3-Cl | $CH_3$ | $OCH_3$ | |
| $SCH_3$ | $SCH_3$ | 3-$CH_3$ | $OCH_3$ | $OCH_3$ | |
| $SCH_3$ | $C_6H_5$ | H | $CH_3$ | $CH_3$ | 218-221°d |
| $SCH_3$ | $C_6H_5$ | H | $CH_3$ | $OCH_3$ | 170-173° |
| $SCH_3$ | $C_6H_5$ | H | $OCH_3$ | $OCH_3$ | 187-189° |

Table 2b

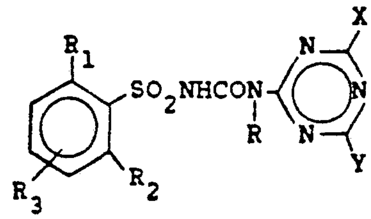

| $R_1$ | $R_2$ | R | $R_3$ | X | Y | m.p. (°C) |
|---|---|---|---|---|---|---|
| $SCH_3$ | $SCH_2CH_3$ | H | H | $CH_3$ | $OCH_3$ | |
| $SCH_2CH_3$ | $SCH_2CH_3$ | H | 3-Cl | $CH_3$ | $OCH_3$ | |
| $SCH_2CH_3$ | $SCH_2CH_2CH_3$ | H | H | $OCH_3$ | $OCH_3$ | |
| $SCH_2CH_2CH_3$ | $SCH_2CH_2CH_3$ | H | H | $CH_3$ | $OCH_3$ | |
| $S(O)CH_3$ | $SCH_3$ | H | H | $CH_3$ | $CH_3$ | |
| $S(O)_2CH_3$ | $SCH_3$ | H | H | $CH_3$ | $OCH_3$ | |
| $S(O)CH_3$ | $S(O)CH_3$ | H | H | $OCH_3$ | $OCH_3$ | |
| $S(O)_2CH_3$ | $S(O)CH_3$ | H | H | $CH_3$ | $OCH_3$ | |
| $SCH_3$ | $N(CH_3)H$ | H | H | $CH_3$ | $OCH_3$ | |
| $S(O)CH_3$ | $N(CH_3)_2$ | H | H | $OCH_3$ | $OCH_3$ | |
| $S(O)_2CH_3$ | $N(CH_3)_2$ | H | H | $CH_3$ | $OCH_3$ | |
| $SCH_3$ | $N(CH_3)CH_2CH_3$ | H | 3-F | $CH_3$ | $OCH_3$ | |
| $SCH_3$ | $N(CH_2CH_3)_2$ | H | H | $OCH_3$ | $OCH_3$ | |
| $CF_3$ | $CF_3$ | H | H | $CH_3$ | $OCH_3$ | |
| $CF_3$ | $CF_3$ | H | 4-Br | $CH_3$ | $CH_3$ | |
| $N(CH_3)_2$ | $N(CH_3)_2$ | H | H | $CH_3$ | $OCH_3$ | |
| $N(CH_3)CH_2CH_3$ | $N(CH_3)_2$ | H | H | $OCH_3$ | $OCH_3$ | |
| $N(CH_2CH_3)_2$ | $N(CH_3)_2$ | H | 4-$CH_3$ | $CH_3$ | $OCH_3$ | |
| $N(CH_3)CH_2CH_3$ | $N(CH_3)CH_2CH_3$ | H | H | $OCH_3$ | $OCH_3$ | |
| $N(CH_2CH_3)_2$ | $N(CH_2CH_3)_2$ | H | H | $CH_3$ | $OCH_3$ | |
| $N(CH_3)_2$ | $C_6H_5$ | H | H | $CH_3$ | $OCH_3$ | |
| $N(CH_3)_2$ | 2'-$C_6H_4Cl$ | H | H | $CH_3$ | $OCH_3$ | |
| $N(CH_3)_2$ | $CH_2CO_2CH_3$ | H | H | $CH_3$ | $CH_3$ | |
| $N(CH_3)_2$ | $CH_2CO_2CH_2CH_3$ | H | H | $CH_3$ | $OCH_3$ | |

Table 2b (continued)

| $R_1$ | $R_2$ | R | $R_3$ | X | Y | m.p. (°C) |
|---|---|---|---|---|---|---|
| $N(CH_3)_2$ | $CH_2CO_2CH_2CH_2CH_3$ | H | H | $OCH_3$ | $OCH_3$ | |
| $N(CH_3)_2$ | $CH_2CO_2CH_2CH{=}CH_2$ | H | H | $OCH_3$ | $OCH_3$ | |
| $N(CH_3)_2$ | $CH_2CO_2CH_2CH_2Cl$ | H | 5-$OCH_3$ | $CH_3$ | $CH_3$ | |
| $N(CH_3)_2$ | $CH_2CO_2CH_2CH_2OCH_3$ | H | H | $CH_3$ | $CH_3$ | |
| $N(CH_3)_2$ | $CHCO_2CH_3$<br>$CH_3$ | H | H | $CH_3$ | $OCH_3$ | |
| $N(CH_3)_2$ | $CHCO_2CH_2CH{=}CH_2$<br>$CH_3$ | H | H | $OCH_3$ | $OCH_3$ | |
| $N(CH_3)_2$ | $OSO_2CH_3$ | H | H | $CH_3$ | $OCH_3$ | |
| $N(CH_3)_2$ | $OSO_2CH_2CH_3$ | H | 3-$CF_3$ | $CH_3$ | $OCH_3$ | |
| $N(CH_3)_2$ | $OSO_2CH_2CH_2CH_3$ | H | H | $CH_3$ | $OCH_3$ | |
| $N(CH_3)_2$ | $OSO_2CF_3$ | H | H | $OCH_3$ | $OCH_3$ | |
| $N(CH_3)_2$ | $CH_2OCH_3$ | H | H | $CH_3$ | $OCH_3$ | |
| $N(CH_3)_2$ | $CH_2OCH_2CH_3$ | H | H | $CH_3$ | $CH_3$ | |
| $N(CH_3)_2$ | $CH_2OCH_2CH_2CH_3$ | H | H | $OCH_3$ | $OCH_3$ | |
| $N(CH_3)_2$ | $CH_2OCF_2H$ | H | H | $CH_3$ | $OCH_3$ | |
| $CO_2CH_3$ | $SO_2N(CH_3)_2$ | H | H | $CH_3$ | $OCH_3$ | |
| $CO_2CH_3$ | $SO_2N(CH_3)OCH_3$ | H | H | $CH_3$ | $CH_3$ | |
| $CO_2CH_2CH_2Cl$ | $SO_2N(CH_3)_2$ | H | H | $OCH_3$ | $OCH_3$ | |
| $CO_2CH_3$ | $C_6H_5$ | H | H | $CH_3$ | $OCH_3$ | |
| $CO_2CH_3$ | 2'-$C_6H_4OCH_3$ | H | H | $OCH_3$ | $OCH_3$ | |
| $CO_2CH_2CH_2CH_3$ | 3'-$C_6H_4CH_3$ | H | H | $CH_3$ | $CH_3$ | |
| $CO_2CH_3$ | $CH_2CO_2CH_3$ | H | H | $CH_3$ | $OCH_3$ | |
| $CO_2CH_2CH_2Cl$ | $CHCO_2CH_3$<br>$CH_3$ | H | H | $OCH_3$ | $OCH_3$ | |
| $CO_2CH_3$ | $CH_2OCH_3$ | H | H | $CH_3$ | $OCH_3$ | |
| $CO_2CH_3$ | $CH_2OCF_2H$ | H | H | $OCH_3$ | $OCH_3$ | |
| $SO_2N(CH_3)_2$ | $SO_2N(CH_3)_2$ | H | H | $CH_3$ | $CH_3$ | |
| $SO_2N(CH_3)OCH_3$ | $SO_2N(CH_3)_3$ | H | H | $CH_3$ | $OCH_3$ | |
| $SO_2N(CH_3)OCH_3$ | $SO_2N(CH_3)OCH_3$ | H | H | $CH_3$ | $CH_3$ | |
| $SO_2N(CH_3)_2$ | $C_6H_5$ | H | H | $OCH_3$ | $OCH_3$ | |
| $SO_2N(CH_3)_2$ | 4'-$C_6H_4Cl$ | H | H | $CH_3$ | $OCH_3$ | |

Table 2b (continued)

| $R_1$ | $R_2$ | R | $R_3$ | X | Y | m.p. (°C) |
|---|---|---|---|---|---|---|
| $SO_2N(CH_3)_2$ | 2'-$C_6H_4OCH_3$ | H | H | $OCH_3$ | $OCH_3$ | |
| $SO_2N(CH_3)_2$ | $CH_2CO_2CH_3$ | H | H | $CH_3$ | $OCH_3$ | |
| $SO_2N(CH_3)_2$ | $CHCO_2CH_3$<br>$CH_3$ | H | H | $CH_3$ | $CH_3$ | |
| $SO_2N(CH_3)_2$ | $OSO_2CH_3$ | $CH_3$ | H | $CH_3$ | $OCH_3$ | |
| $SO_2N(CH_3)_2$ | $OSO_2CF_3$ | H | H | $OCH_3$ | $OCH_3$ | |
| $C_6H_5$ | $C_6H_5$ | H | H | $CH_3$ | $OCH_3$ | |
| $C_6H_5$ | 2'-$C_6H_4Cl$ | H | H | $OCH_3$ | $OCH_3$ | |
| $C_6H_5$ | 3'-$C_6H_4Cl$ | H | H | $CH_3$ | $OCH_3$ | |
| 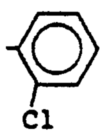 | 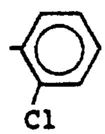 | H | H | $OCH_3$ | $OCH_3$ | |
| 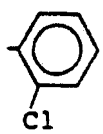 | 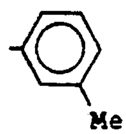 | H | H | $CH_3$ | $CH_3$ | |
| $C_6H_5$ | $CH_2CO_2CH_3$ | H | H | $CH_3$ | $OCH_3$ | |
| $C_6H_5$ | $-CHCO_2CH_3$<br>$CH_3$ | H | H | $OCH_3$ | $OCH_3$ | |
| 2'-$C_6H_4CH_3$ | $CH_2CO_2CH_3$ | H | H | $CH_3$ | $OCH_3$ | |
| $C_6H_5$ | $OSO_2CH_3$ | H | H | $CH_3$ | $OCH_3$ | |
| $C_6H_5$ | $OSO_2CF_3$ | H | H | $CH_3$ | $OCH_3$ | |
| 2'-$C_6H_4F$ | $OSO_2CH_2CH_3$ | H | H | $OCH_3$ | $OCH_3$ | |
| $C_6H_5$ | $CH_2OCH_3$ | $CH_3$ | H | $CH_3$ | $OCH_3$ | |
| 4'-$C_6H_4Cl$ | $CH_2OCF_2H$ | H | H | $CH_3$ | $OCH_3$ | |
| $CH_2CO_2CH_3$ | $CH_2CO_2CH_3$ | H | 3-$CH_3$ | $CH_3$ | $OCH_3$ | |
| $CH_2CO_2CH_3$ | $CH_2CO_2CH_2CH_2Cl$ | H | H | $CH_3$ | $OCH_3$ | |
| $CH_2CO_2CH_2CH_2OCH_3$ | $CH_2CO_2CH_2CH=CH_2$ | H | H | $CH_3$ | $OCH_3$ | |
| $CHCO_2CH_3$<br>$CH_3$ | $CH_2CO_2CH_3$ | H | H | $OCH_3$ | $OCH_3$ | |
| $CHCO_2CH_2CH_2Cl$<br>$CH_3$ | $CH_2CO_2CH_3$ | H | H | $CH_3$ | $CH_3$ | |

Table 2b (continued)

| $R_1$ | $R_2$ | R | $R_3$ | X | Y | m.p. (°C) |
|---|---|---|---|---|---|---|
| $CHCO_2CH_3$ <br> $CH_3$ | $CHCO_2CH_3$ <br> $CH_3$ | H | H | $OCH_3$ | $CH_3$ | |
| $CH_2CO_2CH_3$ | $OSO_2CH_3$ | H | H | $OCH_3$ | $OCH_3$ | |
| $CHCO_2CH_3$ <br> $CH_3$ | $OSO_2CF_3$ | H | H | $CH_3$ | $OCH_3$ | |
| $CH_2CO_2CH_3$ | $CH_2OCH_3$ | H | H | $CH_3$ | $OCH_3$ | |
| $CHCO_2CH_3$ <br> $CH_3$ | $CH_2OCF_2H$ | H | H | $CH_3$ | $CH_3$ | |
| $OSO_2CH_3$ | $OSO_2CH_3$ | H | H | $CH_3$ | $OCH_3$ | |
| $OSO_2CF_3$ | $OSO_2CH_3$ | H | H | $CH_3$ | $CH_3$ | |
| $OSO_2CF_3$ | $OSO_2CF_3$ | H | H | $CH_3$ | $CH_3$ | |
| $OSO_2CH_3$ | $CH_2OCH_3$ | H | 4-$CF_3$ | $CH_3$ | $OCH_3$ | |
| $OSO_2CF_3$ | $CH_2OCH_3$ | H | H | $OCH_3$ | $OCH_3$ | |
| $OSO_2CH_3$ | $CH_2OCF_2H$ | H | H | $CH_3$ | $OCH_3$ | |
| $OSO_2CF_3$ | $CH_2OCF_2H$ | H | 5-Cl | $CH_3$ | $CH_3$ | |
| $CH_2OCH_3$ | $CH_2OCH_3$ | H | H | $CH_3$ | $OCH_3$ | |
| $CH_2OCF_2H$ | $CH_2OCH_3$ | H | H | $OCH_3$ | $OCH_3$ | |
| $CH_2OCF_2H$ | $CH_2OCF_2H$ | H | H | $CH_3$ | $CH_3$ | |
| $SO_2CH_3$ | $C_6H_5$ | H | H | $CH_3$ | $CH_3$ | |
| $SO_2CH_3$ | $C_6H_5$ | H | H | $CH_3$ | $OCH_3$ | 204-208°d |
| $SO_2CH_3$ | $C_6H_5$ | H | H | $CH_3$ | $CH_2OCH_3$ | |
| $SO_2CH_3$ | $C_6H_5$ | H | 3-Cl | $CH_3$ | $CH_2OCH_3$ | |
| $SO_2CH_3$ | $C_6H_5$ | H | H | $CH_3$ | $OCH_2CF_3$ | |
| $SCH_3$ | $SCH_3$ | H | H | $CH_3$ | $CH_3$ | 179-193° |
| $SCH_3$ | $SCH_3$ | H | H | $CH_3$ | $OCH_3$ | |
| $SCH_3$ | $SCH_3$ | H | H | $CH_3$ | $NHCH_3$ | |
| $SCH_3$ | $SCH_3$ | H | H | $CH_3$ | $N(CH_3)_2$ | |
| $SCH_3$ | $SCH_3$ | H | H | $CH_3$ | $CH_2CH_3$ | |
| $SCH_3$ | $SCH_3$ | H | H | $CH_3$ | $OCH_2CH_3$ | |
| $SCH_3$ | $SCH_3$ | H | 5-$CF_3$ | $CH_3$ | $OCH_2CH_3$ | |
| $SCH_3$ | $SCH_3$ | H | H | $CH_3$ | $SCH_3$ | |

Table 2b (continued)

| $R_1$ | $R_2$ | R | $R_3$ | X | Y | m.p. (°C) |
|---|---|---|---|---|---|---|
| $SCH_3$ | $SCH_3$ | H | 3-Cl | $CH_3$ | $OCH_3$ | |
| $SCH_3$ | $SCH_3$ | H | 3-$CH_3$ | $CH_3$ | $OCH_3$ | |
| $SO_2CH_3$ | 2'-$C_6H_4Cl$ | H | H | $CH_3$ | $OCH_3$ | |
| $SO_2CH_3$ | $C_6H_5$ | H | 3-Cl | $CH_3$ | $OCH_3$ | |
| $SO_2CH_3$ | $C_6H_5$ | H | 5-Cl | $CH_3$ | $OCH_3$ | |
| $SO_2CH_3$ | $C_6H_5$ | H | H | $OCH_3$ | $CH_2OCH_3$ | |
| $SO_2CH_3$ | $C_6H_5$ | H | H | $OCH_3$ | $OCH_3$ | 216-220°d |
| $SO_2CH_3$ | $C_6H_5$ | H | H | $OCH_3$ | $OCH_2CF_3$ | |
| $SCH_3$ | $SCH_3$ | H | H | $OCH_3$ | $OCH_3$ | 121-132° |
| $SCH_3$ | $SCH_3$ | H | H | $OCH_3$ | $CH_2OCH_3$ | |
| $SCH_3$ | $SCH_3$ | H | 3-Cl | $CH_3$ | $OCH_3$ | |
| $SCH_3$ | $SCH_3$ | H | 3-$OCH_3$ | $OCH_3$ | $OCH_3$ | |
| $SCH_3$ | $SCH_3$ | H | H | $OCH_3$ | $N(CH_3)_2$ | |
| $SCH_3$ | $SCH_3$ | H | H | $OCH_3$ | $CH_2CH_3$ | |
| $SCH_3$ | $SCH_3$ | H | H | $OCH_3$ | $SCH_3$ | |
| $SCH_3$ | $SCH_3$ | H | 3-Cl | $CH_3$ | $OCH_3$ | |
| $SCH_3$ | $SCH_3$ | H | 3-$CH_3$ | $OCH_3$ | $OCH_3$ | |
| $SCH_3$ | $C_6H_5$ | H | H | $OCH_3$ | $CH_3$ | 130-135°d |

Table 3

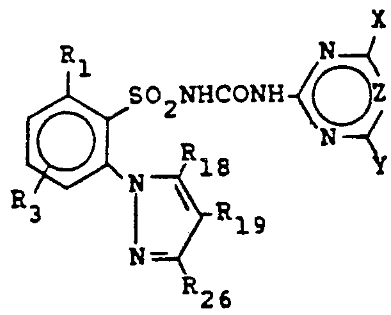

Z = a.) N or b.) CH

| $R_1$ | $R_3$ | $R_{18}$ | $R_{19}$ | $R_{26}$ | X | Y |
|---|---|---|---|---|---|---|
| $SCH_3$ | H | H | H | H | $CH_3$ | $OCH_3$ |
| $SCH_3$ | H | H | H | H | $OCH_3$ | $OCH_3$ |
| $SCH_3$ | H | $CH_3$ | H | H | $CH_3$ | $OCH_3$ |
| $SCH_3$ | H | H | H | $CH_3$ | $OCH_3$ | $OCH_3$ |
| $SCH_3$ | H | $CH_3$ | $CH_3$ | $CH_3$ | $CH_3$ | $CH_3$ |
| $SCH_2CH_3$ | H | H | H | H | $CH_3$ | $OCH_3$ |
| $SCH_2CH_2CH_3$ | H | H | H | H | $CH_3$ | $OCH_3$ |
| $S(O)CH_3$ | H | H | H | H | $CH_3$ | $CH_3$ |
| $S(O)CH_3$ | H | H | H | H | $CH_3$ | $OCH_3$ |
| $S(O)CH_3$ | H | $CH_3$ | H | H | Cl | $OCH_3$ b |
| $S(O)CH_3$ | H | H | H | $CH_3$ | $CH_3$ | $OCH_3$ |
| $S(O)_2CH_3$ | H | H | H | H | $OCH_3$ | $OCH_3$ |
| $S(O)_2CH_3$ | H | $CH_3$ | H | H | $CH_3$ | $OCH_3$ |
| $S(O)_2CH_3$ | H | H | H | $CH_3$ | $OCH_3$ | $OCH_3$ |
| $CF_3$ | H | H | H | H | $CH_3$ | $CH_3$ |
| $CF_3$ | H | $CH_3$ | H | H | $OCH_3$ | $CH_3$ |
| $CF_3$ | H | H | $CH_3$ | H | $OCH_3$ | $OCH_3$ |
| $CF_3$ | H | $CH_3$ | $CH_3$ | $CH_3$ | $CH_3$ | $OCH_3$ |
| $N(CH_3)_2$ | H | H | H | H | $CH_3$ | $OCH_3$ |
| $N(CH_3)_2$ | H | H | H | H | $OCH_3$ | $OCH_3$ |
| $N(CH_3)_2$ | H | $CH_3$ | H | H | $CH_3$ | $OCH_3$ |
| $N(CH_3)_2$ | H | H | $CH_3$ | H | $OCH_3$ | $OCH_3$ |

Table 3 (continued)

| $R_1$ | $R_3$ | $R_{18}$ | $R_{19}$ | $R_{26}$ | X | Y |
|---|---|---|---|---|---|---|
| $N(CH_3)CH_2CH_3$ | H | H | H | H | $CH_3$ | $OCH_3$ |
| $N(CH_2CH_3)_2$ | H | H | H | H | $OCH_3$ | $OCH_3$ |
| $CO_2CH_3$ | H | H | H | H | $CH_3$ | $OCH_3$ |
| $CO_2CH_3$ | H | $CH_3$ | H | H | $OCH_3$ | $OCH_3$ |
| $CO_2CH_3$ | H | H | $CH_3$ | $CH_3$ | $CH_3$ | $CH_3$ |
| $CO_2CH_2CH_2Cl$ | H | H | H | H | $CH_3$ | $OCH_3$ |
| $SO_2N(CH_3)_2$ | H | H | H | H | $OCH_3$ | $OCH_3$ |
| $SO_2N(CH_3)_2$ | H | $CH_3$ | H | $CH_3$ | $CH_3$ | $OCH_3$ |
| $C_6H_5$ | H | H | H | H | $CH_3$ | $OCH_3$ |
| $C_6H_5$ | H | H | H | H | $OCH_3$ | $OCH_3$ |
| $C_6H_5$ | H | $CH_3$ | H | $CH_3$ | Cl | $OCH_3$[b] |
| $C_6H_5$ | H | H | $CH_3$ | H | $CH_3$ | $OCH_3$ |
| 2'-$C_6H_4Cl$ | H | H | H | H | $OCH_3$ | $OCH_3$ |
| 2'-$C_6H_4CH_3$ | H | H | H | H | $OCF_2H$ | $OCH_3$ |
| 2'-$C_6H_4OCH_3$ | H | H | H | H | $CH_3$ | $OCH_3$ |
| 3'-$C_6H_4F$ | H | $CH_3$ | H | $CH_3$ | $OCH_3$ | $OCH_3$ |
| 4'-$C_6H_4Br$ | H | $CH_3$ | $CH_3$ | $CH_3$ | Cl | $OCH_3$[b] |
| $CH_2CO_2CH_3$ | H | H | H | H | $CH_3$ | $OCH_3$ |
| $CH_2CO_2CH_3$ | H | $CH_3$ | H | H | $OCH_3$ | $OCH_3$ |
| $CH_2CO_2CH_3$ | H | H | $CH_3$ | H | Cl | $OCH_3$[b] |
| $CH(CH_3)CO_2CH_3$ | H | H | H | H | $OCH_3$ | $OCF_2H$ |
| $OSO_2CH_3$ | H | H | H | H | $CH_3$ | $OCH_3$ |
| $OSO_2CH_3$ | H | $CH_3$ | H | H | $OCH_3$ | $OCH_3$ |
| $OSO_2CH_3$ | H | H | $CH_3$ | $CH_3$ | Cl | $OCH_3$[b] |
| $OSO_2CH_3$ | H | $CH_3$ | $CH_3$ | H | $OCH_3$ | $OCF_2H$ |
| $OSO_2CF_3$ | H | H | H | H | $CH_3$ | $OCH_3$ |
| $CH_2OCH_3$ | H | H | H | H | $CH_3$ | $OCH_3$ |
| $CH_2OCH_3$ | H | $CH_3$ | H | H | $OCH_3$ | $OCH_3$ |
| $CH_2OCH_3$ | H | H | $CH_3$ | H | $OCH_3$ | $OCF_2H$ |

Table 3 (continued)

| $R_1$ | $R_3$ | $R_{18}$ | $R_{19}$ | $R_{26}$ | X | Y |
|---|---|---|---|---|---|---|
| $CH_2OCH_3$ | H | $CH_3$ | $CH_3$ | $CH_3$ | $CH_3$ | $CH_3$ |
| $CH_2OCF_2H$ | H | H | H | H | $OCH_3$ | $OCH_3$ |
| $CH_2OCF_2H$ | H | $CH_3$ | H | $CH_3$ | $OCH_3$ | $OCF_2H$ |
| $CH_2OCF_2H$ | H | H | $CH_3$ | H | Cl | $OCH_3$[b] |
| $CH_2OCF_2H$ | H | $CH_3$ | $CH_3$ | H | $CH_3$ | $OCH_3$ |

Table 4

a.) Z = N

b.) Z = CH

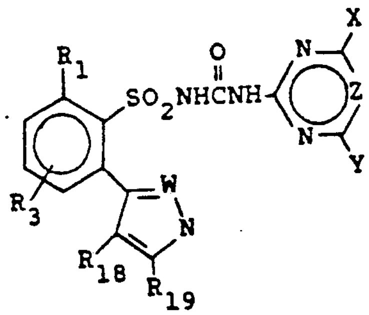

c.) Z = N

d.) Z = CH

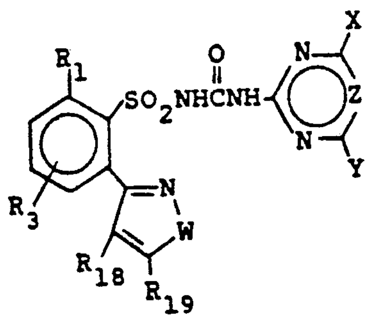

e.) Z = N

f.) Z = CH

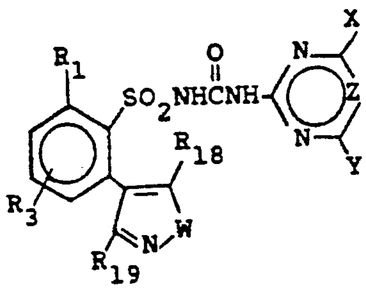

| $R_1$ | $R_3$ | W | $R_{18}$ | $R_{19}$ | X | Y |
|---|---|---|---|---|---|---|
| $SCH_3$ | 3-$CH_3$ | O | $CH_3$ | $CH_3$ | $CH_3$ | $OCH_3$ |
| $N(CH_3)_2$ | H | O | H | H | $CH_3$ | $OCH_3$ |
| $N(CH_3)_2$ | H | O | H | H | $OCH_3$ | $OCH_3$ |
| $N(CH_3)_2$ | H | O | $CH_3$ | H | $CH_3$ | $OCH_3$ |

Table 4 (continued)

| $R_1$ | $R_3$ | W | $R_{18}$ | $R_{19}$ | X | Y |
|---|---|---|---|---|---|---|
| $N(CH_3)_2$ | H | O | H | $CH_3$ | $OCH_3$ | $OCH_3$ |
| $N(CH_3)CH_2CH_3$ | H | O | H | H | $CH_3$ | $OCH_3$ |
| $N(CH_2CH_3)_2$ | H | O | H | H | $OCH_3$ | $OCH_3$ |
| $SO_2N(CH_3)_2$ | H | O | H | H | $OCH_3$ | $OCH_3$ |
| $SO_2N(CH_3)_2$ | H | O | $CH_3$ | H | $CH_3$ | $OCH_3$ |
| $C_6H_5$ | H | O | H | H | $CH_3$ | $CH_3$ |
| $C_6H_5$ | H | O | H | H | $CH_3$ | $OCH_3$ |
| $C_6H_5$ | H | O | H | H | $OCH_3$ | $OCH_3$ |
| $C_6H_5$ | H | O | $CH_3$ | H | $CH_3$ | $OCH_3$ |
| $C_6H_5$ | H | O | H | $CH_3$ | $OCH_3$ | $OCH_3$ |
| $C_6H_5$ | H | O | $CH_3$ | $CH_3$ | $OCH_3$ | $OCF_2H$ |
| $2'-C_6H_4Cl$ | H | O | H | H | $CH_3$ | $OCH_3$ |
| $2'-C_6H_4OCH_3$ | H | O | H | H | $OCH_3$ | $OCH_3$ |
| $2'-C_6H_4CH_3$ | H | O | H | H | Cl[b,d,f] | $OCH_3$ |
| $3'-C_6H_4Br$ | H | O | H | H | $CH_3$ | $OCH_3$ |
| $4'-C_6H_4F$ | H | O | H | H | $OCH_3$ | $OCH_3$ |
| $CH_2CO_2CH_3$ | H | O | H | H | $CH_3$ | $OCH_3$ |
| $CH_2CO_2CH_3$ | H | O | $CH_3$ | H | $OCH_3$ | $OCH_3$ |
| $CH_2CO_2CH_3$ | H | O | H | $CH_3$ | Cl[b,d,f] | $OCH_3$ |
| $CH(CH_3)CO_2CH_3$ | H | O | H | H | $OCH_3$ | $OCF_2H$ |
| $OSO_2CH_3$ | H | O | H | H | $CH_3$ | $OCH_3$ |
| $OSO_2CH_3$ | H | O | $CH_3$ | H | $OCH_3$ | $OCH_3$ |
| $OSO_2CH_3$ | H | O | H | $CH_3$ | Cl[b,d,f] | $OCH_3$ |
| $OSO_2CH_3$ | H | O | $CH_3$ | $CH_3$ | $OCH_3$ | $OCF_2H$ |
| $OSO_2CF_3$ | H | O | H | H | $CH_3$ | $OCH_3$ |

Table 4 (continued)

| $R_1$ | $R_3$ | W | $R_{18}$ | $R_{19}$ | X | Y |
|---|---|---|---|---|---|---|
| $CH_2OCH_3$ | H | O | H | H | $CH_3$ | $OCH_3$ |
| $CH_2OCH_3$ | H | O | $CH_3$ | H | $OCH_3$ | $OCH_3$ |
| $CH_2OCH_3$ | H | O | H | $CH_3$ | $OCH_3$ | $OCF_2H$ |
| $CH_2OCH_3$ | H | O | $CH_3$ | $CH_3$ | $CH_3$ | $CH_3$ |
| $CH_2OCF_2H$ | H | O | H | H | $OCH_3$ | $OCH_3$ |
| $CH_2OCF_2H$ | H | O | $CH_3$ | H | $OCH_3$ | $OCF_2H$ |
| $CH_2OCF_2H$ | H | O | H | $CH_3$ | Cl[b,d,f] | $OCH_3$ |
| $CH_2OCF_2H$ | H | O | $CH_3$ | $CH_3$ | $CH_3$ | $OCH_3$ |
| $SCH_3$ | $3\text{-}OCH_3$ | $NCH_3$ | $CH_3$ | $CH_3$ | $CH_3$ | $OCH_3$ |
| $N(CH_3)_2$ | H | $NCH_3$ | H | H | $CH_3$ | $OCH_3$ |
| $N(CH_3)_2$ | H | $NCH_3$ | H | H | $OCH_3$ | $OCH_3$ |
| $N(CH_3)_2$ | H | $NCH_3$ | $CH_3$ | H | $CH_3$ | $OCH_3$ |
| $N(CH_3)_2$ | H | $NCH_3$ | H | $CH_3$ | $OCH_3$ | $OCH_3$ |
| $N(CH_3)CH_2CH_3$ | H | $NCH_3$ | H | H | $CH_3$ | $OCH_3$ |
| $N(CH_2CH_3)_2$ | H | $NCH_3$ | H | H | $OCH_3$ | $OCH_3$ |
| $SO_2N(CH_3)_2$ | H | $NCH_3$ | H | H | $OCH_3$ | $OCH_3$ |
| $SO_2N(CH_3)_2$ | H | NH | $CH_3$ | H | $CH_3$ | $OCH_3$ |
| $C_6H_5$ | H | $NCH_3$ | H | H | $CH_3$ | $CH_3$ |
| $C_6H_5$ | H | $NCH_3$ | H | H | $CH_3$ | $OCH_3$ |
| $C_6H_5$ | H | $NCH_3$ | H | H | $OCH_3$ | $OCH_3$ |
| $C_6H_5$ | H | $NCH_3$ | $CH_3$ | H | $CH_3$ | $OCH_3$ |
| $C_6H_5$ | H | $NCH_3$ | H | $CH_3$ | $OCH_3$ | $OCH_3$ |
| $C_6H_5$ | H | $NCH_3$ | $CH_3$ | $CH_3$ | $OCH_3$ | $OCF_2H$ |
| $2'\text{-}C_6H_4Cl$ | H | $NCH_3$ | H | H | $CH_3$ | $OCH_3$ |
| $2'\text{-}C_6H_4OCH_3$ | H | $NCH_3$ | H | H | $OCH_3$ | $OCH_3$ |
| $2'\text{-}C_6H_4CH_3$ | H | $NCH_3$ | H | H | Cl[b,d,f] | $OCH_3$ |
| $3'\text{-}C_6H_4Br$ | H | NH | H | H | $CH_3$ | $OCH_3$ |
| $4'\text{-}C_6H_4F$ | H | $NCH_3$ | H | H | $OCH_3$ | $OCH_3$ |
| $CH_2CO_2CH_3$ | H | $NCH_3$ | H | H | $CH_3$ | $OCH_3$ |
| $CH_2CO_2CH_3$ | H | $NCH_3$ | $CH_3$ | H | $OCH_3$ | $OCH_3$ |

Table 4 (continued)

| $R_1$ | $R_3$ | W | $R_{18}$ | $R_{19}$ | X | Y |
|---|---|---|---|---|---|---|
| $CH_2CO_2CH_3$ | H | $NCH_3$ | H | $CH_3$ | Cl[b,d,f] | $OCH_3$ |
| $CHCO_2CH_3$ (with $CH_3$ on CH) | H | $NCH_3$ | H | H | $OCH_3$ | $OCF_2H$ |
| $OSO_2CH_3$ | H | $NCH_3$ | H | H | $CH_3$ | $OCH_3$ |
| $OSO_2CH_3$ | H | $NCH_3$ | $CH_3$ | H | $OCH_3$ | $OCH_3$ |
| $OSO_2CH_3$ | H | $NCH_3$ | H | $CH_3$ | Cl[b,d,f] | $OCH_3$ |
| $OSO_2CH_3$ | H | $NCH_3$ | $CH_3$ | $CH_3$ | $OCH_3$ | $OCF_2H$ |
| $OSO_2CF_3$ | H | $NCH_3$ | H | H | $CH_3$ | $OCH_3$ |
| $CH_2OCH_3$ | H | $NCH_3$ | H | H | $CH_3$ | $OCH_3$ |
| $CH_2OCH_3$ | H | $NCH_3$ | $CH_3$ | H | $OCH_3$ | $OCH_3$ |
| $CH_2OCH_3$ | H | NH | H | $CH_3$ | $OCH_3$ | $OCF_2H$ |
| $CH_2OCH_3$ | H | $NCH_3$ | $CH_3$ | $CH_3$ | $CH_3$ | $CH_3$ |
| $CH_2OCF_2H$ | H | $NCH_3$ | H | H | $OCH_3$ | $OCH_3$ |
| $CH_2OCF_2H$ | H | $NCH_3$ | $CH_3$ | H | $OCH_3$ | $OCF_2H$ |
| $CH_2OCF_2H$ | H | $NCH_3$ | H | $CH_3$ | $OCF_2H$ | $OCH_3$ |
| $CH_2OCF_2H$ | H | $NCH_3$ | $CH_3$ | $CH_3$ | $CH_3$ | $OCH_3$ |
| $SCH_3$ | 3-Cl | S | $CH_3$ | $CH_3$ | $CH_3$ | $OCH_3$ |
| $N(CH_3)_2$ | H | S | H | H | $CH_3$ | $OCH_3$ |
| $N(CH_3)_2$ | H | S | H | H | $OCH_3$ | $OCH_3$ |
| $N(CH_3)_2$ | H | S | $CH_3$ | H | $CH_3$ | $OCH_3$ |
| $N(CH_3)_2$ | H | S | H | $CH_3$ | $OCH_3$ | $OCH_3$ |
| $N(CH_3)CH_2CH_3$ | H | S | H | H | $CH_3$ | $OCH_3$ |
| $N(CH_2CH_3)_2$ | H | S | H | H | $OCH_3$ | $OCH_3$ |
| $SO_2N(CH_3)_2$ | H | S | H | H | $OCH_3$ | $OCH_3$ |
| $SO_2N(CH_3)_2$ | H | S | $CH_3$ | H | $CH_3$ | $OCH_3$ |
| $C_6H_5$ | H | S | H | H | $CH_3$ | $CH_3$ |
| $C_6H_5$ | H | S | H | H | $CH_3$ | $OCH_3$ |
| $C_6H_5$ | H | S | H | H | $OCH_3$ | $OCH_3$ |
| $C_6H_5$ | H | S | $CH_3$ | H | $CH_3$ | $OCH_3$ |
| $C_6H_5$ | H | S | H | $CH_3$ | $OCH_3$ | $OCH_3$ |
| $C_6H_5$ | H | S | $CH_3$ | $CH_3$ | $OCH_3$ | $OCF_2H$ |

Table 4 (continued)

| $R_1$ | $R_3$ | W | $R_{18}$ | $R_{19}$ | X | Y |
|---|---|---|---|---|---|---|
| 2'-$C_6H_4Cl$ | H | S | H | H | $CH_3$ | $OCH_3$ |
| 2'-$C_6H_4OCH_3$ | H | S | H | H | $OCH_3$ | $OCH_3$ |
| 2'-$C_6H_4CH_3$ | H | S | H | H | Cl[b,d,f] | $OCH_3$ |
| 3'-$C_6H_4Br$ | H | S | H | H | $CH_3$ | $OCH_3$ |
| 4'-$C_6H_4F$ | H | S | H | H | $OCH_3$ | $OCH_3$ |
| $CH_2CO_2CH_3$ | H | S | H | H | $CH_3$ | $OCH_3$ |
| $CH_2CO_2CH_3$ | H | S | $CH_3$ | H | $OCH_3$ | $OCH_3$ |
| $CH_2CO_2CH_3$ | H | S | H | $CH_3$ | $OCF_2H$ | $OCH_3$ |
| $CHCO_2CH_3$ (with $CH_3$ branch) | H | S | H | H | $OCH_3$ | $OCF_2H$ |
| $OSO_2CH_3$ | H | S | H | H | $CH_3$ | $OCH_3$ |
| $OSO_2CH_3$ | H | S | $CH_3$ | H | $OCH_3$ | $OCH_3$ |
| $OSO_2CH_3$ | H | S | H | $CH_3$ | Cl[b,d,f] | $OCH_3$ |
| $OSO_2CH_3$ | H | S | $CH_3$ | $CH_3$ | $OCH_3$ | $OCF_2H$ |
| $OSO_2CF_3$ | H | S | H | H | $CH_3$ | $OCH_3$ |
| $CH_2OCH_3$ | H | S | H | H | $CH_3$ | $OCH_3$ |
| $CH_2OCH_3$ | H | S | $CH_3$ | H | $OCH_3$ | $OCH_3$ |
| $CH_2OCH_3$ | H | S | H | $CH_3$ | $OCH_3$ | $OCF_2H$ |
| $CH_2OCH_3$ | H | S | $CH_3$ | $CH_3$ | $CH_3$ | $CH_3$ |
| $CH_2OCF_2H$ | H | S | H | H | $OCH_3$ | $OCH_3$ |
| $CH_2OCF_2H$ | H | S | $CH_3$ | H | $OCH_3$ | $OCF_2H$ |
| $CH_2OCF_2H$ | H | S | H | $CH_3$ | Cl[b,d,f] | $OCH_3$ |
| $CH_2OCF_2H$ | H | S | $CH_3$ | $CH_3$ | $CH_3$ | $OCH_3$ |

Table 5

a.) Z = N

b.) Z = CH

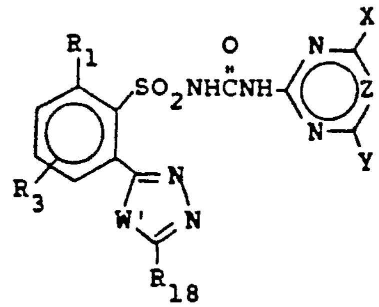

c.) Z = N

d.) Z = CH

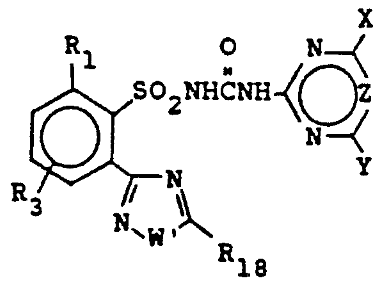

e.) Z = N

f.) Z = CH

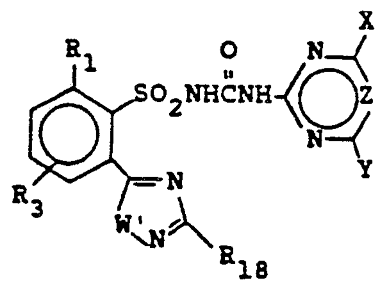

| $R_1$ | $R_3$ | W' | $R_{18}$ | X | Y |
|---|---|---|---|---|---|
| $N(CH_3)_2$ | H | O | H | $CH_3$ | $OCH_3$ |
| $N(CH_3)_2$ | H | O | H | $OCH_3$ | $OCH_3$ |
| $N(CH_3)_2$ | H | O | $CH_3$ | $CH_3$ | $OCH_3$ |
| $N(CH_3)CH_2CH_3$ | H | O | H | $CH_3$ | $OCH_3$ |
| $N(CH_2CH_3)_2$ | H | O | H | $OCH_3$ | $OCH_3$ |
| $SO_2N(CH_3)_2$ | H | O | H | $OCH_3$ | $OCH_3$ |
| $SO_2N(CH_3)_2$ | H | O | $CH_3$ | $CH_3$ | $OCH_3$ |

Table 5 (continued)

| $R_1$ | $R_3$ | W' | $R_{18}$ | X | Y |
|---|---|---|---|---|---|
| $C_6H_5$ | H | O | H | $CH_3$ | $CH_3$ |
| $C_6H_5$ | H | O | H | $CH_3$ | $OCH_3$ |
| $C_6H_5$ | H | O | H | $OCH_3$ | $OCH_3$ |
| $C_6H_5$ | H | O | $CH_3$ | $OCH_3$ | $OCH_3$ |
| 2'-$C_6H_4Cl$ | H | O | H | $CH_3$ | $OCH_3$ |
| 2'-$C_6H_4CH_3$ | H | O | H | $OCH_3$ | $OCH_3$ |
| 2'-$C_6H_4OCH_3$ | H | O | H | $OCF_2H$ | $OCH_3$ |
| 3'-$C_6H_4Br$ | H | O | H | $CH_3$ | $OCH_3$ |
| 4'-$C_6H_4F$ | H | O | H | Cl | $OCH_3$ [b,d,f] |
| $CH_2CO_2CH_3$ | H | O | H | $CH_3$ | $OCH_3$ |
| $CH_2CO_2CH_3$ | H | O | H | $OCH_3$ | $OCH_3$ |
| $CH_2CO_2CH_3$ | H | O | $CH_3$ | Cl | $OCH_3$ [b,d,f] |
| $CHCO_2CH_3$ (with $CH_3$ on CH) | H | O | H | $OCH_3$ | $OCF_2H$ |
| $OSO_2CH_3$ | H | O | H | $CH_3$ | $OCH_3$ |
| $OSO_2CH_3$ | H | O | H | $OCH_3$ | $OCH_3$ |
| $OSO_2CH_3$ | H | O | $CH_3$ | Cl | $OCH_3$ [b,d,f] |
| $OSO_2CH_3$ | H | O | $CH_3$ | $OCH_3$ | $OCF_2H$ |
| $OSO_2CF_3$ | H | O | H | $CH_3$ | $OCH_3$ |
| $CH_2OCH_3$ | H | O | H | $CH_3$ | $OCH_3$ |
| $CH_2OCH_3$ | H | O | H | $OCH_3$ | $OCH_3$ |
| $CH_2OCH_3$ | H | O | $CH_3$ | $OCH_3$ | $OCF_2H$ |
| $CH_2OCH_3$ | H | O | $CH_3$ | $CH_3$ | $CH_3$ |
| $CH_2OCF_2H$ | H | O | H | $OCH_3$ | $OCH_3$ |
| $CH_2OCF_2H$ | H | O | H | $OCH_3$ | $OCF_2H$ |
| $CH_2OCF_2H$ | H | O | $CH_3$ | Cl | $OCH_3$ [b,d,f] |
| $CH_2OCF_2H$ | H | O | $CH_3$ | $CH_3$ | $OCH_3$ |
| $N(CH_3)_2$ | H | S | H | $CH_3$ | $OCH_3$ |
| $N(CH_3)_2$ | H | S | H | $OCH_3$ | $OCH_3$ |
| $N(CH_3)_2$ | H | S | $CH_3$ | $CH_3$ | $OCH_3$ |
| $N(CH_3)CH_2CH_3$ | H | S | H | $CH_3$ | $OCH_3$ |

Table 5 (continued)

| $R_1$ | $R_3$ | W' | $R_{18}$ | X | Y |
|---|---|---|---|---|---|
| $N(CH_2CH_3)_2$ | H | S | H | $OCH_3$ | $OCH_3$ |
| $SO_2N(CH_3)_2$ | H | S | H | $OCH_3$ | $OCH_3$ |
| $SO_2N(CH_3)_2$ | H | S | $CH_3$ | $CH_3$ | $OCH_3$ |
| $C_6H_5$ | H | S | H | $CH_3$ | $CH_3$ |
| $C_6H_5$ | H | S | H | $CH_3$ | $OCH_3$ |
| $C_6H_5$ | H | S | H | $OCH_3$ | $OCH_3$ |
| $C_6H_5$ | H | S | $CH_3$ | $OCH_3$ | $OCH_3$ |
| $2'-C_6H_4Cl$ | H | S | H | $CH_3$ | $OCH_3$ |
| $2'-C_6H_4CH_3$ | H | S | H | $OCH_3$ | $OCH_3$ |
| $2'-C_6H_4OCH_3$ | H | S | H | $OCF_2H$ | $OCH_3$ |
| $3'-C_6H_4Br$ | H | S | H | $CH_3$ | $OCH_3$ |
| $4'-C_6H_4F$ | H | S | H | Cl | $OCH_3$ b,d,f |
| $CH_2CO_2CH_3$ | H | S | H | $CH_3$ | $OCH_3$ |
| $CH_2CO_2CH_3$ | H | S | H | $OCH_3$ | $OCH_3$ |
| $CH_2CO_2CH_3$ | H | S | $CH_3$ | Cl | $OCH_3$ b,d,f |
| $CH(CH_3)CO_2CH_3$ | H | S | H | $OCH_3$ | $OCF_2H$ |
| $OSO_2CH_3$ | H | S | H | $CH_3$ | $OCH_3$ |
| $OSO_2CH_3$ | H | S | H | $OCH_3$ | $OCH_3$ |
| $OSO_2CH_3$ | H | S | $CH_3$ | Cl | $OCH_3$ b,d,f |
| $OSO_2CH_3$ | H | S | $CH_3$ | $OCH_3$ | $OCF_2H$ |
| $OSO_2CF_3$ | H | S | H | $CH_3$ | $OCH_3$ |
| $CH_2OCH_3$ | H | S | H | $CH_3$ | $OCH_3$ |
| $CH_2OCH_3$ | H | S | H | $OCH_3$ | $OCH_3$ |
| $CH_2OCH_3$ | H | S | $CH_3$ | $OCH_3$ | $OCF_2H$ |
| $CH_2OCH_3$ | H | S | $CH_3$ | $CH_3$ | $CH_3$ |
| $CH_2OCF_2H$ | H | S | H | $OCH_3$ | $OCH_3$ |
| $CH_2OCF_2H$ | H | S | H | $OCH_3$ | $OCF_2H$ |
| $CH_2OCF_2H$ | H | S | $CH_3$ | Cl | $OCH_3$ b,d,f |
| $CH_2OCF_2H$ | H | S | $CH_3$ | $CH_3$ | $OCH_3$ |

Table 6

a.) Z = N

b.) Z = CH

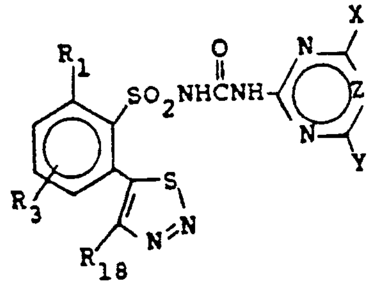

c.) Z = N

d.) Z = CH

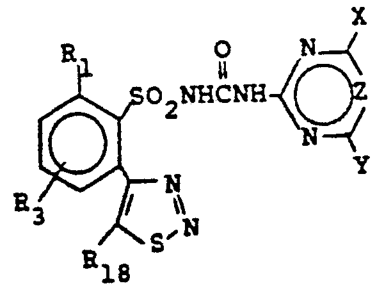

| $R_1$ | $R_3$ | $R_{18}$ | X | Y |
|---|---|---|---|---|
| $N(CH_3)_2$ | H | H | $CH_3$ | $OCH_3$ |
| $N(CH_3)_2$ | H | H | $OCH_3$ | $OCH_3$ |
| $N(CH_3)_2$ | H | $CH_3$ | $CH_3$ | $OCH_3$ |
| $N(CH_3)CH_2CH_3$ | H | H | $CH_3$ | $OCH_3$ |
| $N(CH_2CH_3)_2$ | H | H | $OCH_3$ | $OCH_3$ |
| $SO_2N(CH_3)_2$ | H | H | $OCH_3$ | $OCH_3$ |
| $SO_2N(CH_3)_2$ | H | $CH_3$ | $CH_3$ | $OCH_3$ |
| $C_6H_5$ | H | H | $CH_3$ | $CH_3$ |
| $C_6H_5$ | H | H | $CH_3$ | $OCH_3$ |
| $C_6H_5$ | H | H | $OCH_3$ | $OCH_3$ |
| $C_6H_5$ | H | $CH_3$ | $OCH_3$ | $OCH_3$ |
| 2'-$C_6H_4Cl$ | H | H | $CH_3$ | $OCH_3$ |
| 2'-$C_6H_4CH_3$ | H | H | $OCH_3$ | $OCH_3$ |
| 2'-$C_6H_4OCH_3$ | H | H | $OCF_2H$ | $OCH_3$ |
| 3'-$C_6H_4Br$ | H | H | $CH_3$ | $OCH_3$ |

Table 6 (continued)

| $R_1$ | $R_3$ | $R_{18}$ | X | Y |
|---|---|---|---|---|
| 4'-$C_6H_4F$ | H | H | Cl | $OCH_3$ [b,d] |
| $CH_2CO_2CH_3$ | H | H | $CH_3$ | $OCH_3$ |
| $CH_2CO_2CH_3$ | H | H | $OCH_3$ | $OCH_3$ |
| $CH_2CO_2CH_3$ | H | $CH_3$ | Cl | $OCH_3$ [b,d] |
| $CHCO_2CH_3$ (with $CH_3$ attached to the CH) | H | H | $OCH_3$ | $OCF_2H$ |
| $OSO_2CH_3$ | H | H | $CH_3$ | $OCH_3$ |
| $OSO_2CH_3$ | H | H | $OCH_3$ | $OCH_3$ |
| $OSO_2CH_3$ | H | $CH_3$ | Cl | $OCH_3$ [b,d] |
| $OSO_2CH_3$ | H | $CH_3$ | $OCH_3$ | $OCF_2H$ |
| $OSO_2CF_3$ | H | H | $CH_3$ | $OCH_3$ |
| $CH_2OCH_3$ | H | H | $CH_3$ | $OCH_3$ |
| $CH_2OCH_3$ | H | H | $OCH_3$ | $OCH_3$ |
| $CH_2OCH_3$ | H | $CH_3$ | $OCH_3$ | $OCF_2H$ |
| $CH_2OCH_3$ | H | $CH_3$ | $CH_3$ | $CH_3$ |
| $CH_2OCF_2H$ | H | H | $OCH_3$ | $OCH_3$ |
| $CH_2OCF_2H$ | H | H | $OCH_3$ | $OCF_2H$ |
| $CH_2OCF_2H$ | H | $CH_3$ | Cl | $OCH_3$ [b,d] |
| $CH_2OCF_2H$ | H | $CH_3$ | $CH_3$ | $OCH_3$ |

Table 7

a.) Z = N

b.) Z = CH

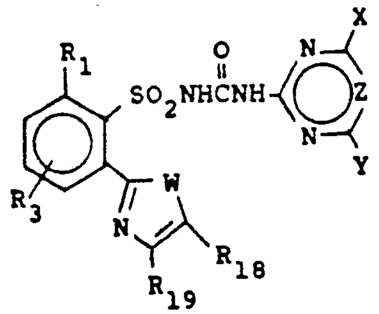

c.) Z = N

d.) Z = CH

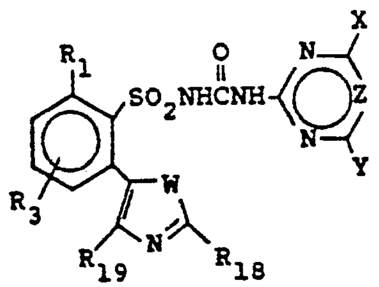

e.) Z = N

f.) Z = CH

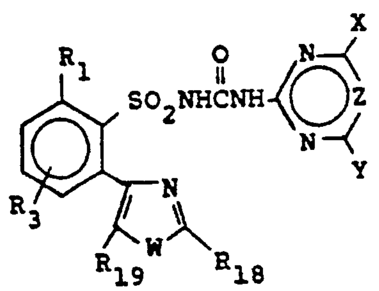

| $R_1$ | $R_3$ | W | $R_{18}$ | $R_{19}$ | X | Y |
|---|---|---|---|---|---|---|
| $N(CH_3)_2$ | H | O | H | H | $CH_3$ | $OCH_3$ |
| $N(CH_3)_2$ | H | O | H | H | $OCH_3$ | $OCH_3$ |
| $N(CH_3)_2$ | H | O | $CH_3$ | H | $CH_3$ | $OCH_3$ |
| $N(CH_3)_2$ | H | O | H | $CH_3$ | $OCH_3$ | $OCH_3$ |
| $N(CH_3)CH_2CH_3$ | H | O | H | H | $CH_3$ | $OCH_3$ |

Table 7 (continued)

| $R_1$ | $R_3$ | W | $R_{18}$ | $R_{19}$ | X | Y |
|---|---|---|---|---|---|---|
| $N(CH_2CH_3)_2$ | H | O | H | H | $OCH_3$ | $OCH_3$ |
| $SO_2N(CH_3)_2$ | H | O | H | H | $OCH_3$ | $OCH_3$ |
| $SO_2N(CH_3)_2$ | H | O | $CH_3$ | H | $CH_3$ | $OCH_3$ |
| $C_6H_5$ | H | O | H | H | $CH_3$ | $CH_3$ |
| $C_6H_5$ | H | O | H | H | $CH_3$ | $OCH_3$ |
| $C_6H_5$ | H | O | H | H | $OCH_3$ | $OCH_3$ |
| $C_6H_5$ | H | O | $CH_3$ | H | $CH_3$ | $OCH_3$ |
| $C_6H_5$ | H | O | H | $CH_3$ | $OCH_3$ | $OCH_3$ |
| $C_6H_5$ | H | O | $CH_3$ | $CH_3$ | $OCH_3$ | $OCF_2H$ |
| $2'-C_6H_4Cl$ | H | O | H | H | $CH_3$ | $OCH_3$ |
| $2'-C_6H_4OCH_3$ | H | O | H | H | $OCH_3$ | $OCH_3$ |
| $2'-C_6H_4CH_3$ | H | O | H | H | Cl | $OCH_3$ b,d,f |
| $3'-C_6H_4Br$ | H | O | H | H | $CH_3$ | $OCH_3$ |
| $4'-C_6H_4F$ | H | O | H | H | $OCH_3$ | $OCH_3$ |
| $CH_2CO_2CH_3$ | H | O | H | H | $CH_3$ | $OCH_3$ |
| $CH_2CO_2CH_3$ | H | O | $CH_3$ | H | $OCH_3$ | $OCH_3$ |
| $CH_2CO_2CH_3$ | H | O | H | $CH_3$ | Cl | $OCH_3$ b,d,f |
| $CH(CH_3)CO_2CH_3$ | H | O | H | H | $OCH_3$ | $OCF_2H$ |
| $OSO_2CH_3$ | H | O | H | H | $CH_3$ | $OCH_3$ |
| $OSO_2CH_3$ | H | O | $CH_3$ | H | $OCH_3$ | $OCH_3$ |
| $OSO_2CH_3$ | H | O | H | $CH_3$ | Cl | $OCH_3$ b,d,f |
| $OSO_2CH_3$ | H | O | $CH_3$ | $CH_3$ | $OCH_3$ | $OCF_2H$ |
| $OSO_2CF_3$ | H | O | H | H | $CH_3$ | $OCH_3$ |
| $CH_2OCH_3$ | H | O | H | H | $CH_3$ | $OCH_3$ |
| $CH_2OCH_3$ | H | O | $CH_3$ | H | $OCH_3$ | $OCH_3$ |
| $CH_2OCH_3$ | H | O | H | $CH_3$ | $OCH_3$ | $OCF_2H$ |
| $CH_2OCH_3$ | H | O | $CH_3$ | $CH_3$ | $CH_3$ | $CH_3$ |
| $CH_2OCF_2H$ | H | O | H | H | $OCH_3$ | $OCH_3$ |
| $CH_2OCF_2H$ | H | O | $CH_3$ | H | $OCH_3$ | $OCF_2H$ |

Table 7 (continued)

| $R_1$ | $R_3$ | W | $R_{18}$ | $R_{19}$ | X | Y |
|---|---|---|---|---|---|---|
| $CH_2OCF_2H$ | H | O | H | $CH_3$ | Cl | $OCH_3$ [b,d,f] |
| $CH_2OCF_2H$ | H | O | $CH_3$ | $CH_3$ | $CH_3$ | $OCH_3$ |
| $N(CH_3)_2$ | H | $NCH_3$ | H | H | $CH_3$ | $OCH_3$ |
| $N(CH_3)_2$ | H | $NCH_3$ | H | H | $OCH_3$ | $OCH_3$ |
| $N(CH_3)_2$ | H | $NCH_3$ | $CH_3$ | H | $CH_3$ | $OCH_3$ |
| $N(CH_3)_2$ | H | $NCH_3$ | H | $CH_3$ | $OCH_3$ | $OCH_3$ |
| $N(CH_3)CH_2CH_3$ | H | $NCH_3$ | H | H | $CH_3$ | $OCH_3$ |
| $N(CH_2CH_3)_2$ | H | $NCH_3$ | H | H | $OCH_3$ | $OCH_3$ |
| $SO_2N(CH_3)_2$ | H | $NCH_3$ | H | H | $OCH_3$ | $OCH_3$ |
| $SO_2N(CH_3)_2$ | H | NH | $CH_3$ | H | $CH_3$ | $OCH_3$ |
| $C_6H_5$ | H | $NCH_3$ | H | H | $CH_3$ | $CH_3$ |
| $C_6H_5$ | H | $NCH_3$ | H | H | $CH_3$ | $OCH_3$ |
| $C_6H_5$ | H | $NCH_3$ | H | H | $OCH_3$ | $OCH_3$ |
| $C_6H_5$ | H | $NCH_3$ | $CH_3$ | H | $CH_3$ | $OCH_3$ |
| $C_6H_5$ | H | $NCH_3$ | H | $CH_3$ | $OCH_3$ | $OCH_3$ |
| $C_6H_5$ | H | $NCH_3$ | $CH_3$ | $CH_3$ | $OCH_3$ | $OCF_2H$ |
| 2'-$C_6H_4Cl$ | H | $NCH_3$ | H | H | $CH_3$ | $OCH_3$ |
| 2'-$C_6H_4OCH_3$ | H | $NCH_3$ | H | H | $OCH_3$ | $OCH_3$ |
| 2'-$C_6H_4CH_3$ | H | $NCH_3$ | H | H | Cl | $OCH_3$ [b,d,f] |
| 3'-$C_6H_4Br$ | H | NH | H | H | $CH_3$ | $OCH_3$ |
| 4'-$C_6H_4F$ | H | $NCH_3$ | H | H | $OCH_3$ | $OCH_3$ |
| $CH_2CO_2CH_3$ | H | $NCH_3$ | H | H | $CH_3$ | $OCH_3$ |
| $CH_2CO_2CH_3$ | H | $NCH_3$ | $CH_3$ | H | $OCH_3$ | $OCH_3$ |
| $CH_2CO_2CH_3$ | H | $NCH_3$ | H | $CH_3$ | Cl | $OCH_3$ [b,d,f] |
| $CH(CH_3)CO_2CH_3$ | H | $NCH_3$ | H | H | $OCH_3$ | $OCF_2H$ |
| $OSO_2CH_3$ | H | $NCH_3$ | H | H | $CH_3$ | $OCH_3$ |
| $OSO_2CH_3$ | H | $NCH_3$ | $CH_3$ | H | $OCH_3$ | $OCH_3$ |
| $OSO_2CH_3$ | H | $NCH_3$ | H | $CH_3$ | Cl | $OCH_3$ [b,d,f] |
| $OSO_2CH_3$ | H | $NCH_3$ | $CH_3$ | $CH_3$ | $OCH_3$ | $OCF_2H$ |

Table 7 (continued)

| $R_1$ | $R_3$ | W | $R_{18}$ | $R_{19}$ | X | Y |
|---|---|---|---|---|---|---|
| $OSO_2CF_3$ | H | $NCH_3$ | H | H | $CH_3$ | $OCH_3$ |
| $CH_2OCH_3$ | H | $NCH_3$ | H | H | $CH_3$ | $OCH_3$ |
| $CH_2OCH_3$ | H | $NCH_3$ | $CH_3$ | H | $OCH_3$ | $OCH_3$ |
| $CH_2OCH_3$ | H | NH | H | $CH_3$ | $OCH_3$ | $OCF_2H$ |
| $CH_2OCH_3$ | H | $NCH_3$ | $CH_3$ | $CH_3$ | $CH_3$ | $CH_3$ |
| $CH_2OCF_2H$ | H | $NCH_3$ | H | H | $OCH_3$ | $OCH_3$ |
| $CH_2OCF_2H$ | H | $NCH_3$ | $CH_3$ | H | $OCH_3$ | $OCF_2H$ |
| $CH_2OCF_2H$ | H | $NCH_3$ | H | $CH_3$ | Cl | $OCH_3$ [b,d,f] |
| $CH_2OCF_2H$ | H | $NCH_3$ | $CH_3$ | $CH_3$ | $CH_3$ | $OCH_3$ |
| $SCH_3$ | 3-Cl | S | $CH_3$ | $CH_3$ | $CH_3$ | $OCH_3$ |
| $N(CH_3)_2$ | H | S | H | H | $CH_3$ | $OCH_3$ |
| $N(CH_3)_2$ | H | S | H | H | $OCH_3$ | $OCH_3$ |
| $N(CH_3)_2$ | H | S | $CH_3$ | H | $CH_3$ | $OCH_3$ |
| $N(CH_3)_2$ | H | S | H | $CH_3$ | $OCH_3$ | $OCH_3$ |
| $N(CH_3)CH_2CH_3$ | H | S | H | H | $CH_3$ | $OCH_3$ |
| $N(CH_2CH_3)_2$ | H | S | H | H | $OCH_3$ | $OCH_3$ |
| $SO_2N(CH_3)_2$ | H | S | H | H | $OCH_3$ | $OCH_3$ |
| $SO_2N(CH_3)_2$ | H | S | $CH_3$ | H | $CH_3$ | $OCH_3$ |
| $C_6H_5$ | H | S | H | H | $CH_3$ | $CH_3$ |
| $C_6H_5$ | H | S | H | H | $CH_3$ | $OCH_3$ |
| $C_6H_5$ | H | S | H | H | $OCH_3$ | $OCH_3$ |
| $C_6H_5$ | H | S | $CH_3$ | H | $CH_3$ | $OCH_3$ |
| $C_6H_5$ | H | S | H | $CH_3$ | $OCH_3$ | $OCH_3$ |
| $C_6H_5$ | H | S | $CH_3$ | $CH_3$ | $OCH_3$ | $OCF_2H$ |
| $2'-C_6H_4Cl$ | H | S | H | H | $CH_3$ | $OCH_3$ |
| $2'-C_6H_4OCH_3$ | H | S | H | H | $OCH_3$ | $OCH_3$ |
| $2'-C_6H_4CH_3$ | H | S | H | H | Cl | $OCH_3$ [b,d,f] |
| $3'-C_6H_4Br$ | H | S | H | H | $CH_3$ | $OCH_3$ |
| $4'-C_6H_4F$ | H | S | H | H | $OCH_3$ | $OCH_3$ |
| $CH_2CO_2CH_3$ | H | S | H | H | $CH_3$ | $OCH_3$ |
| $CH_2CO_2CH_3$ | H | S | $CH_3$ | H | $OCH_3$ | $OCH_3$ |

Table 7 (continued)

| $R_1$ | $R_3$ | W | $R_{18}$ | $R_{19}$ | X | Y |
|---|---|---|---|---|---|---|
| $CH_2CO_2CH_3$ | H | S | H | $CH_3$ | Cl | $OCH_3$ [b,d,f] |
| $CHCO_2CH_3$<br>$CH_3$ | H | S | H | H | $OCH_3$ | $OCF_2H$ |
| $OSO_2CH_3$ | H | S | H | H | $CH_3$ | $OCH_3$ |
| $OSO_2CH_3$ | H | S | $CH_3$ | H | $OCH_3$ | $OCH_3$ |
| $OSO_2CH_3$ | H | S | H | $CH_3$ | Cl | $OCH_3$ [b,d,f] |
| $OSO_2CH_3$ | H | S | $CH_3$ | $CH_3$ | $OCH_3$ | $OCF_2H$ |
| $OSO_2CF_3$ | H | S | H | H | $CH_3$ | $OCH_3$ |
| $CH_2OCH_3$ | H | S | H | H | $CH_3$ | $OCH_3$ |
| $CH_2OCH_3$ | H | S | $CH_3$ | H | $OCH_3$ | $OCH_3$ |
| $CH_2OCH_3$ | H | S | H | $CH_3$ | $OCH_3$ | $OCF_2H$ |
| $CH_2OCH_3$ | H | S | $CH_3$ | $CH_3$ | $CH_3$ | $CH_3$ |
| $CH_2OCF_2H$ | H | S | H | H | $OCH_3$ | $OCH_3$ |
| $CH_2OCF_2H$ | H | S | $CH_3$ | H | $OCH_3$ | $OCF_2H$ |
| $CH_2OCF_2H$ | H | S | H | $CH_3$ | Cl | $OCH_3$ [b,d,f] |
| $CH_2OCF_2H$ | H | S | $CH_3$ | $CH_3$ | $CH_3$ | $OCH_3$ |

Table 8

a.) Z = N

b.) Z = CH

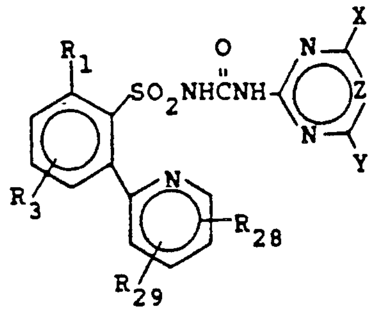

c.) Z = N

d.) Z = CH

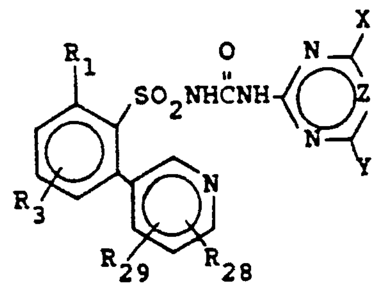

e.) Z = N

f.) Z = CH

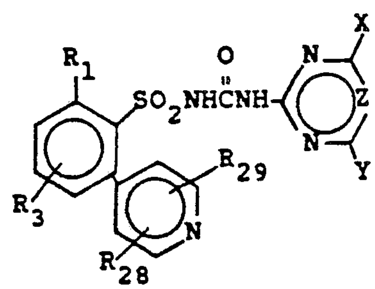

| $R_1$ | $R_3$ | $R_{28}$ | $R_{29}$ | X | Y |
|---|---|---|---|---|---|
| $N(CH_3)_2$ | H | H | H | $CH_3$ | $OCH_3$ |
| $N(CH_3)_2$ | H | H | H | $OCH_3$ | $OCH_3$ |
| $N(CH_3)_2$ | H | 3-$CH_3$ | 5-$CH_3$ | $CH_3$ | $OCH_3$ |
| $N(CH_3)CH_2CH_3$ | H | H | H | $CH_3$ | $OCH_3$ |
| $N(CH_2CH_3)_2$ | H | H | H | $OCH_3$ | $OCH_3$ |
| $SO_2N(CH_3)_2$ | H | H | H | $OCH_3$ | $OCH_3$ |
| $SO_2N(CH_3)_2$ | H | 4-$CH_3$ | 5-$CH_3$ | $CH_3$ | $OCH_3$ |

Table 8 (continued)

| $R_1$ | $R_3$ | $R_{28}$ | $R_{29}$ | X | Y |
|---|---|---|---|---|---|
| $C_6H_5$ | H | H | H | $CH_3$ | $CH_3$ |
| $C_6H_5$ | H | H | H | $CH_3$ | $OCH_3$ |
| $C_6H_5$ | H | H | H | $OCH_3$ | $OCH_3$ |
| $C_6H_5$ | H | 4-$CH_3$ | 6-$CH_3$ | $OCH_3$ | $OCH_3$ |
| 2'-$C_6H_4Cl$ | H | H | H | $CH_3$ | $OCH_3$ |
| 2'-$C_6H_4CH_3$ | H | H | H | $OCH_3$ | $OCH_3$ |
| 2'-$C_6H_4OCH_3$ | H | H | H | $OCF_2H$ | $OCH_3$ |
| 3'-$C_6H_4Br$ | H | H | H | $CH_3$ | $OCH_3$ |
| 4'-$C_6H_4F$ | H | H | H | Cl[b,d,f] | $OCH_3$ |
| $CH_2CO_2CH_3$ | H | H | H | $CH_3$ | $OCH_3$ |
| $CH_2CO_2CH_3$ | H | H | H | $OCH_3$ | $OCH_3$ |
| $CH_2CO_2CH_3$ | H | 5-$CH_3$ | 6-$CH_3$ | Cl[b,d,f] | $OCH_3$ |
| $CHCO_2CH_3$ | H | H | H | $OCH_3$ | $OCF_2H$ |
| $CH_3$ | | | | | |
| $OSO_2CH_3$ | H | H | H | $CH_3$ | $OCH_3$ |
| $OSO_2CH_3$ | H | H | H | $OCH_3$ | $OCH_3$ |
| $OSO_2CH_3$ | H | 6-$CH_3$ | H | Cl[b,d,f] | $OCH_3$ |
| $OSO_2CH_3$ | H | 3-$CH_3$ | H | $OCH_3$ | $OCF_2H$ |
| $OSO_2CF_3$ | H | H | H | $CH_3$ | $OCH_3$ |
| $CH_2OCH_3$ | H | H | H | $CH_3$ | $OCH_3$ |
| $CH_2OCH_3$ | H | H | H | $OCH_3$ | $OCH_3$ |
| $CH_2OCH_3$ | H | 6-$CH_3$ | H | $OCH_3$ | $OCF_2H$ |
| $CH_2OCH_3$ | H | 3-$CH_3$ | H | $CH_3$ | $CH_3$ |
| $CH_2OCF_2H$ | H | H | H | $OCH_3$ | $OCH_3$ |
| $CH_2OCF_2H$ | H | H | H | $OCH_3$ | $OCF_2H$ |
| $CH_2OCF_2H$ | H | 6-$CH_3$ | H | Cl[b,d,f] | $OCH_3$ |
| $CH_2OCF_2H$ | H | 3-$CH_3$ | H | $CH_3$ | $OCH_3$ |

Table 9

a.) Z = N

b.) Z = CH

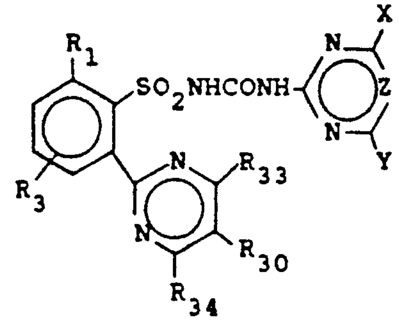

| $R_1$ | $R_3$ | $R_{33}$ | $R_{30}$ | $R_{34}$ | X | Y |
|---|---|---|---|---|---|---|
| $N(CH_3)_2$ | H | $CH_3$ | H | $CH_3$ | $CH_3$ | $OCH_3$ |
| $N(CH_3)_2$ | H | $CH_3$ | H | $CH_3$ | $OCH_3$ | $OCH_3$ |
| $N(CH_3)_2$ | H | H | $CH_3$ | $CH_3$ | $CH_3$ | $OCH_3$ |
| $N(CH_3)_2$ | H | H | $CH_3$ | H | $OCH_3$ | $OCH_3$ |
| $N(CH_3)CH_2CH_3$ | H | $CH_3$ | H | $CH_3$ | $CH_3$ | $OCH_3$ |
| $N(CH_2CH_3)_2$ | H | $CH_3$ | H | $CH_3$ | $OCH_3$ | $OCH_3$ |
| $SO_2N(CH_3)_2$ | H | $CH_3$ | H | $CH_3$ | $OCH_3$ | $OCH_3$ |
| $SO_2N(CH_3)_2$ | H | $CH_3$ | H | $CH_3$ | $CH_3$ | $OCH_3$ |
| $C_6H_5$ | H | $CH_3$ | H | $CH_3$ | $CH_3$ | $CH_3$ |
| $C_6H_5$ | H | $CH_3$ | H | $CH_3$ | $CH_3$ | $OCH_3$ |
| $C_6H_5$ | H | $CH_3$ | H | $CH_3$ | $OCH_3$ | $OCH_3$ |
| $C_6H_5$ | H | $CH_3$ | $CH_3$ | $CH_3$ | $CH_3$ | $OCH_3$ |
| $C_6H_5$ | H | H | $CH_3$ | H | $OCH_3$ | $OCH_3$ |
| $2'-C_6H_4Cl$ | H | $CH_3$ | H | $CH_3$ | $CH_3$ | $OCH_3$ |
| $3'-C_6H_4Br$ | H | $CH_3$ | H | $OCH_3$ | $OCH_3$ | $OCH_3$ |
| $4'-C_6H_4F$ | H | $CH_3$ | H | $CH_3$ | $CH_3$ | $OCH_3$ |
| $2'-C_6H_4CH_3$ | H | $CH_3$ | H | $CH_3$ | $OCH_3$ | $OCH_3$ |
| $2'-C_6H_4OCH_3$ | H | $CH_3$ | H | $CH_3$ | $OCH_3$ | $OCF_2H$ |
| $CH_2CO_2CH_3$ | H | $CH_3$ | H | $CH_3$ | $CH_3$ | $OCH_3$ |
| $CH_2CO_2CH_3$ | H | $CH_3$ | H | $CH_3$ | $OCH_3$ | $OCH_3$ |
| $CH_2CO_2CH_3$ | H | H | $CH_3$ | $CH_3$ | $Cl^b$ | $OCH_3$ |

Table 9 (continued)

| $R_1$ | $R_3$ | $R_{33}$ | $R_{30}$ | $R_{34}$ | X | Y |
|---|---|---|---|---|---|---|
| $CHCO_2CH_3$ (with $CH_3$ on CH) | H | $CH_3$ | H | $CH_3$ | $OCH_3$ | $OCF_2H$ |
| $OSO_2CH_3$ | H | $CH_3$ | H | $CH_3$ | $CH_3$ | $OCH_3$ |
| $OSO_2CH_3$ | H | $CH_3$ | H | $CH_3$ | $OCH_3$ | $OCH_3$ |
| $OSO_2CH_3$ | H | H | $CH_3$ | H | $Cl^b$ | $OCH_3$ |
| $OSO_2CH_3$ | H | $CH_3$ | $CH_3$ | $CH_3$ | $OCH_3$ | $OCF_2H$ |
| $OSO_2CF_3$ | H | $CH_3$ | H | $CH_3$ | $CH_3$ | $OCH_3$ |
| $CH_2OCH_3$ | H | $CH_3$ | H | $CH_3$ | $CH_3$ | $OCH_3$ |
| $CH_2OCH_3$ | H | $CH_3$ | H | $CH_3$ | $OCH_3$ | $OCH_3$ |
| $CH_2OCH_3$ | H | H | $CH_3$ | H | $OCH_3$ | $OCF_2H$ |
| $CH_2OCH_3$ | H | $CH_3$ | $CH_3$ | $CH_3$ | $CH_3$ | $CH_3$ |
| $CH_2OCF_2H$ | H | $CH_3$ | H | $CH_3$ | $OCH_3$ | $OCH_3$ |
| $CH_2OCF_2H$ | H | $CH_3$ | H | $CH_3$ | $OCH_3$ | $OCF_2H$ |
| $CH_2OCF_2H$ | H | H | $CH_3$ | H | $Cl^b$ | $OCH_3$ |
| $CH_2OCF_2H$ | H | $CH_3$ | $CH_3$ | $CH_3$ | $CH_3$ | $OCH_3$ |

Table 10

a.) Z = N

b.) Z = CH

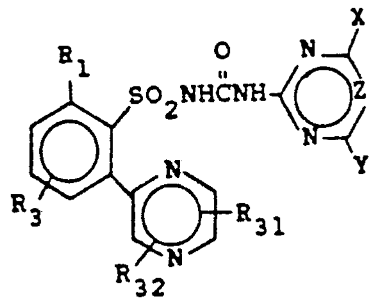

| $R_1$ | $R_3$ | $R_{31}$ | $R_{32}$ | X | Y |
|---|---|---|---|---|---|
| $N(CH_3)_2$ | H | H | H | $CH_3$ | $OCH_3$ |
| $N(CH_3)_2$ | H | H | H | $OCH_3$ | $OCH_3$ |
| $N(CH_3)_2$ | H | 3-$CH_3$ | 6-$CH_3$ | $CH_3$ | $OCH_3$ |
| $N(CH_3)CH_2CH_3$ | H | H | H | $CH_3$ | $OCH_3$ |
| $N(CH_2CH_3)_2$ | H | H | H | $OCH_3$ | $OCH_3$ |
| $SO_2N(CH_3)_2$ | H | H | H | $OCH_3$ | $OCH_3$ |
| $SO_2N(CH_3)_2$ | H | 3-$CH_3$ | 6-$CH_3$ | $CH_3$ | $OCH_3$ |
| $C_6H_5$ | H | H | H | $CH_3$ | $CH_3$ |
| $C_6H_5$ | H | H | H | $CH_3$ | $OCH_3$ |
| $C_6H_5$ | H | H | H | $OCH_3$ | $OCH_3$ |
| $C_6H_5$ | H | 5-$CH_3$ | 6-$CH_3$ | $OCH_3$ | $OCH_3$ |
| 2'-$C_6H_4Cl$ | H | H | H | $CH_3$ | $OCH_3$ |
| 2'-$C_6H_4CH_3$ | H | H | H | $OCH_3$ | $OCH_3$ |
| 2'-$C_6H_4OCH_3$ | H | H | H | $OCF_2H$ | $OCH_3$ |
| 3'-$C_6H_4Br$ | H | H | H | $CH_3$ | $OCH_3$ |
| 4'-$C_6H_4F$ | H | H | H | Cl[b] | $OCH_3$ |
| $CH_2CO_2CH_3$ | H | H | H | $CH_3$ | $OCH_3$ |
| $CH_2CO_2CH_3$ | H | H | H | $OCH_3$ | $OCH_3$ |
| $CH_2CO_2CH_3$ | H | 3-$CH_3$ | 5-$CH_3$ | Cl[b] | $OCH_3$ |
| $CHCO_2CH_3$ (with $CH_3$ on CH) | H | H | H | $OCH_3$ | $OCF_2H$ |
| $OSO_2CH_3$ | H | H | H | $CH_3$ | $OCH_3$ |

Table 10 (continued)

| $R_1$ | $R_3$ | $R_{31}$ | $R_{32}$ | X | Y |
|---|---|---|---|---|---|
| $OSO_2CH_3$ | H | H | H | $OCH_3$ | $OCH_3$ |
| $OSO_2CH_3$ | H | 6-$CH_3$ | H | Cl[b] | $OCH_3$ |
| $OSO_2CH_3$ | H | 6-$CH_3$ | H | $OCH_3$ | $OCF_2H$ |
| $OSO_2CF_3$ | H | H | H | $CH_3$ | $OCH_3$ |
| $CH_2OCH_3$ | H | H | H | $CH_3$ | $OCH_3$ |
| $CH_2OCH_3$ | H | H | H | $OCH_3$ | $OCH_3$ |
| $CH_2OCH_3$ | H | 5-$CH_3$ | H | $OCH_3$ | $OCF_2H$ |
| $CH_2OCH_3$ | H | 6-$CH_3$ | H | $CH_3$ | $CH_3$ |
| $CH_2OCF_2H$ | H | H | H | $OCH_3$ | $OCH_3$ |
| $CH_2OCF_2H$ | H | H | H | $OCH_3$ | $OCF_2H$ |
| $CH_2OCF_2H$ | H | 5-$CH_3$ | H | Cl[b] | $OCH_3$ |
| $CH_2OCF_2H$ | H | 6-$CH_3$ | H | $CH_3$ | $OCH_3$ |

Table 11

a.) Z = N
b.) Z = CH

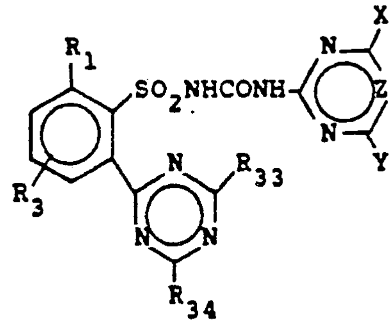

| $R_1$ | $R_3$ | $R_{33}$ | $R_{34}$ | X | Y |
|---|---|---|---|---|---|
| $N(CH_3)_2$ | H | $CH_3$ | $CH_3$ | $CH_3$ | $OCH_3$ |
| $N(CH_3)_2$ | H | $CH_3$ | $CH_3$ | $OCH_3$ | $OCH_3$ |
| $N(CH_3)_2$ | H | $CH_3$ | $OCH_3$ | $CH_3$ | $OCH_3$ |
| $N(CH_3)CH_2CH_3$ | H | $CH_3$ | $CH_3$ | $CH_3$ | $OCH_3$ |
| $N(CH_2CH_3)_2$ | H | H | H | $OCH_3$ | $OCH_3$ |
| $SO_2N(CH_3)_2$ | H | $CH_3$ | $CH_3$ | $OCH_3$ | $OCH_3$ |
| $SO_2N(CH_3)_2$ | H | $OCH_3$ | $OCH_3$ | $CH_3$ | $OCH_3$ |
| $C_6H_5$ | H | H | $OCH_3$ | $CH_3$ | $CH_3$ |
| $C_6H_5$ | H | $CH_3$ | $CH_3$ | $CH_3$ | $OCH_3$ |
| $C_6H_5$ | H | $CH_3$ | $CH_3$ | $OCH_3$ | $OCH_3$ |
| $C_6H_5$ | H | $CH_3$ | $OCH_3$ | $OCH_3$ | $OCH_3$ |
| 2'-$C_6H_4Cl$ | H | $CH_3$ | $CH_3$ | $CH_3$ | $OCH_3$ |
| 2'-$C_6H_4CH_3$ | H | $CH_3$ | $CH_3$ | $OCH_3$ | $OCH_3$ |
| 2'-$C_6H_4OCH_3$ | H | H | $CH_3$ | $OCF_2H$ | $OCH_3$ |
| 3'-$C_6H_4Br$ | H | $CH_3$ | $CH_3$ | $CH_3$ | $OCH_3$ |
| 4'-$C_6H_4F$ | H | $CH_3$ | $CH_3$ | Cl[b] | $OCH_3$ |
| $CH_2CO_2CH_3$ | H | $OCH_3$ | $OCH_3$ | $CH_3$ | $OCH_3$ |
| $CH_2CO_2CH_3$ | H | $CH_3$ | $CH_3$ | $OCH_3$ | $OCH_3$ |
| $CH_2CO_2CH_3$ | H | $CH_3$ | $CH_3$ | Cl[b] | $OCH_3$ |
| $CH(CH_3)CO_2CH_3$ | H | $CH_3$ | $CH_3$ | $OCH_3$ | $OCF_2H$ |

Table 11 (continued)

| $R_1$ | $R_3$ | $R_{33}$ | $R_{34}$ | X | Y |
|---|---|---|---|---|---|
| $OSO_2CH_3$ | H | $CH_3$ | $CH_3$ | $CH_3$ | $OCH_3$ |
| $OSO_2CH_3$ | H | $CH_3$ | $CH_3$ | $OCH_3$ | $OCH_3$ |
| $OSO_2CH_3$ | H | $OCH_3$ | H | $Cl^b$ | $OCH_3$ |
| $OSO_2CH_3$ | H | $CH_3$ | $CH_3$ | $OCH_3$ | $OCF_2H$ |
| $OSO_2CF_3$ | H | $CH_3$ | $CH_3$ | $CH_3$ | $OCH_3$ |
| $CH_2OCH_3$ | H | $CH_3$ | $CH_3$ | $CH_3$ | $OCH_3$ |
| $CH_2OCH_3$ | H | $CH_3$ | $CH_3$ | $OCH_3$ | $OCH_3$ |
| $CH_2OCH_3$ | H | $CH_3$ | H | $OCH_3$ | $OCF_2H$ |
| $CH_2OCH_3$ | H | $CH_3$ | $CH_3$ | $CH_3$ | $CH_3$ |
| $CH_2OCF_2H$ | H | $CH_3$ | $CH_3$ | $OCH_3$ | $OCH_3$ |
| $CH_2OCF_2H$ | H | $CH_3$ | $CH_3$ | $OCH_3$ | $OCF_2H$ |
| $CH_2OCF_2H$ | H | $CH_3$ | H | $Cl^b$ | $OCH_3$ |
| $CH_2OCF_2H$ | H | $CH_3$ | $CH_3$ | $CH_3$ | $OCH_3$ |

Table 12

a.) Z = N

b.) Z = CH

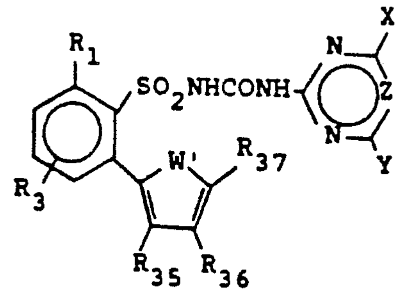

| $R_1$ | $R_3$ | W' | $R_{35}$ | $R_{36}$ | $R_{37}$ | X | Y |
|---|---|---|---|---|---|---|---|
| $N(CH_3)_2$ | H | O | H | H | H | $CH_3$ | $OCH_3$ |
| $N(CH_3)_2$ | H | O | H | H | H | $OCH_3$ | $OCH_3$ |
| $N(CH_3)_2$ | H | O | $CH_3$ | H | $CH_3$ | $CH_3$ | $OCH_3$ |
| $N(CH_3)_2$ | H | O | H | $CH_3$ | H | $OCH_3$ | $OCH_3$ |
| $N(CH_3)CH_2CH_3$ | H | O | H | H | H | $CH_3$ | $OCH_3$ |
| $N(CH_2CH_3)_2$ | H | O | H | H | H | $OCH_3$ | $OCH_3$ |
| $SO_2N(CH_3)_2$ | H | O | H | H | H | $OCH_3$ | $OCH_3$ |
| $SO_2N(CH_3)_2$ | H | O | $CH_3$ | H | $CH_3$ | $CH_3$ | $OCH_3$ |
| $C_6H_5$ | H | O | H | H | H | $CH_3$ | $CH_3$ |
| $C_6H_5$ | H | O | H | H | H | $CH_3$ | $OCH_3$ |
| $C_6H_5$ | H | O | H | H | H | $OCH_3$ | $OCH_3$ |
| $C_6H_5$ | H | O | $CH_3$ | H | $CH_3$ | $CH_3$ | $OCH_3$ |
| $C_6H_5$ | H | O | H | $CH_3$ | H | $OCH_3$ | $OCH_3$ |
| 2'-$C_6H_4Cl$ | H | O | H | H | H | $CH_3$ | $OCH_3$ |
| 2'-$C_6H_4Br$ | H | O | H | H | H | $OCH_3$ | $OCH_3$ |
| 2'-$C_6H_4F$ | H | O | H | H | H | $CH_3$ | $OCH_3$ |
| 3'-$C_6H_4CH_3$ | H | O | H | H | H | $OCH_3$ | $OCH_3$ |
| 4'-$C_6H_4OCH_3$ | H | O | H | H | H | $OCH_3$ | $OCF_2H$ |
| $CH_2CO_2CH_3$ | H | O | H | H | H | $CH_3$ | $OCH_3$ |
| $CH_2CO_2CH_3$ | H | O | $CH_3$ | H | $CH_3$ | $OCH_3$ | $OCH_3$ |
| $CH_2CO_2CH_3$ | H | O | H | $CH_3$ | $CH_3$ | $Cl^b$ | $OCH_3$ |
| $CH(CH_3)CO_2CH_3$ | H | O | H | H | H | $OCH_3$ | $OCF_2H$ |

Table 12 (continued)

| $R_1$ | $R_3$ | W' | $R_{35}$ | $R_{36}$ | $R_{37}$ | X | Y |
|---|---|---|---|---|---|---|---|
| $OSO_2CH_3$ | H | O | H | H | H | $CH_3$ | $OCH_3$ |
| $OSO_2CH_3$ | H | O | $CH_3$ | H | $CH_3$ | $OCH_3$ | $OCH_3$ |
| $OSO_2CH_3$ | H | O | H | $CH_3$ | H | Cl[b] | $OCH_3$ |
| $OSO_2CH_3$ | H | O | $CH_3$ | $CH_3$ | $CH_3$ | $OCH_3$ | $OCF_2H$ |
| $OSO_2CF_3$ | H | O | H | H | H | $CH_3$ | $OCH_3$ |
| $CH_2OCH_3$ | H | O | H | H | H | $CH_3$ | $OCH_3$ |
| $CH_2OCH_3$ | H | O | $CH_3$ | H | $CH_3$ | $OCH_3$ | $OCH_3$ |
| $CH_2OCH_3$ | H | O | H | $CH_3$ | H | $OCH_3$ | $OCF_2H$ |
| $CH_2OCH_3$ | H | O | $CH_3$ | $CH_3$ | $CH_3$ | $CH_3$ | $CH_3$ |
| $CH_2OCF_2H$ | H | O | H | H | H | $OCH_3$ | $OCH_3$ |
| $CH_2OCF_2H$ | H | O | $CH_3$ | H | $CH_3$ | $OCH_3$ | $OCF_2H$ |
| $CH_2OCF_2H$ | H | O | H | $CH_3$ | H | Cl[b] | $OCH_3$ |
| $CH_2OCF_2H$ | H | O | $CH_3$ | $CH_3$ | $CH_3$ | $CH_3$ | $OCH_3$ |
| $N(CH_3)_2$ | H | S | H | H | H | $CH_3$ | $OCH_3$ |
| $N(CH_3)_2$ | H | S | H | H | H | $OCH_3$ | $OCH_3$ |
| $N(CH_3)_2$ | H | S | $CH_3$ | H | $CH_3$ | $CH_3$ | $OCH_3$ |
| $N(CH_3)_2$ | H | S | H | $CH_3$ | H | $OCH_3$ | $OCH_3$ |
| $N(CH_3)CH_2CH_3$ | H | S | H | H | H | $CH_3$ | $OCH_3$ |
| $N(CH_2CH_3)_2$ | H | S | H | H | H | $OCH_3$ | $OCH_3$ |
| $SO_2N(CH_3)_2$ | H | S | H | H | H | $OCH_3$ | $OCH_3$ |
| $SO_2N(CH_3)_2$ | H | S | $CH_3$ | H | $CH_3$ | $CH_3$ | $OCH_3$ |
| $C_6H_5$ | H | S | H | H | H | $CH_3$ | $CH_3$ |
| $C_6H_5$ | H | S | H | H | H | $CH_3$ | $OCH_3$ |
| $C_6H_5$ | H | S | H | H | H | $OCH_3$ | $OCH_3$ |
| $C_6H_5$ | H | S | $CH_3$ | H | $CH_3$ | $CH_3$ | $OCH_3$ |
| $C_6H_5$ | H | S | H | $CH_3$ | H | $OCH_3$ | $OCH_3$ |
| 2'-$C_6H_4Cl$ | H | S | H | H | H | $CH_3$ | $OCH_3$ |
| 2'-$C_6H_4Br$ | H | S | H | H | H | $OCH_3$ | $OCH_3$ |
| 2'-$C_6H_4F$ | H | S | H | H | H | $CH_3$ | $OCH_3$ |
| 3'-$C_6H_4CH_3$ | H | S | H | H | H | $OCH_3$ | $OCH_3$ |

Table 12 (continued)

| $R_1$ | $R_3$ | W' | $R_{35}$ | $R_{36}$ | $R_{37}$ | X | Y |
|---|---|---|---|---|---|---|---|
| 4'-$C_6H_4OCH_3$ | H | S | H | H | H | $OCH_3$ | $OCF_2H$ |
| $CH_2CO_2CH_3$ | H | S | H | H | H | $CH_3$ | $OCH_3$ |
| $CH_2CO_2CH_3$ | H | S | $CH_3$ | H | $CH_3$ | $OCH_3$ | $OCH_3$ |
| $CH_2CO_2CH_3$ | H | S | H | $CH_3$ | $CH_3$ | Cl[b] | $OCH_3$ |
| $CHCO_2CH_3$ (with $CH_3$ on CH) | H | S | H | H | H | $OCH_3$ | $OCF_2H$ |
| $OSO_2CH_3$ | H | S | H | H | H | $CH_3$ | $OCH_3$ |
| $OSO_2CH_3$ | H | S | $CH_3$ | H | $CH_3$ | $OCH_3$ | $OCH_3$ |
| $OSO_2CH_3$ | H | S | H | $CH_3$ | H | Cl[b] | $OCH_3$ |
| $OSO_2CH_3$ | H | S | $CH_3$ | $CH_3$ | $CH_3$ | $OCH_3$ | $OCF_2H$ |
| $OSO_2CF_3$ | H | S | H | H | H | $CH_3$ | $OCH_3$ |
| $CH_2OCH_3$ | H | S | H | H | H | $CH_3$ | $OCH_3$ |
| $CH_2OCH_3$ | H | S | $CH_3$ | H | $CH_3$ | $OCH_3$ | $OCH_3$ |
| $CH_2OCH_3$ | H | S | H | $CH_3$ | H | $OCH_3$ | $OCF_2H$ |
| $CH_2OCH_3$ | H | S | $CH_3$ | $CH_3$ | $CH_3$ | $CH_3$ | $CH_3$ |
| $CH_2OCF_2H$ | H | S | H | H | H | $OCH_3$ | $OCH_3$ |
| $CH_2OCF_2H$ | H | S | $CH_3$ | H | $CH_3$ | $OCH_3$ | $OCF_2H$ |
| $CH_2OCF_2H$ | H | S | H | $CH_3$ | H | Cl[b] | $OCH_3$ |
| $CH_2OCF_2H$ | H | S | $CH_3$ | $CH_3$ | $CH_3$ | $CH_3$ | $OCH_3$ |

Table 13

a.) Z = N
b.) Z = CH

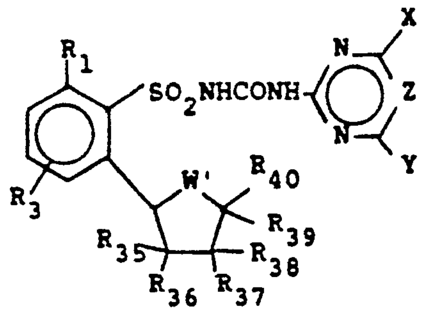

| $R_1$ | $R_3$ | W' | $R_{35}$ | $R_{36}$ | $R_{37}$ | $R_{38}$ | $R_{39}$ | $R_{40}$ | X | Y |
|---|---|---|---|---|---|---|---|---|---|---|
| $SCH_3$ | H | O | H | H | H | H | H | H | $CH_3$ | $OCH_3$ |
| $SCH_3$ | H | O | H | H | H | H | H | H | $OCH_3$ | $OCH_3$ |
| $SCH_3$ | H | O | $CH_3$ | H | H | H | H | H | $OCH_3$ | $OCH_3$ |
| $SCH_3$ | H | O | H | H | $CH_3$ | H | H | H | Cl[b] | $OCH_3$ |
| $SCH_3$ | H | O | H | H | H | H | $CH_3$ | H | $CH_3$ | $CH_3$ |
| $SCH_2CH_3$ | H | O | $CH_3$ | H | $CH_3$ | H | $CH_3$ | H | $CH_3$ | $OCH_3$ |
| $SCH_2CH_2CH_3$ | H | O | H | H | H | H | H | H | $OCH_3$ | $OCH_3$ |
| $S(O)CH_3$ | H | O | $CH_3$ | H | $CH_3$ | $CH_3$ | $CH_3$ | H | $CH_3$ | $OCH_3$ |
| $S(O)CH_3$ | H | O | $CH_3$ | H | $CH_3$ | H | $CH_3$ | H | $OCH_3$ | $OCH_3$ |
| $S(O)CH_3$ | H | O | H | H | H | H | H | H | Cl[b] | $OCH_3$ |
| $S(O)CH_3$ | H | O | H | H | H | H | H | H | $CH_3$ | $OCH_3$ |
| $S(O)_2CH_3$ | H | O | H | H | H | H | H | H | $OCH_3$ | $OCH_3$ |
| $S(O)_2CH_3$ | H | O | $CH_3$ | $CH_3$ | H | H | $CH_3$ | $CH_3$ | $CH_3$ | $OCH_3$ |
| $S(O)_2CH_3$ | H | O | H | H | $CH_3$ | $CH_3$ | H | H | $OCH_3$ | $OCH_3$ |
| $CF_3$ | H | O | H | H | H | H | H | H | $CH_3$ | $OCH_3$ |
| $CF_3$ | H | O | $CH_3$ | H | H | H | H | H | $OCH_3$ | $OCH_3$ |
| $CF_3$ | H | O | H | H | $CH_3$ | H | H | H | Cl[b] | $OCH_3$ |
| $CF_3$ | H | O | H | H | H | H | $CH_3$ | H | $CH_3$ | $OCH_3$ |
| $N(CH_3)_2$ | H | O | H | H | H | H | H | H | $CH_3$ | $OCH_3$ |
| $N(CH_3)_2$ | H | O | H | H | H | H | H | H | $OCH_3$ | $OCH_3$ |
| $N(CH_3)_2$ | H | O | H | H | H | H | H | H | Cl[b] | $OCH_3$ |
| $N(CH_3)_2$ | H | O | $CH_3$ | H | H | H | H | $CH_3$ | $OCH_3$ | $OCH_3$ |

Table 13 (continued)

| $R_1$ | $R_3$ | W' | $R_{35}$ | $R_{36}$ | $R_{37}$ | $R_{38}$ | $R_{39}$ | $R_{40}$ | X | Y |
|---|---|---|---|---|---|---|---|---|---|---|
| $N(CH_3)CH_2CH_3$ | H | O | H | H | H | H | H | H | $OCH_3$ | $OCH_3$ |
| $N(CH_2CH_3)_2$ | H | O | H | H | H | H | H | H | $OCH_3$ | $OCH_3$ |
| $CO_2CH_3$ | H | O | H | H | H | H | H | H | $CH_3$ | $OCH_3$ |
| $CO_2CH_3$ | H | O | H | H | $CH_3$ | H | H | H | $OCH_3$ | $OCH_3$ |
| $CO_2CH_3$ | H | O | H | H | $CH_3$ | $CH_3$ | H | H | $CH_3$ | $CH_3$ |
| $CO_2CH_2CH_2Cl$ | H | O | H | H | H | H | H | H | $CH_3$ | $OCH_3$ |
| $SO_2N(CH_3)_2$ | H | O | H | H | H | H | H | H | $OCH_3$ | $OCH_3$ |
| $SO_2N(CH_3)_2$ | H | O | H | H | H | H | H | H | $CH_3$ | $OCH_3$ |
| $C_6H_5$ | H | O | H | H | H | H | H | H | $CH_3$ | $CH_3$ |
| $C_6H_5$ | H | O | H | H | H | H | H | H | $CH_3$ | $OCH_3$ |
| $C_6H_5$ | H | O | H | H | H | H | H | H | $OCH_3$ | $OCH_3$ |
| $C_6H_5$ | H | O | $CH_3$ | H | $CH_3$ | H | $CH_3$ | H | $CH_3$ | $OCH_3$ |
| $C_6H_5$ | H | O | $CH_3$ | H | H | H | $CH_3$ | H | $OCH_3$ | $OCH_3$ |
| $2'-C_6H_4Cl$ | H | O | H | H | H | H | H | H | $CH_3$ | $OCH_3$ |
| $2'-C_6H_4Br$ | H | O | $CH_3$ | H | $CH_3$ | H | H | H | $OCH_3$ | $OCH_3$ |
| $2'-C_6H_4F$ | H | O | H | H | H | H | H | $CH_3$ | $CH_3$ | $OCH_3$ |
| $3'-C_6H_4CH_3$ | H | O | H | H | H | H | H | H | $OCH_3$ | $OCH_3$ |
| $4'-C_6H_4OCH_3$ | H | O | H | H | H | H | H | H | $OCF_2H$ | $OCH_3$ |
| $CH_2CO_2CH_3$ | H | O | H | H | H | H | H | H | $CH_3$ | $OCH_3$ |
| $CH_2CO_2CH_3$ | H | O | H | H | $CH_3$ | $CH_3$ | H | H | $OCH_3$ | $OCH_3$ |
| $CH_2CO_2CH_3$ | H | O | H | H | H | H | H | H | $Cl^b$ | $OCH_3$ |
| $CHCO_2CH_3$ ($CH_3$) | H | O | H | H | H | H | H | H | $OCF_2H$ | $OCH_3$ |
| $OSO_2CH_3$ | H | O | H | H | H | H | H | H | $CH_3$ | $OCH_3$ |
| $OSO_2CH_3$ | H | O | H | H | H | H | H | H | $OCH_3$ | $OCH_3$ |
| $OSO_2CH_3$ | H | O | H | H | H | H | H | H | $Cl^b$ | $OCH_3$ |
| $OSO_2CH_3$ | H | O | H | H | H | H | H | H | $OCF_2H$ | $OCH_3$ |
| $OSO_2CF_3$ | H | O | H | H | H | H | H | $CH_3$ | $CH_3$ | $OCH_3$ |
| $CH_2OCH_3$ | H | O | H | H | H | H | H | H | $CH_3$ | $OCH_3$ |
| $CH_2OCH_3$ | H | O | H | H | H | H | H | H | $CH_3$ | $CH_3$ |
| $CH_2OCH_3$ | H | O | H | H | $CH_3$ | H | H | H | $OCF_2H$ | $OCH_3$ |

Table 13 (continued)

| $R_1$ | $R_3$ | W' | $R_{35}$ | $R_{36}$ | $R_{37}$ | $R_{38}$ | $R_{39}$ | $R_{40}$ | X | Y |
|---|---|---|---|---|---|---|---|---|---|---|
| $CH_2OCH_3$ | H | O | H | H | $CH_3$ | H | $CH_3$ | H | $CH_3$ | $CH_3$ |
| $CH_2OCF_2H$ | H | O | H | H | H | H | H | H | $CH_3$ | $OCH_3$ |
| $CH_2OCF_2H$ | H | O | H | H | H | H | H | H | $OCH_3$ | $OCH_3$ |
| $CH_2OCF_2H$ | H | O | $CH_3$ | H | H | H | $CH_3$ | H | Cl[b] | $OCH_3$ |
| $CH_2OCF_2H$ | H | O | $CH_3$ | $CH_3$ | $CH_3$ | H | $CH_3$ | H | $CH_3$ | $OCH_3$ |
| $SCH_3$ | H | S | H | H | H | H | H | H | $CH_3$ | $OCH_3$ |
| $SCH_3$ | H | S | H | H | H | H | H | H | $OCH_3$ | $OCH_3$ |
| $SCH_3$ | H | S | $CH_3$ | H | H | H | H | H | $OCH_3$ | $OCH_3$ |
| $SCH_3$ | H | S | H | H | $CH_3$ | H | H | H | Cl[b] | $OCH_3$ |
| $SCH_3$ | H | S | H | H | H | H | $CH_3$ | H | $CH_3$ | $CH_3$ |
| $SCH_2CH_3$ | H | S | $CH_3$ | H | $CH_3$ | H | $CH_3$ | H | $CH_3$ | $OCH_3$ |
| $SCH_2CH_2CH_3$ | H | S | H | H | H | H | H | H | $OCH_3$ | $OCH_3$ |
| $S(O)CH_3$ | H | S | $CH_3$ | H | $CH_3$ | $CH_3$ | $CH_3$ | H | $CH_3$ | $OCH_3$ |
| $S(O)CH_3$ | H | S | $CH_3$ | H | $CH_3$ | H | $CH_3$ | H | $OCH_3$ | $OCH_3$ |
| $S(O)CH_3$ | H | S | H | H | H | H | H | H | Cl[b] | $OCH_3$ |
| $S(O)CH_3$ | H | S | H | H | H | H | H | H | $CH_3$ | $OCH_3$ |
| $S(O)_2CH_3$ | H | S | H | H | H | H | H | H | $OCH_3$ | $OCH_3$ |
| $S(O)_2CH_3$ | H | S | $CH_3$ | $CH_3$ | H | H | $CH_3$ | $CH_3$ | $CH_3$ | $OCH_3$ |
| $S(O)_2CH_3$ | H | S | H | H | $CH_3$ | $CH_3$ | H | H | $OCH_3$ | $OCH_3$ |
| $CF_3$ | H | S | H | H | H | H | H | H | $CH_3$ | $OCH_3$ |
| $CF_3$ | H | S | $CH_3$ | H | H | H | H | H | $OCH_3$ | $OCH_3$ |
| $CF_3$ | H | S | H | H | $CH_3$ | H | H | H | Cl[b] | $OCH_3$ |
| $CF_3$ | H | S | H | H | H | H | $CH_3$ | H | $CH_3$ | $OCH_3$ |
| $N(CH_3)_2$ | H | S | H | H | H | H | H | H | $CH_3$ | $OCH_3$ |
| $N(CH_3)_2$ | H | S | H | H | H | H | H | H | $OCH_3$ | $OCH_3$ |
| $N(CH_3)_2$ | H | S | H | H | H | H | H | H | Cl[b] | $OCH_3$ |
| $N(CH_3)_2$ | H | S | $CH_3$ | H | H | H | H | $CH_3$ | $OCH_3$ | $OCH_3$ |
| $N(CH_3)CH_2CH_3$ | H | S | H | H | H | H | H | H | $OCH_3$ | $OCH_3$ |
| $N(CH_2CH_3)_2$ | H | S | H | H | H | H | H | H | $OCH_3$ | $OCH_3$ |
| $CO_2CH_3$ | H | S | H | H | H | H | H | H | $CH_3$ | $OCH_3$ |

Table 13 (continued)

| $R_1$ | $R_3$ | W' | $R_{35}$ | $R_{36}$ | $R_{37}$ | $R_{38}$ | $R_{39}$ | $R_{40}$ | X | Y |
|---|---|---|---|---|---|---|---|---|---|---|
| $CO_2CH_3$ | H | S | H | H | $CH_3$ | H | H | H | $OCH_3$ | $OCH_3$ |
| $CO_2CH_3$ | H | S | H | H | $CH_3$ | $CH_3$ | H | H | $CH_3$ | $CH_3$ |
| $CO_2CH_2CH_2Cl$ | H | S | H | H | H | H | H | H | $CH_3$ | $OCH_3$ |
| $SO_2N(CH_3)_2$ | H | S | H | H | H | H | H | H | $OCH_3$ | $OCH_3$ |
| $SO_2N(CH_3)_2$ | H | S | H | H | H | H | H | H | $CH_3$ | $OCH_3$ |
| $C_6H_5$ | H | S | H | H | H | H | H | H | $CH_3$ | $CH_3$ |
| $C_6H_5$ | H | S | H | H | H | H | H | H | $CH_3$ | $OCH_3$ |
| $C_6H_5$ | H | S | H | H | H | H | H | H | $OCH_3$ | $OCH_3$ |
| $C_6H_5$ | H | S | $CH_3$ | H | $CH_3$ | H | $CH_3$ | H | $CH_3$ | $OCH_3$ |
| $C_6H_5$ | H | S | $CH_3$ | H | H | H | $CH_3$ | H | $OCH_3$ | $OCH_3$ |
| $2'-C_6H_4Cl$ | H | S | H | H | H | H | H | H | $CH_3$ | $OCH_3$ |
| $2'-C_6H_4Br$ | H | S | $CH_3$ | H | $CH_3$ | H | H | H | $OCH_3$ | $OCH_3$ |
| $2'-C_6H_4F$ | H | S | H | H | H | H | H | $CH_3$ | $CH_3$ | $OCH_3$ |
| $3'-C_6H_4CH_3$ | H | S | H | H | H | H | H | H | $OCH_3$ | $OCH_3$ |
| $4'-C_6H_4OCH_3$ | H | S | H | H | H | H | H | H | $OCF_2H$ | $OCH_3$ |
| $CH_2CO_2CH_3$ | H | S | H | H | H | H | H | H | $CH_3$ | $OCH_3$ |
| $CH_2CO_2CH_3$ | H | S | H | H | $CH_3$ | $CH_3$ | H | H | $OCH_3$ | $OCH_3$ |
| $CH_2CO_2CH_3$ | H | S | H | H | H | H | H | H | Cl[b] | $OCH_3$ |
| $CH(CH_3)CO_2CH_3$ | H | S | H | H | H | H | H | H | $OCF_2H$ | $OCH_3$ |
| $OSO_2CH_3$ | H | S | H | H | H | H | H | H | $CH_3$ | $OCH_3$ |
| $OSO_2CH_3$ | H | S | H | H | H | H | H | H | $OCH_3$ | $OCH_3$ |
| $OSO_2CH_3$ | H | S | H | H | H | H | H | H | Cl[b] | $OCH_3$ |
| $OSO_2CH_3$ | H | S | H | H | H | H | H | H | $OCF_2H$ | $OCH_3$ |
| $OSO_2CF_3$ | H | S | H | H | H | H | H | $CH_3$ | $CH_3$ | $OCH_3$ |
| $CH_2OCH_3$ | H | S | H | H | H | H | H | H | $CH_3$ | $OCH_3$ |
| $CH_2OCH_3$ | H | S | H | H | H | H | H | H | $CH_3$ | $CH_3$ |
| $CH_2OCH_3$ | H | S | H | H | $CH_3$ | H | H | H | $OCF_2H$ | $OCH_3$ |
| $CH_2OCH_3$ | H | S | H | H | $CH_3$ | H | $CH_3$ | H | $CH_3$ | $CH_3$ |
| $CH_2OCF_2H$ | H | S | H | H | H | H | H | H | $CH_3$ | $OCH_3$ |
| $CH_2OCF_2H$ | H | S | H | H | H | H | H | H | $OCH_3$ | $OCH_3$ |

Table 13 (continued)

| $R_1$ | $R_3$ | W' | $R_{35}$ | $R_{36}$ | $R_{37}$ | $R_{38}$ | $R_{39}$ | $R_{40}$ | X | Y |
|---|---|---|---|---|---|---|---|---|---|---|
| $CH_2OCF_2H$ | H | S | $CH_3$ | H | H | H | $CH_3$ | H | Cl[b] | $OCH_3$ |
| $CH_2OCF_2H$ | H | S | $CH_3$ | $CH_3$ | $CH_3$ | H | $CH_3$ | H | $CH_3$ | $OCH_3$ |

Table 14

a.) Z = N

b.) Z = CH

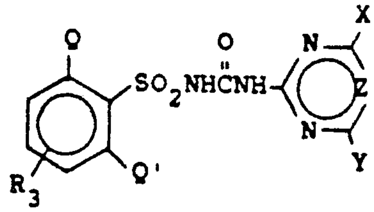

In the table below, $R_{18}$, $R_{19}$ and $R_{20}$-$R_{24}$ are H.

| Q | Q' | $R_3$ | X | Y |
|---|---|---|---|---|
| Q-1 | Q-1 | H | $CH_3$ | $CH_3$ |
| Q-1 | Q-2 | H | $CH_3$ | $OCH_3$ |
| Q-1 | Q-3 | H | $OCH_3$ | $OCH_3$ |
| Q-1 | Q-4 | H | Cl[b] | $OCH_3$ |
| Q-1 | Q-5 | H | $OCF_2H$ | $OCH_3$ |
| Q-1 | Q-6 | H | $CH_3$ | $CH_3$ |
| Q-1 | Q-7 | H | $CH_3$ | $OCH_3$ |
| Q-1 | Q-8 | H | $OCH_3$ | $OCH_3$ |
| Q-1 | Q-9 | H | Cl[b] | $OCH_3$ |
| Q-1 | Q-10 | H | $OCF_2H$ | $OCH_3$ |
| Q-1 | Q-11 | H | $CH_3$ | $CH_3$ |
| Q-1 | Q-12 | H | $CH_3$ | $OCH_3$ |
| Q-1 | Q-13 | H | $OCH_3$ | $OCH_3$ |
| Q-1 | Q-14 | H | Cl[b] | $OCH_3$ |
| Q-1 | Q-15 | H | $OCF_2H$ | $OCH_3$ |
| Q-1 | Q-16 | H | $CH_3$ | $CH_3$ |
| Q-1 | Q-17 | H | $CH_3$ | $OCH_3$ |
| Q-1 | Q-18 | H | $OCH_3$ | $OCH_3$ |
| Q-1 | Q-19 | H | Cl[b] | $OCH_3$ |
| Q-1 | Q-20 | H | $OCF_2H$ | $OCH_3$ |
| Q-2 | Q-2 | H | $CH_3$ | $CH_3$ |
| Q-2 | Q-3 | H | $CH_3$ | $OCH_3$ |
| Q-2 | Q-4 | H | $OCH_3$ | $OCH_3$ |

Table 14 (continued)

| Q | Q' | $R_3$ | X | Y |
|---|---|---|---|---|
| Q-2 | Q-5 | H | $Cl^b$ | $OCH_3$ |
| Q-2 | Q-6 | H | $OCF_2H$ | $OCH_3$ |
| Q-2 | Q-7 | H | $CH_3$ | $CH_3$ |
| Q-2 | Q-8 | H | $CH_3$ | $OCH_3$ |
| Q-2 | Q-9 | H | $OCH_3$ | $OCH_3$ |
| Q-2 | Q-10 | H | $Cl^b$ | $OCH_3$ |
| Q-2 | Q-11 | H | $OCF_2H$ | $OCH_3$ |
| Q-2 | Q-12 | H | $CH_3$ | $CH_3$ |
| Q-2 | Q-13 | H | $CH_3$ | $OCH_3$ |
| Q-2 | Q-14 | H | $OCH_3$ | $OCH_3$ |
| Q-2 | Q-15 | H | $Cl^b$ | $OCH_3$ |
| Q-2 | Q-16 | H | $OCF_2H$ | $OCH_3$ |
| Q-2 | Q-17 | H | $CH_3$ | $CH_3$ |
| Q-2 | Q-18 | H | $CH_3$ | $OCH_3$ |
| Q-2 | Q-19 | H | $OCH_3$ | $OCH_3$ |
| Q-2 | Q-20 | H | $Cl^b$ | $OCH_3$ |
| Q-3 | Q-3 | H | $OCF_2H$ | $OCH_3$ |
| Q-3 | Q-4 | H | $CH_3$ | $CH_3$ |
| Q-3 | Q-5 | H | $CH_3$ | $OCH_3$ |
| Q-3 | Q-6 | H | $OCH_3$ | $OCH_3$ |
| Q-3 | Q-7 | H | $Cl^b$ | $OCH_3$ |
| Q-3 | Q-8 | H | $OCF_2H$ | $OCH_3$ |
| Q-3 | Q-9 | H | $CH_3$ | $CH_3$ |
| Q-3 | Q-10 | H | $CH_3$ | $OCH_3$ |
| Q-3 | Q-11 | H | $OCH_3$ | $OCH_3$ |
| Q-3 | Q-12 | H | $Cl^b$ | $OCH_3$ |
| Q-3 | Q-13 | H | $OCF_2H$ | $OCH_3$ |
| Q-3 | Q-14 | H | $CH_3$ | $CH_3$ |
| Q-3 | Q-15 | H | $CH_3$ | $OCH_3$ |
| Q-3 | Q-16 | H | $OCH_3$ | $OCH_3$ |

Table 14 (continued)

| Q | Q' | $R_3$ | X | Y |
|---|---|---|---|---|
| Q-3 | Q-17 | H | Cl[b] | $OCH_3$ |
| Q-3 | Q-18 | H | $OCF_2H$ | $OCH_3$ |
| Q-3 | Q-19 | H | $CH_3$ | $CH_3$ |
| Q-3 | Q-20 | H | $CH_3$ | $OCH_3$ |
| Q-4 | Q-4 | H | $OCH_3$ | $OCH_3$ |
| Q-4 | Q-5 | H | Cl[b] | $OCH_3$ |
| Q-4 | Q-6 | H | $OCF_2H$ | $OCH_3$ |
| Q-4 | Q-7 | H | $CH_3$ | $CH_3$ |
| Q-4 | Q-8 | H | $CH_3$ | $OCH_3$ |
| Q-4 | Q-9 | H | $OCH_3$ | $OCH_3$ |
| Q-4 | Q-10 | H | Cl[b] | $OCH_3$ |
| Q-4 | Q-11 | H | $OCF_2H$ | $OCH_3$ |
| Q-4 | Q-12 | H | $CH_3$ | $CH_3$ |
| Q-4 | Q-13 | H | $CH_3$ | $OCH_3$ |
| Q-4 | Q-14 | H | $OCH_3$ | $OCH_3$ |
| Q-4 | Q-15 | H | Cl[b] | $OCH_3$ |
| Q-4 | Q-16 | H | $OCF_2H$ | $OCH_3$ |
| Q-4 | Q-17 | H | $CH_3$ | $CH_3$ |
| Q-4 | Q-18 | H | $CH_3$ | $OCH_3$ |
| Q-4 | Q-19 | H | $OCH_3$ | $OCH_3$ |
| Q-4 | Q-20 | H | Cl[b] | $OCH_3$ |
| Q-5 | Q-5 | H | $CH_3$ | $CH_3$ |
| Q-5 | Q-6 | H | $CH_3$ | $OCH_3$ |
| Q-5 | Q-7 | H | $OCH_3$ | $OCH_3$ |
| Q-5 | Q-8 | H | Cl[b] | $OCH_3$ |
| Q-5 | Q-9 | H | $OCF_2H$ | $OCH_3$ |
| Q-5 | Q-10 | H | $CH_3$ | $CH_3$ |
| Q-5 | Q-11 | H | $CH_3$ | $OCH_3$ |
| Q-5 | Q-12 | H | $CH_3$ | $OCH_3$ |
| Q-5 | Q-13 | H | $OCH_3$ | $OCH_3$ |

Table 14 (continued)

| Q | Q' | $R_3$ | X | Y |
|---|---|---|---|---|
| Q-5 | Q-14 | H | Cl[b] | $OCH_3$ |
| Q-5 | Q-15 | H | $OCF_2H$ | $OCH_3$ |
| Q-5 | Q-16 | H | $CH_3$ | $CH_3$ |
| Q-5 | Q-17 | H | $CH_3$ | $OCH_3$ |
| Q-5 | Q-18 | H | $OCH_3$ | $OCH_3$ |
| Q-5 | Q-19 | H | Cl[b] | $OCH_3$ |
| Q-5 | Q-20 | H | $OCF_2H$ | $OCH_3$ |
| Q-6 | Q-6 | H | $CH_3$ | $CH_3$ |
| Q-6 | Q-7 | H | $CH_3$ | $OCH_3$ |
| Q-6 | Q-8 | H | $OCH_3$ | $OCH_3$ |
| Q-6 | Q-9 | H | Cl[b] | $OCH_3$ |
| Q-6 | Q-10 | H | $OCF_2H$ | $OCH_3$ |
| Q-6 | Q-11 | H | $CH_3$ | $CH_3$ |
| Q-6 | Q-12 | H | $CH_3$ | $OCH_3$ |
| Q-6 | Q-13 | H | $OCH_3$ | $OCH_3$ |
| Q-6 | Q-14 | H | Cl[b] | $OCH_3$ |
| Q-6 | Q-15 | H | $OCF_2H$ | $OCH_3$ |
| Q-6 | Q-16 | H | $CH_3$ | $CH_3$ |
| Q-6 | Q-17 | H | $CH_3$ | $OCH_3$ |
| Q-6 | Q-18 | H | $OCH_3$ | $OCH_3$ |
| Q-6 | Q-19 | H | Cl[b] | $OCH_3$ |
| Q-6 | Q-20 | H | $OCF_2H$ | $OCH_3$ |
| Q-7 | Q-7 | H | $CH_3$ | $CH_3$ |
| Q-7 | Q-8 | H | $CH_3$ | $OCH_3$ |
| Q-7 | Q-9 | H | $OCH_3$ | $OCH_3$ |
| Q-7 | Q-10 | H | Cl[b] | $OCH_3$ |
| Q-7 | Q-11 | H | $OCF_2H$ | $OCH_3$ |
| Q-7 | Q-12 | H | $CH_3$ | $CH_3$ |
| Q-7 | Q-13 | H | $CH_3$ | $OCH_3$ |
| Q-7 | Q-14 | H | $OCH_3$ | $OCH_3$ |

Table 14 (continued)

| Q | Q' | $R_3$ | X | Y |
|---|---|---|---|---|
| Q-7 | Q-15 | H | Cl[b] | $OCH_3$ |
| Q-7 | Q-16 | H | $OCF_2H$ | $OCH_3$ |
| Q-7 | Q-17 | H | $CH_3$ | $CH_3$ |
| Q-7 | Q-18 | H | $CH_3$ | $OCH_3$ |
| Q-7 | Q-19 | H | $OCH_3$ | $OCH_3$ |
| Q-7 | Q-20 | H | Cl[b] | $OCH_3$ |
| Q-8 | Q-8 | H | $CH_3$ | $CH_3$ |
| Q-8 | Q-9 | H | $CH_3$ | $OCH_3$ |
| Q-8 | Q-10 | H | $OCH_3$ | $OCH_3$ |
| Q-8 | Q-11 | H | Cl[b] | $OCH_3$ |
| Q-8 | Q-12 | H | $OCF_2H$ | $OCH_3$ |
| Q-8 | Q-13 | H | $CH_3$ | $CH_3$ |
| Q-8 | Q-14 | H | $CH_3$ | $OCH_3$ |
| Q-8 | Q-15 | H | $OCH_3$ | $OCH_3$ |
| Q-8 | Q-16 | H | Cl[b] | $OCH_3$ |
| Q-8 | Q-17 | H | $OCF_2H$ | $OCH_3$ |
| Q-8 | Q-18 | H | $CH_3$ | $CH_3$ |
| Q-8 | Q-19 | H | $CH_3$ | $OCH_3$ |
| Q-8 | Q-20 | H | $OCH_3$ | $OCH_3$ |
| Q-9 | Q-9 | H | Cl[b] | $OCH_3$ |
| Q-9 | Q-10 | H | $OCF_2H$ | $OCH_3$ |
| Q-9 | Q-11 | H | $CH_3$ | $CH_3$ |
| Q-9 | Q-12 | H | $CH_3$ | $OCH_3$ |
| Q-9 | Q-13 | H | $OCH_3$ | $OCH_3$ |
| Q-9 | Q-14 | H | Cl[b] | $OCH_3$ |
| Q-9 | Q-15 | H | $CH_3$ | $CH_3$ |
| Q-9 | Q-16 | H | $CH_3$ | $OCH_3$ |
| Q-9 | Q-17 | H | $OCH_3$ | $OCH_3$ |
| Q-9 | Q-18 | H | Cl[b] | $OCH_3$ |

Table 14 (continued)

| Q | Q' | $R_3$ | X | Y |
|---|---|---|---|---|
| Q-9 | Q-19 | H | $OCF_2H$ | $OCH_3$ |
| Q-9 | Q-20 | H | $CH_3$ | $CH_3$ |
| Q-10 | Q-10 | H | $CH_3$ | $OCH_3$ |
| Q-10 | Q-11 | H | $OCH_3$ | $OCH_3$ |
| Q-10 | Q-12 | H | Cl[b] | $OCH_3$ |
| Q-10 | Q-13 | H | $OCF_2H$ | $OCH_3$ |
| Q-10 | Q-14 | H | $CH_3$ | $CH_3$ |
| Q-10 | Q-15 | H | $CH_3$ | $OCH_3$ |
| Q-10 | Q-16 | H | $OCH_3$ | $OCH_3$ |
| Q-10 | Q-17 | H | Cl[b] | $OCH_3$ |
| Q-10 | Q-18 | H | $OCF_2H$ | $OCH_3$ |
| Q-10 | Q-19 | H | $CH_3$ | $CH_3$ |
| Q-10 | Q-20 | H | $CH_3$ | $OCH_3$ |
| Q-11 | Q-11 | H | $OCH_3$ | $OCH_3$ |
| Q-11 | Q-12 | H | Cl[b] | $OCH_3$ |
| Q-11 | Q-13 | H | $OCF_2H$ | $OCH_3$ |
| Q-11 | Q-14 | H | $CH_3$ | $CH_3$ |
| Q-11 | Q-15 | H | $CH_3$ | $OCH_3$ |
| Q-11 | Q-15 | H | $OCH_3$ | $OCH_3$ |
| Q-11 | Q-16 | H | Cl[b] | $OCH_3$ |
| Q-11 | Q-17 | H | $OCF_2H$ | $OCH_3$ |
| Q-11 | Q-18 | H | $CH_3$ | $CH_3$ |
| Q-11 | Q-19 | H | $CH_3$ | $OCH_3$ |
| Q-11 | Q-20 | H | $OCH_3$ | $OCH_3$ |
| Q-12 | Q-12 | H | Cl[b] | $OCH_3$ |
| Q-12 | Q-13 | H | $OCF_2H$ | $OCH_3$ |
| Q-12 | Q-14 | H | $CH_3$ | $CH_3$ |
| Q-12 | Q-15 | H | $CH_3$ | $OCH_3$ |
| Q-12 | Q-16 | H | $OCH_3$ | $OCH_3$ |
| Q-12 | Q-17 | H | Cl[b] | $OCH_3$ |

Table 14 (continued)

| Q | Q' | $R_3$ | X | Y |
|---|---|---|---|---|
| Q-12 | Q-18 | H | $OCF_2H$ | $OCH_3$ |
| Q-12 | Q-19 | H | $CH_3$ | $CH_3$ |
| Q-12 | Q-20 | H | $CH_3$ | $OCH_3$ |
| Q-13 | Q-13 | H | $OCH_3$ | $OCH_3$ |
| Q-13 | Q-14 | H | Cl[b] | $OCH_3$ |
| Q-13 | Q-15 | H | $OCF_2H$ | $OCH_3$ |
| Q-13 | Q-16 | H | $CH_3$ | $CH_3$ |
| Q-13 | Q-17 | H | $CH_3$ | $OCH_3$ |
| Q-13 | Q-18 | H | $OCH_3$ | $OCH_3$ |
| Q-13 | Q-19 | H | Cl[b] | $OCH_3$ |
| Q-13 | Q-20 | H | $OCF_2H$ | $OCH_3$ |
| Q-14 | Q-14 | H | $CH_3$ | $CH_3$ |
| Q-14 | Q-15 | H | $CH_3$ | $OCH_3$ |
| Q-14 | Q-16 | H | $OCH_3$ | $OCH_3$ |
| Q-14 | Q-17 | H | Cl[b] | $OCH_3$ |
| Q-14 | Q-18 | H | $OCF_2H$ | $OCH_3$ |
| Q-14 | Q-19 | H | $CH_3$ | $CH_3$ |
| Q-14 | Q-20 | H | $CH_3$ | $OCH_3$ |
| Q-15 | Q-15 | H | $CH_3$ | $OCH_3$ |
| Q-15 | Q-16 | H | $OCH_3$ | $OCH_3$ |
| Q-15 | Q-17 | H | Cl[b] | $OCH_3$ |
| Q-15 | Q-18 | H | $OCF_2H$ | $OCH_3$ |
| Q-15 | Q-19 | H | $CH_3$ | $CH_3$ |
| Q-15 | Q-20 | H | $CH_3$ | $OCH_3$ |
| Q-16 | Q-16 | H | $OCH_3$ | $OCH_3$ |
| Q-16 | Q-17 | H | Cl[b] | $OCH_3$ |
| Q-16 | Q-18 | H | $OCF_2H$ | $OCH_3$ |
| Q-16 | Q-19 | H | $CH_3$ | $CH_3$ |
| Q-16 | Q-20 | H | $CH_3$ | $OCH_3$ |
| Q-17 | Q-17 | H | $OCH_3$ | $OCH_3$ |

Table 14 (continued)

| Q | Q' | $R_3$ | X | Y |
|---|---|---|---|---|
| Q-17 | Q-18 | H | Cl[b] | $OCH_3$ |
| Q-17 | Q-19 | H | $OCF_2H$ | $OCH_3$ |
| Q-17 | Q-20 | H | $CH_3$ | $CH_3$ |
| Q-18 | Q-18 | H | $CH_3$ | $OCH_3$ |
| Q-18 | Q-19 | H | $OCH_3$ | $OCH_3$ |
| Q-18 | Q-20 | H | Cl[b] | $OCH_3$ |
| Q-19 | Q-19 | H | $CH_3$ | $CH_3$ |
| Q-19 | Q-20 | H | $CH_3$ | $OCH_3$ |
| Q-20 | Q-20 | H | $OCH_3$ | $OCH_3$ |

Table 15

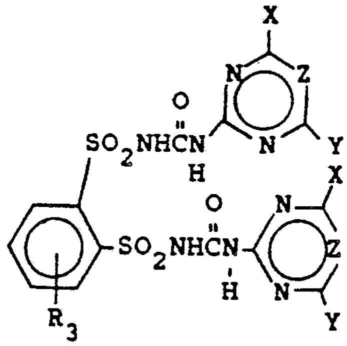

| $R_3$ | X | Y | Z | m.p.(°C) |
|---|---|---|---|---|
| H | $CH_3$ | $CH_3$ | CH | 186-189°C(d) |
| H | $OCH_3$ | $CH_3$ | CH | 145-146°C(d) |
| H | $OCH_3$ | $OCH_3$ | CH | 201-203°C(d) |
| H | $CH_3$ | $C_2H_5$ | CH | |
| H | $OCH_3$ | $C_2H_5$ | CH | |
| H | $CH_3$ | $OC_2H_5$ | CH | |
| H | $OCH_3$ | $OC_2H_5$ | CH | |
| H | $CH_3$ | $CH_2OCH_3$ | CH | |
| H | $OCH_3$ | $CH_2OCH_3$ | CH | |
| H | $OCH_3$ | $N(CH_3)_2$ | CH | |
| H | $CH_3$ | $CH_3$ | N | 123-125°C(d) |
| H | $OCH_3$ | $CH_3$ | N | 111-112°C(d) |
| H | $OCH_3$ | $OCH_3$ | N | 152-153°C(d) |
| H | $CH_3$ | $C_2H_5$ | N | |
| H | $OCH_3$ | $C_2H_5$ | N | |
| H | $CH_3$ | $OC_2H_5$ | N | |
| H | $OCH_3$ | $OC_2H_5$ | N | |
| H | $CH_3$ | $CH_2OCH_3$ | N | |
| H | $OCH_3$ | $CH_2OCH_3$ | N | |
| H | $OCF_2H$ | $OCH_3$ | N | |
| H | $CH_3$ | $HNCH_3$ | CH | |

Table 15 (continued)

| $R_3$ | X | Y | Z | m.p.(°C) |
|---|---|---|---|---|
| H | $OCH_3$ | $HNCH_3$ | CH | |
| H | $CH_3$ | $HNCH_3$ | N | |
| H | $OCH_3$ | $HNCH_3$ | N | |
| H | $OCH_3$ | $N(CH_3)_2$ | N | |
| H | Cl | $OCH_3$ | CH | |
| H | Cl | $N(CH_3)_2$ | CH | |
| H | $CH_3$ | $SCH_3$ | N | |
| H | $OCH_3$ | $SCH_3$ | N | |
| H | $CH_3$ | $SCH_3$ | CH | |
| H | $OCH_3$ | $SCH_3$ | CH | |
| 6-Cl | $OCH_3$ | $OCH_3$ | CH | |
| 6-Cl | $OCH_3$ | $N(CH_3)_2$ | CH | |
| 5-Cl | $CH_3$ | $OCH_3$ | CH | |
| 5-Cl | $CH_3$ | $CH_3$ | CH | |
| 4-Cl | $CH_3$ | $N(CH_3)_2$ | CH | |
| 4-Cl | $CH_3$ | $CH_3$ | N | |
| 3-Cl | $OCH_3$ | $CH_3$ | N | |
| 3-Cl | $OCH_3$ | $OCH_3$ | N | |
| 6-Br | $CH_3$ | $C_2H_5$ | N | |
| 6-Br | $OCH_3$ | $C_2H_5$ | N | |
| 5-Br | $CH_3$ | $OC_2H_5$ | N | |
| 5-Br | $OCH_3$ | $OC_2H_5$ | N | |
| 4-Br | $CH_3$ | $CH_2OCH_3$ | N | |
| 3-Br | $OCH_3$ | $CH_2OCH_3$ | N | |
| 6-F | $OCH_3$ | $OCH_3$ | N | |
| 5-F | $OCH_3$ | $CH_3$ | N | |
| 4-F | $OCH_3$ | $N(CH_3)_2$ | N | |
| 4-$OCH_3$ | Cl | $OCH_3$ | CH | |
| 3-$OCH_3$ | $CH_3$ | $CH_3$ | CH | |
| 6-$CF_3$ | $OCH_3$ | $CH_3$ | CH | |

Table 15 (continued)

| $R_3$ | X | Y | Z | m.p.(°C) |
|---|---|---|---|---|
| 5-$CF_3$ | $OCH_3$ | $OCH_3$ | CH | |
| 4-$CF_3$ | $CH_3$ | $C_2H_5$ | CH | |
| 4-$CF_3$ | $OCH_3$ | $C_2H_5$ | CH | |
| 3-$CF_3$ | $CH_3$ | $OC_2H_5$ | CH | |

Table 16a

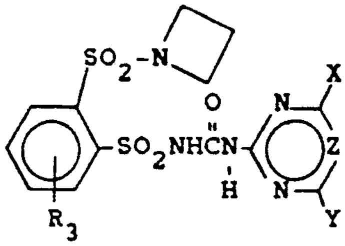

| $R_3$ | X | Y | Z | m.p.(°C) |
|---|---|---|---|---|
| H | $CH_3$ | $CH_3$ | CH | 200-205°C |
| H | $OCH_3$ | $CH_3$ | CH | 220-221°C |
| H | $OCH_3$ | $OCH_3$ | CH | 218-220°C |
| H | $CH_3$ | $C_2H_5$ | CH | |
| H | $OCH_3$ | $C_2H_5$ | CH | |
| H | $CH_3$ | $OC_2H_5$ | CH | |
| H | $OCH_3$ | $OC_2H_5$ | CH | |
| H | $CH_3$ | $CH_2OCH_3$ | CH | |
| H | $OCH_3$ | $CH_2OCH_3$ | CH | |
| H | $OCH_3$ | $N(CH_3)_2$ | CH | |
| H | $CH_3$ | $CH_3$ | N | 211°C |
| H | $OCH_3$ | $CH_3$ | N | 201°C |
| H | $OCH_3$ | $OCH_3$ | N | 175-177°C |
| H | $CH_3$ | $C_2H_5$ | N | |
| H | $OCH_3$ | $C_2H_5$ | N | |
| H | $CH_3$ | $OC_2H_5$ | N | |
| H | $OCH_3$ | $OC_2H_5$ | N | |
| H | $CH_3$ | $CH_2OCH_3$ | N | |
| H | $OCH_3$ | $CH_2OCH_3$ | N | |
| H | $OCF_2H$ | $OCH_3$ | N | |
| H | $OCH_3$ | $N(CH_3)_2$ | N | |
| H | Cl | $OCH_3$ | CH | |
| H | Cl | $N(CH_3)_2$ | CH | |
| H | $CH_3$ | $SCH_3$ | N | |
| H | $OCH_3$ | $SCH_3$ | N | |

Table 16a (continued)

| $R_3$ | X | Y | Z | m.p.(°C) |
|---|---|---|---|---|
| H | $CH_3$ | $SCH_3$ | CH | |
| H | $OCH_3$ | $SCH_3$ | CH | |
| 6-Cl | $OCH_3$ | $OCH_3$ | CH | |
| 6-Cl | $OCH_3$ | $N(CH_3)_2$ | CH | |
| 5-Cl | $CH_3$ | $OCH_3$ | CH | |
| 5-Cl | $CH_3$ | $CH_3$ | CH | |
| 4-Cl | $CH_3$ | $N(CH_3)_2$ | CH | |
| 4-Cl | $CH_3$ | $CH_3$ | N | |
| 3-Cl | $OCH_3$ | $CH_3$ | N | |
| 3-Cl | $OCH_3$ | $OCH_3$ | N | |
| 6-Br | $CH_3$ | $C_2H_5$ | N | |
| 6-Br | $OCH_3$ | $C_2H_5$ | N | |
| 5-Br | $CH_3$ | $OC_2H_5$ | N | |
| 5-Br | $OCH_3$ | $OC_2H_5$ | N | |
| 4-Br | $CH_3$ | $CH_2OCH_3$ | N | |
| 3-Br | $OCH_3$ | $CH_2OCH_3$ | N | |
| 6-F | $OCH_3$ | $OCH_3$ | N | |
| 5-F | $OCH_3$ | $CH_3$ | N | |
| 4-F | $OCH_3$ | $N(CH_3)_2$ | N | |
| 4-$OCH_3$ | Cl | $OCH_3$ | CH | |
| 3-$OCH_3$ | $CH_3$ | $CH_3$ | CH | |
| 6-$CF_3$ | $OCH_3$ | $CH_3$ | CH | |
| 5-$CF_3$ | $OCH_3$ | $OCH_3$ | CH | |
| 4-$CF_3$ | $CH_3$ | $C_2H_5$ | CH | |
| 4-$CF_3$ | $OCH_3$ | $C_2H_5$ | CH | |
| 3-$CF_3$ | $CH_3$ | $OC_2H_5$ | CH | |
| H | $CH_3$ | $NHCH_3$ | CH | |
| H | $OCH_3$ | $NHCH_3$ | CH | |
| H | $CH_3$ | $NHCH_3$ | N | |
| H | $OCH_3$ | $NHCH_3$ | N | |

Table 16b

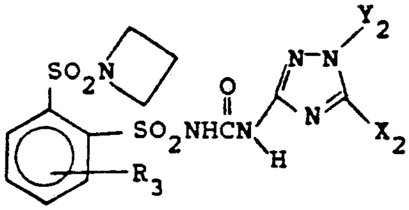

| $R_3$ | $Y_2$ | $X_2$ |
|---|---|---|
| H | $CH_3$ | $SCH_3$ |
| H | $CH_2CF_3$ | $SCH_3$ |
| H | $CH_3$ | $OCH_3$ |
| H | $C_2H_5$ | $SCH_3$ |
| H | $C_2H_5$ | $CH_3$ |
| H | $C_2H_5$ | $OCH_3$ |
| H | $CH_3$ | $CH_3$ |
| H | $CH_2CF_3$ | $OCH_3$ |
| 6-Cl | $CH_3$ | $OCH_3$ |
| 5-Cl | $CH_3$ | $OCH_3$ |
| 4-Cl | $CH_3$ | $OCH_3$ |
| 5-Br | $CH_3$ | $OCH_3$ |
| 4-Br | $CH_3$ | $OCH_3$ |
| 4-F | $CH_3$ | $OCH_3$ |
| 3-F | $CH_3$ | $OCH_3$ |
| 6-$OCH_3$ | $CH_3$ | $OCH_3$ |
| 5-$OCH_3$ | $CH_3$ | $OCH_3$ |
| 4-$OCH_3$ | $CH_3$ | $OCH_3$ |
| 6-$CF_3$ | $CH_3$ | $OCH_3$ |
| 5-$CF_3$ | $CH_3$ | $OCH_3$ |

Table 16c

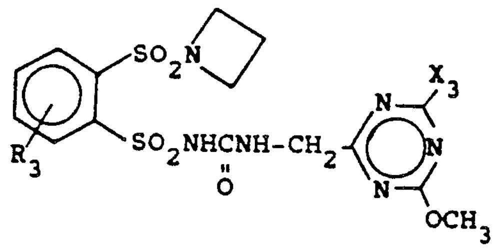

| $R_3$ | $X_3$ | m.p.(°C) |
|---|---|---|
| H | $CH_3$ | |
| H | $OCH_3$ | |
| 6-Cl | $CH_3$ | |
| 5-Br | $OCH_3$ | |
| 4-F | $CH_3$ | |
| 3-$CF_3$ | $OCH_3$ | |
| 5-$CH_3$ | $CH_3$ | |
| 4-$OCH_3$ | $OCH_3$ | |

Table 17

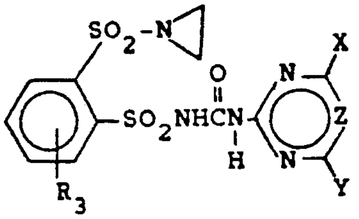

| $R_3$ | X | Y | Z | m.p.(°C) |
|---|---|---|---|---|
| H | $CH_3$ | $CH_3$ | CH | |
| H | $OCH_3$ | $CH_3$ | CH | |
| H | $OCH_3$ | $OCH_3$ | CH | |
| H | $CH_3$ | $C_2H_5$ | CH | |
| H | $OCH_3$ | $C_2H_5$ | CH | |
| H | $CH_3$ | $OC_2H_5$ | CH | |
| H | $OCH_3$ | $OC_2H_5$ | CH | |
| H | $CH_3$ | $CH_2OCH_3$ | CH | |
| H | $OCH_3$ | $CH_2OCH_3$ | CH | |
| H | $OCH_3$ | $N(CH_3)_2$ | CH | |
| H | $CH_3$ | $CH_3$ | N | |
| H | $OCH_3$ | $CH_3$ | N | |
| H | $OCH_3$ | $OCH_3$ | N | |
| H | $CH_3$ | $C_2H_5$ | N | |
| H | $OCH_3$ | $C_2H_5$ | N | |
| H | $CH_3$ | $OC_2H_5$ | N | |
| H | $OCH_3$ | $OC_2H_5$ | N | |
| H | $CH_3$ | $CH_2OCH_3$ | N | |
| H | $OCH_3$ | $CH_2OCH_3$ | N | |
| H | $OCF_2H$ | $OCH_3$ | N | |
| H | $OCH_3$ | $N(CH_3)_2$ | N | |

Table 18

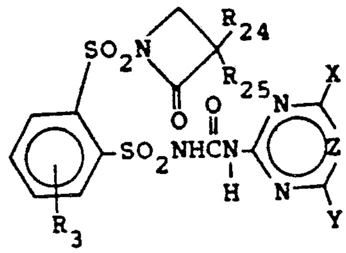

| $R_{24}$ | $R_{25}$ | $R_3$ | X | Y | Z | m.p.(°C) |
|---|---|---|---|---|---|---|
| $CH_3$ | $CH_3$ | H | $CH_3$ | $CH_3$ | CH | |
| $CH_3$ | $CH_3$ | H | $OCH_3$ | $CH_3$ | CH | |
| $CH_3$ | $CH_3$ | H | $OCH_3$ | $OCH_3$ | CH | |
| $CH_3$ | $CH_3$ | H | $CH_3$ | $C_2H_5$ | CH | |
| $CH_3$ | $CH_3$ | H | $OCH_3$ | $C_2H_5$ | CH | |
| $CH_3$ | $CH_3$ | H | $CH_3$ | $OC_2H_5$ | CH | |
| $CH_3$ | $CH_3$ | H | $OCH_3$ | $OC_2H_5$ | CH | |
| $CH_3$ | $CH_3$ | H | $CH_3$ | $CH_2OCH_3$ | CH | |
| $CH_3$ | $CH_3$ | H | $OCH_3$ | $CH_2OCH_3$ | CH | |
| $CH_3$ | $CH_3$ | H | $OCH_3$ | $N(CH_3)_2$ | CH | |
| $CH_3$ | $CH_3$ | H | $CH_3$ | $CH_3$ | N | |
| $CH_3$ | $CH_3$ | H | $OCH_3$ | $CH_3$ | N | |
| $CH_3$ | $CH_3$ | H | $OCH_3$ | $OCH_3$ | N | |
| H | $CH_3$ | H | $CH_3$ | $C_2H_5$ | N | |
| H | $CH_3$ | H | $OCH_3$ | $C_2H_5$ | N | |
| H | $CH_3$ | H | $CH_3$ | $OC_2H_5$ | N | |
| H | $CH_3$ | H | $OCH_3$ | $OC_2H_5$ | N | |
| H | $CH_3$ | H | $CH_3$ | $CH_2OCH_3$ | N | |
| H | $CH_3$ | H | $OCH_3$ | $CH_2OCH_3$ | N | |
| H | H | H | $OCF_2H$ | $OCH_3$ | N | |
| H | H | H | $OCH_3$ | $N(CH_3)_2$ | N | |
| H | H | H | $CH_3$ | $CH(OCH_3)_2$ | CH | |

Table 19a

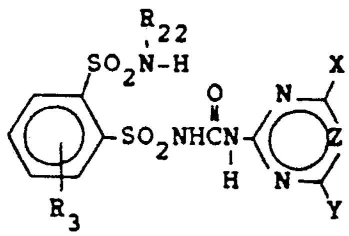

| $R_{22}$ | $R_3$ | X | Y | Z | m.p.(°C) |
|---|---|---|---|---|---|
| $\overset{O}{\overset{\|}{C}}CF_3$ | H | $OCH_3$ | $C_2H_5$ | CH | |
| $\overset{O}{\overset{\|}{C}}Ph$ | H | $CH_3$ | $OC_2H_5$ | CH | |
| $\overset{O}{\overset{\|}{C}}OCH_3$ | H | $OCH_3$ | $OC_2H_5$ | CH | |
| $\overset{O}{\overset{\|}{C}}OCH_2CH_3$ | H | $CH_3$ | $CH_2OCH_3$ | CH | |
| $\overset{O}{\overset{\|}{C}}O(CH_2)_3CH_3$ | H | $OCH_3$ | $CH_2OCH_3$ | CH | |
| $\overset{O}{\overset{\|}{C}}NH_2$ | H | $OCH_3$ | $N(CH_3)_2$ | CH | |
| $\overset{O}{\overset{\|}{C}}NHCH_3$ | H | $CH_3$ | $CH_3$ | N | |
| $\overset{O}{\overset{\|}{C}}NHCH_2CH_3$ | H | $OCH_3$ | $CH_3$ | N | |
| $\overset{O}{\overset{\|}{C}}NH(CH_2)_3CH_3$ | H | $OCH_3$ | $OCH_3$ | N | |
| $\overset{O}{\overset{\|}{C}}NHPh$ | H | $CH_3$ | $C_2H_5$ | N | |
| $\overset{O}{\overset{\|}{C}}N(CH_3)_2$ | H | $OCH_3$ | $C_2H_5$ | N | |
| $\overset{O}{\overset{\|}{C}}N(CH_3)CH_2CH_3$ | H | $CH_3$ | $OC_2H_5$ | N | |
| $\overset{O}{\overset{\|}{C}}N(CH_3)Ph$ | H | $OCH_3$ | $OC_2H_5$ | N | |
| $\overset{O}{\overset{\|}{C}}N(CH_2CH_3)_2$ | H | $CH_3$ | $CH_2OCH_3$ | N | |
| $\overset{O}{\overset{\|}{C}}N(CH_2CH_3)Ph$ | H | $OCH_3$ | $CH_2OCH_3$ | N | |

Table 19a (continued)

| $R_{22}$ | $R_3$ | X | Y | Z | m.p.(°C) |
|---|---|---|---|---|---|
| 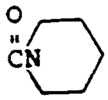 | H | $OCF_2H$ | $OCH_3$ | N | |
| 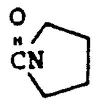 | H | $OCH_3$ | $N(CH_3)_2$ | N | |
| $\overset{O}{\overset{\parallel}{C}}CF_3$ | 4-Cl | $CH_3$ | $N(CH_3)_2$ | CH | |
| $\overset{O}{\overset{\parallel}{C}}Ph$ | 4-Cl | $CH_3$ | $CH_3$ | N | |
| $\overset{O}{\overset{\parallel}{C}}OCH_3$ | 3-Cl | $OCH_3$ | $CH_3$ | N | |
| $\overset{O}{\overset{\parallel}{C}}OCH_2CH_3$ | 3-Cl | $OCH_3$ | $OCH_3$ | N | |
| $\overset{O}{\overset{\parallel}{C}}O(CH_2)_3CH_3$ | 6-Br | $OCH_3$ | $C_2H_5$ | N | |
| $\overset{O}{\overset{\parallel}{C}}NH_2$ | 5-Br | $CH_3$ | $OC_2H_5$ | N | |
| $\overset{O}{\overset{\parallel}{C}}NHCH_3$ | 5-Br | $OCH_3$ | $OC_2H_5$ | N | |
| $\overset{O}{\overset{\parallel}{C}}NH_2$ | 4-Br | $CH_3$ | $CH_2OCH_3$ | N | |
| $\overset{O}{\overset{\parallel}{C}}NHCH_2CH_3$ | 3-Br | $OCH_3$ | $CH_2OCH_3$ | N | |
| $\overset{O}{\overset{\parallel}{C}}NH(CH_2)_3CH_3$ | 6-F | $OCH_3$ | $OCH_3$ | N | |
| $\overset{O}{\overset{\parallel}{C}}NHPh$ | 5-F | $OCH_3$ | $CH_3$ | N | |
| $\overset{O}{\overset{\parallel}{C}}N(CH_3)_2$ | 4-F | $OCH_3$ | $N(CH_3)_2$ | N | |
| 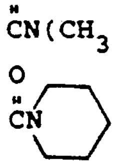 | 4-$OCH_3$ | Cl | $OCH_3$ | CH | |
| 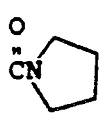 | 3-$OCH_3$ | $CH_3$ | $CH_3$ | CH | |
| 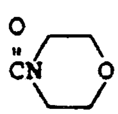 | 6-$CF_3$ | $OCH_3$ | $CH_3$ | CH | |

Table 19a (continued)

| $R_{22}$ | $R_3$ | X | Y | Z | m.p.(°C) |
|---|---|---|---|---|---|
| $\overset{O}{\overset{\|}{C}}OCH_3$ | 4-$CF_3$ | $OCH_3$ | $C_2H_5$ | CH | |
| $\overset{O}{\overset{\|}{C}}NH_2$ | 3-$CF_3$ | $CH_3$ | $OC_2H_5$ | CH | |
| $CF_2H$ | H | $OCH_3$ | $CH_3$ | CH | |
| $CF_2H$ | H | $CH_3$ | $CH_3$ | CH | |
| $CF_2H$ | H | $OCH_3$ | $CH_3$ | N | |
| $CF_2H$ | H | $OCH_3$ | $OCH_3$ | N | |

Table 19b

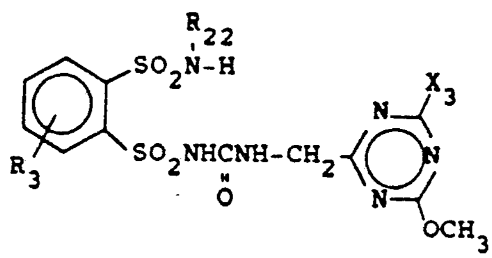

| $R_3$ | $R_{22}$ | $X_3$ | m.p.(°C) |
|---|---|---|---|
| H | $\overset{O}{\overset{\|}{C}}OCH_2CH_3$ | $CH_3$ | |
| H | $CF_2H$ | $OCH_3$ | |

Table 20

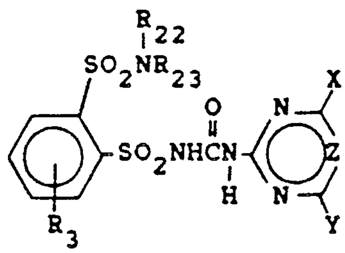

| $R_{22}$ | $R_3$ | $R_{23}$ | X | Y | Z | m.p.(°C) |
|---|---|---|---|---|---|---|
| $\overset{O}{\parallel}CCF_3$ | H | $CH_3$ | $OCH_3$ | $C_2H_5$ | CH | |
| $\overset{O}{\parallel}CPh$ | H | $C_2H_5$ | $CH_3$ | $OC_2H_5$ | CH | |
| $\overset{O}{\parallel}COCH_3$ | H | $CH_3$ | $OCH_3$ | $OC_2H_5$ | CH | |
| $\overset{O}{\parallel}COCH_2CH_3$ | H | $CH_3$ | $CH_3$ | $CH_2OCH_3$ | CH | |
| $\overset{O}{\parallel}CO(CH_2)_3CH_3$ | H | $CH_3$ | $OCH_3$ | $CH_2OCH_3$ | CH | |
| $\overset{O}{\parallel}CNH_2$ | H | n-$C_3H_7$ | $OCH_3$ | $N(CH_3)_2$ | CH | |
| $\overset{O}{\parallel}CNHCH_3$ | H | $CH_3$ | $CH_3$ | $CH_3$ | N | |

Table 21

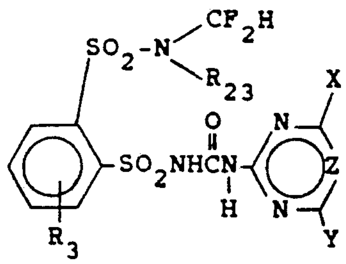

| $R_3$ | $R_{23}$ | X | Y | Z | m.p.(°C) |
|---|---|---|---|---|---|
| H | $CH_3$ | $CH_3$ | $CH_3$ | CH | 210-215°C (dec) |
| H | $CH_3$ | $OCH_3$ | $CH_3$ | CH | 210-211°C |
| H | $CH_3$ | $OCH_3$ | $OCH_3$ | CH | 201-203°C |
| H | $CH_3$ | $CH_3$ | $C_2H_5$ | CH | |
| H | $CH_3$ | $OCH_3$ | $C_2H_5$ | CH | |
| H | $CH_3$ | $CH_3$ | $OC_2H_5$ | CH | |
| H | $CH_3$ | $OCH_3$ | $OC_2H_5$ | CH | |
| H | $CH_3$ | $CH_3$ | $CH_2OCH_3$ | CH | |
| H | $CH_3$ | $OCH_3$ | $CH_2OCH_3$ | CH | |
| H | $CH_3$ | $OCH_3$ | $N(CH_3)_2$ | CH | |
| H | $CH_3$ | $CH_3$ | $CH_3$ | N | |
| H | $CH_3$ | $OCH_3$ | $CH_3$ | N | 185-186°C |
| H | $CH_3$ | $OCH_3$ | $OCH_3$ | N | 179-181°C |
| H | $CH_3$ | $CH_3$ | $C_2H_5$ | N | |
| H | $CH_3$ | $OCH_3$ | $C_2H_5$ | N | |
| H | $CH_3$ | $CH_3$ | $OC_2H_5$ | N | |
| H | $CH_3$ | $OCH_3$ | $OC_2H_5$ | N | |
| H | $CH_3$ | $CH_3$ | $CH_2OCH_3$ | N | |
| H | $CH_3$ | $OCH_3$ | $CH_2OCH_3$ | N | |
| H | $CH_3$ | $OCF_2H$ | $OCH_3$ | N | |
| H | $CH_3$ | $OCH_3$ | $N(CH_3)_2$ | N | |
| H | $C_2H_5$ | $CH_3$ | $CH_3$ | CH | |
| H | $C_2H_5$ | $OCH_3$ | $CH_3$ | CH | |
| H | $C_2H_5$ | $OCH_3$ | $OCH_3$ | CH | |

Table 21 (continued)

| $R_3$ | $R_{23}$ | X | Y | Z | m.p. (°C) |
|---|---|---|---|---|---|
| H | $C_2H_5$ | $CH_3$ | $C_2H_5$ | CH | |
| H | $C_2H_5$ | $OCH_3$ | $C_2H_5$ | CH | |
| H | $C_2H_5$ | $CH_3$ | $OC_2H_5$ | CH | |
| H | $C_2H_5$ | $OCH_3$ | $CH_3$ | N | |
| H | $C_2H_5$ | $CH_3$ | $CH_3$ | N | |
| H | $C_2H_5$ | $OCH_3$ | $OCH_3$ | N | |
| H | $C_2H_5$ | $OCH_3$ | $N(CH_3)_2$ | CH | |
| H | $n-C_3H_7$ | $CH_3$ | $CH_3$ | N | |
| H | $n-C_3H_7$ | $OCH_3$ | $CH_3$ | N | |
| H | $n-C_3H_7$ | $OCH_3$ | $OCH_3$ | N | |
| H | $n-C_3H_7$ | $CH_3$ | $C_2H_5$ | N | |
| H | $n-C_3H_7$ | $OCH_3$ | $C_2H_5$ | N | |
| H | $n-C_3H_7$ | $CH_3$ | $OC_2H_5$ | N | |
| H | $n-C_3H_7$ | $OCH_3$ | $CH_3$ | CH | |
| H | $n-C_3H_7$ | $CH_3$ | $CH_3$ | CH | |
| H | $n-C_3H_7$ | $OCH_3$ | $OCH_3$ | CH | |
| H | $n-C_3H_7$ | $OCF_2H$ | $OCH_3$ | N | |
| H | $n-C_3H_7$ | $OCH_3$ | $N(CH_3)_2$ | N | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table 22

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carrriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering*, December 4, 1967, pp. 147ff and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley and Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 18

Wettable Powder

| | |
|---|---|
| N-[(4,6-dimethoxypyrimidin-2-yl)aminocarbonyl]-3-methylsulfonyl-1,1′-biphenyl-2-sulfonamide | 80% |
| sodium alkylnaphthalenesulfonate | 2% |
| sodium lininsulfonate | 2% |
| synthetic amorphous silica | 3% |
| kaolinite | 13% |

The ingredients are blended, hammer-milled until all the solids are essentially under 50 microns, reblended, and packaged.

Example 19

Wettable Powder

| | |
|---|---|
| N-[(4,6-dimethoxy-1,3,5-triazin-2-yl)aminocarbonyl]-2,6-*bis*(methylthio)benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 20

Granule

| | |
|---|---|
| Wettable Powder of Example 19 | 5% |
| attapulgite granules (U.S.S. 20—40 mesh; 0.84—0.42 mm) | 95% |

A slurry of wettable powder containing 25% solids is sprayed on the surface of attapulgite granules in a double-cone blender. The granules are dried and packaged.

Example 21

Extruded Pellet

| | |
|---|---|
| N-[(4,6-dimethoxypyrimidin-2-yl)aminocarbonyl]-3-methylsulfonyl-1,1′-biphenyl-2-sulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 22

Wettable Powder

| | |
|---|---|
| 2-(Azetidin-1-ylsulfonyl)-N-[(4-methoxy-6-methylpyrimidin-2-yl)aminocarbonyl]benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 23

Dust

| | |
|---|---|
| 2-(Azetidin-1-ylsulfonyl)-N-[(4,6-dimethoxy-1,3,5-triazin-2-yl)aminocarbonyl]benzenesulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 24

Oil Suspension

| | |
|---|---|
| N,N′-bis(4,6-Dimethoxypyrimidin-2-yl)aminocarbonyl]-1,2-benzenedisulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Other formulations of compounds of Formula I may be compounded as illustrated in our EP—A—0135332.

Utility

Test results indicate that the compounds of the present invention are active herbicides. They should have utility for broad-spectrum pre and/or postemergence weed control in areas where complete control of all vegetation is desired, such as around fuel storage tanks, ammunition depots, industrial storage areas, parking lots, drive-in theaters, around billboards, highway and railroad structures. Alternatively, many of the subject compounds should be useful for the selective pre or postemergence weed control in crops, especially wheat, sugar beets and rice.

The rates of application for the compounds of the invention are determined by a number of factors, including their use as selective or general herbicides, the crop species involved, the types of weeds to be controlled, weather and climate, formulations selected, mode of application, amount of foliage present, etc. In general terms, the subject compounds should be applied at levels of around 0.005 to 10 kg/ha, the lower rates being suggested for use on lighter soils and/or those having a low organic matter content, or for situations where only short-term persistence is required.

The compounds of the invention may be used in combination with any other commercial herbicide, examples of which are those of the triazine, triazole, uracil, urea, amide, diphenylether, carbamate and bipyridylium types.

The herbicidal properties of the subject compounds were discovered in a number of greenhouse tests. The test procedures and results follow.

Test A

Seeds of crabgrass (*Digitaria* spp.), barnyard grass (*Echinochloa crusgalli*), wild oats (*Avena fatua*), sicklepod (*Cassia obtusifolia*), morningglory (*Ipomoea* spp.), cocklebur (*Xanthium pensylvanicum*), sorghum, corn, soybean, sugar beet, rice, wheat, and purple nutsedge (*Cyperus rotundus*) tubers were planted and treated pre-emergence with the test chemicals dissolved in a non-phytotoxic solvent. At the same time, these crop and weed species, along with cotton and bush bean, were treated with a soil-foliage application. At the time of treatment, the plants ranged in height from 2 to 18 cm. Treated plants and

controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

C = chlorosis/necrosis;
D = defoliation;
E = emergence inhibition;
G = growth retardation;
H = formative effect;
U = unusual pigmentation; and
6Y = abscised buds or flowers.

Compounds

Compound 1

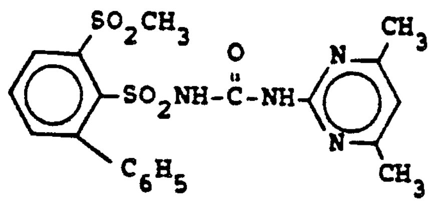

Compound 2

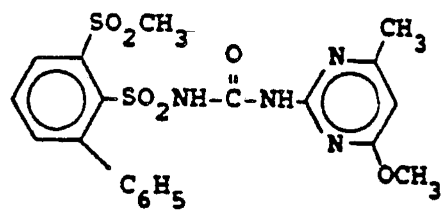

Compound 3

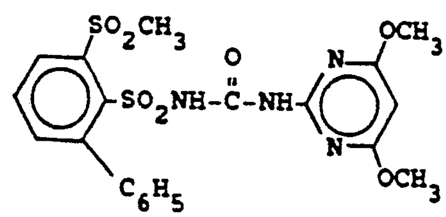

Compound 4

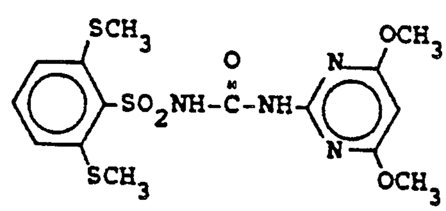

Compounds (continued)

Compound 5

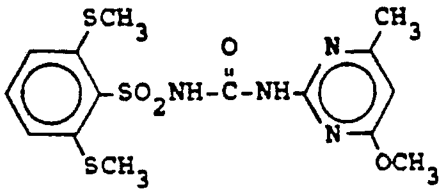

Compound 6

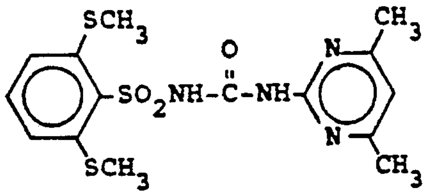

Compound 7

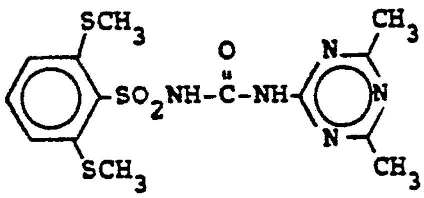

Compound 8

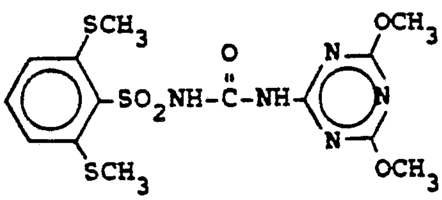

Compound 9

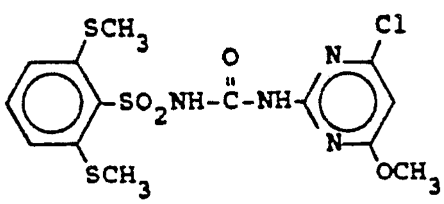

Compounds (continued)

Compound 10

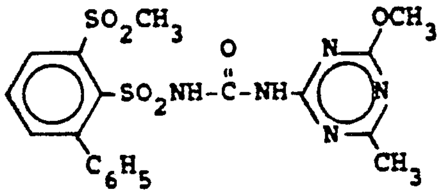

Compound 11

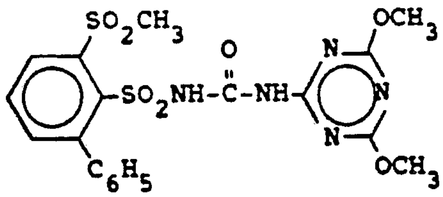

Compound 12

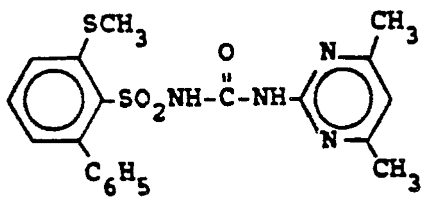

Compound 13

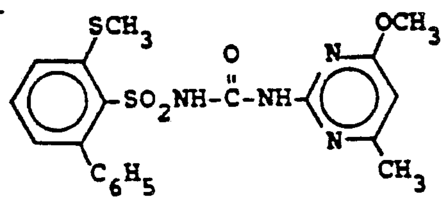

Compound 14

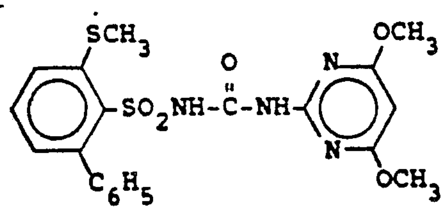

Compounds (continued)

Compound 15

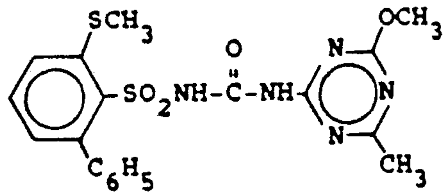

Compound 16

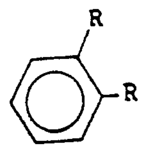

R is 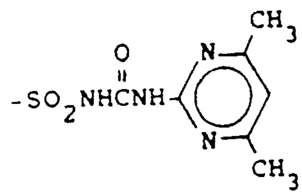

Compound 17

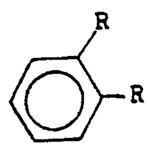

R is 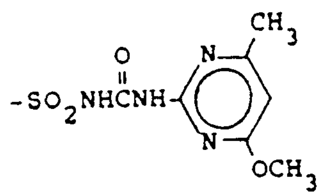

Compound 18

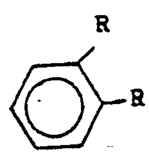

R is 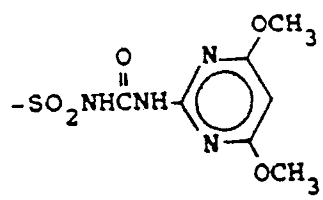

Compound 19

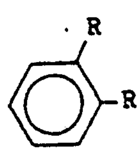

R is 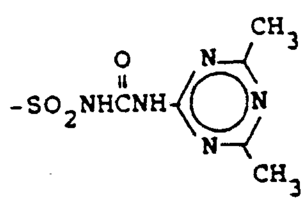

Compound 20

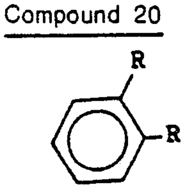

R is 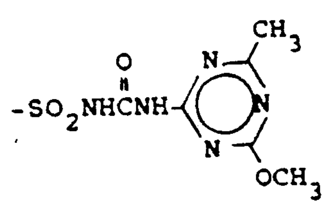

Compounds (continued)

Compound 21

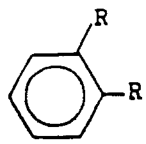

R is 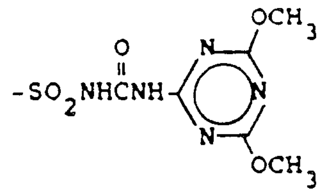

Compound 22

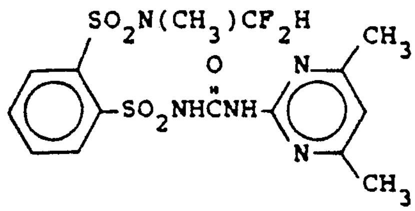

Compound 23

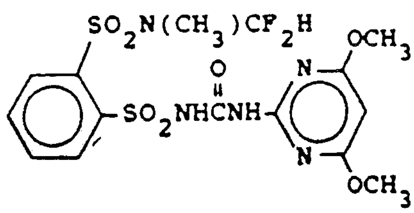

Compound 24

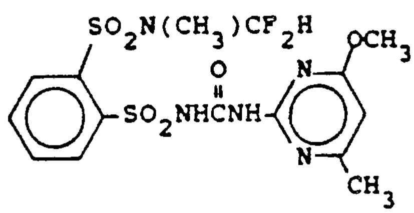

Compound 25

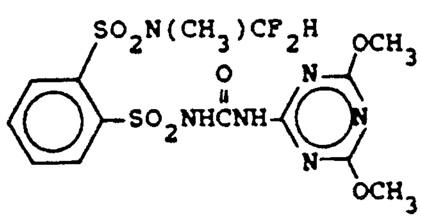

Compound 26

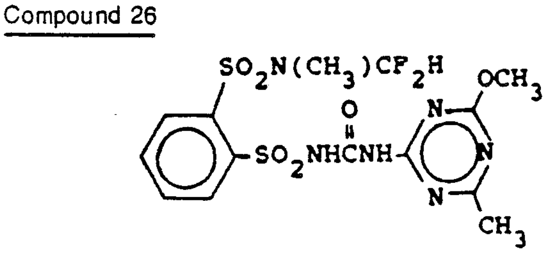

Compounds (continued)

Compound 27

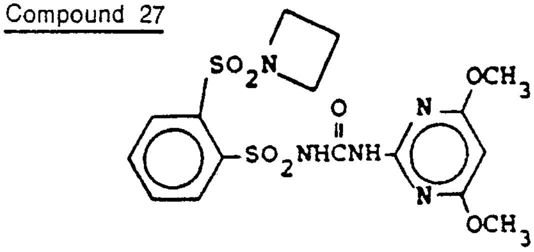

Compound 28

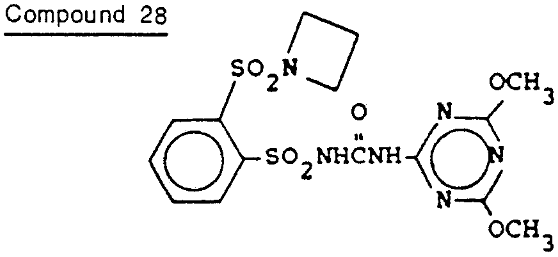

Compound 29

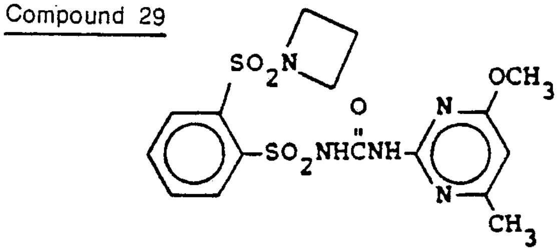

Compound 30

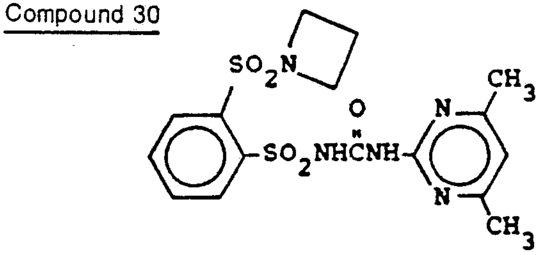

Compound 31

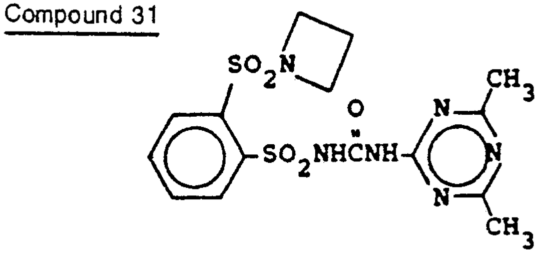

Compound 32

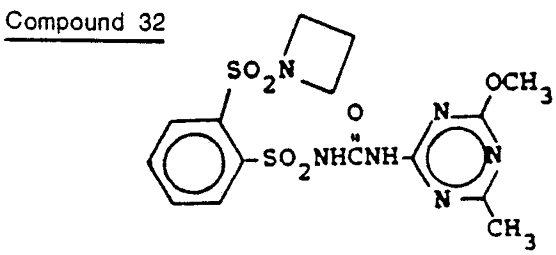

Table A

| | Cmpd. 1 | Compound 2 | | Cmpd. 3 |
|---|---|---|---|---|
| Rate g/ha | 50 | 50 | 400 | 50 |
| POSTEMERGENCE | | | | |
| Bush bean | 3C,3H | 5C,9G,6Y | 6C,9G,6Y | 4C,8H,6Y |
| Cotton | 4C,8G | 4C,9G | 4C,9G | 4C,9G |
| Morningglory | 9C | 4C,9G | 5C,9G | 5C,9G |
| Cocklebur | 9C | 9C | 9C | 5C,9G |
| Sicklepod | 4C,3H | 2C,2H | 2C,6G | 4C,9G |
| Nutsedge | 4C,9G | 8G | 4C,9G | 4C,9G |
| Crabgrass | 4G | 2C,5G | 2C,8G | 2C,5G |
| Barnyardgrass | 4C,9H | 2C,7H | 5C,9G | 3C,9H |
| Wild Oats | 3C,5G | 2C,4G | 4C,9G | 2C |
| Wheat | 5G | 3G | 9G | 3G |
| Corn | 2C,6H | 2C,9H | 1U,9G | 2C,8H |
| Soybean | 5C,9G | 5C,9G | 5C,9G | 5C,9G |
| Rice | 2C,7G | 2C,4G | 2C,8G | 1C,1H |
| Sorghum | 3C,8H | 3C,9G | 5C,9G | 2C,9G |
| Sugar beet | 2C,8G | 2C | 4C,8G | 2C,8G |
| PREEMERGENCE | | | | |
| Morningglory | 2C,9G | 2C,8H | 9C | 4C,9G |
| Cocklebur | 9H | 9H | 9H | 9H |
| Sicklepod | 3C,6G | 2C,2G | 2C,9G | 4C,5H |
| Nutsedge | 2C,6G | 5G | 10E | 4C,6G |
| Crabgrass | 2G | 2G | 7G | 2G |
| Barnyardgrass | 5C,6G | 3C,7G | 5C,9H | 5C,6G |
| Wild Oats | 2C,8G | 3C,8G | 5C,9H | 3C |
| Wheat | 8G | 2C,8G | 3C,9H | 1C,4G |
| Corn | 2C,8H | 2C,9H | 4C,9H | 9G |
| Soybean | 4C,5G | 4C,6H | 9H | 5C,6H |
| Rice | 4C,6G | 4C,6G | 4C,9H | 3C,3G |
| Sorghum | 4C,9H | 3C,9H | 10H | 5C,9G |
| Sugar beet | 3C,9G | 2C,9G | 9G | 8G |

Table A (continued)

| | Cmpd. 4 | Cmpd. 5 | Cmpd. 6 | Cmpd. 7 |
|---|---|---|---|---|
| Rate g/ha | 50 | 50 | 50 | 50 |
| POSTEMERGENCE | | | | |
| Bush bean | 4C,9G,6Y | 2C,7G,6Y | 4C,8H,6Y | 3C,8G,6Y |
| Cotton | 5C,9G | 4C,9G | 5C,9H | 2C,2H,5G |
| Morningglory | 5C,9G | 4C,9G | 4C,7G | 2C |
| Cocklebur | 9C | 9C | 9C | 3C,8H |
| Sicklepod | 9C | 5C,9G | 5C,9G | 2C,5G |
| Nutsedge | 9C | 2C,9G | 5C,9G | 1C,5G |
| Crabgrass | 2C,9G | 2C,8G | 2C,8G | 5G |
| Barnyardgrass | 9C | 9C | 9C | 3C,9H |
| Wild Oats | 9C | 9C | 5C,9G | 5G,2C |
| Wheat | 9C | 10C | 9C | 1C,3G |
| Corn | 9C | 3U,9G | 2C,9H | 2C,9H |
| Soybean | 4C,9G | 5C,9G | 5C,9G | 3C,7G |
| Rice | 5C,9G | 5C,9G | 5C,9G | 4C,9G |
| Sorghum | 5U,9G | 5C,9G | 4C,9G | 3C,9G |
| Sugar beet | 9C | 9C | 9C | 2C,8G |
| PREEMERGENCE | | | | |
| Morningglory | 9C | 9C | 9G | 8G |
| Cocklebur | 9H | 9H | 9H | - |
| Sicklepod | 9C | 4C,9G | 9G | 9G |
| Nutsedge | 10E | 10E | 10E | 0 |
| Crabgrass | 4C,9G | 3C,5G | 1C | 2G |
| Barnyardgrass | 5C,9H | 5C,9H | 5C,9H | 2C,8H |
| Wild Oats | 5C,9G | 5C,9H | 5C,9G | 2C,8G |
| Wheat | 10C | 10H | 4C,9H | 7G |
| Corn | 10E | 9H | 3C,9H | 2C,8G |
| Soybean | 9H | 8H | 9H | 1C,3G |
| Rice | 10E | 10E | 10E | 2C,9H |
| Sorghum | 5C,9H | 10H | 5C,9H | 5C,9H |
| Sugar beet | 10C | 9C | 5C,9G | 5C,9G |

Table A (continued)

| | Cmpd. 8 | Cmpd. 9 |
|---|---|---|
| Rate g/ha | 50 | 50 |
| POSTEMERGENCE | | |
| Bush bean | 5C,9G,6Y | 2H |
| Cotton | 4C,9G | 2C |
| Morningglory | 2C,4G | 1C |
| Cocklebur | 3C,9H | 2C,5G |
| Sicklepod | 4C,9G | 5G |
| Nutsedge | 4G | 2G |
| Crabgrass | 2C,5G | 5G |
| Barnyardgrass | 2C,8H | 2C,3G |
| Wild Oats | 2C,8H | 0 |
| Wheat | 6C,9G | 0 |
| Corn | 4C,9H | 2C,3G |
| Soybean | 3C,9G | 2C,5G |
| Rice | 5C,9G | 2C |
| Sorghum | 2C,9G | 9H |
| Sugar beet | 3C,9G | 2C,8G |
| PREEMERGENCE | | |
| Morningglory | 8G | 7G |
| Cocklebur | 9H | 8H |
| Sicklepod | 7G | 6G |
| Nutsedge | 10E | 10E |
| Crabgrass | 0 | 2G |
| Barnyardgrass | 2C,5G | 2C,7G |
| Wild Oats | 2C,7G | 2C,7G |
| Wheat | 2C,7G | 9G |
| Corn | 2C,7G | 2C,5G |
| Soybean | 1C,1H | 1C,1H |
| Rice | 3C,9H | 3C,9H |
| Sorghum | 2C,9H | 5C,9H |
| Sugar beet | 3C,9G | 3C,9G |

Table A (continued)

| | Cmpd. 10 | Cmpd. 11 | Cmpd. 12 | Cmpd. 13 |
|---|---|---|---|---|
| Rate g/ha | 50 | 50 | 50 | 50 |
| POSTEMERGENCE | | | | |
| Cotton | 5C,9G | 5C,9G | 2C,8H | 2C,8G |
| Morningglory | &C | &C | 3C,9G | 3C,8G |
| Cocklebur | 9C | 9C | 5C,9G | 3C,9G |
| Sicklepod | 4C,8G | 4C,7G | 3C | 2C |
| Nutsedge | 6C,9G | 4C,9G | 2C,8G | 3C,9G |
| Crabgrass | 5C,8G | 0 | 2G | 0 |
| Barnyardgrass | &C | 9C | 3C,8H | 3C,8H |
| Wild Oats | 9C | 9C | 5G | 0 |
| Wheat | 9C | 3C,9G | 0 | 2G |
| Corn | 3C,9G | 2U,9G | 0 | 0 |
| Soybean | 3C,9G | 4C,9G | 2C,9G | 2C,9G |
| Rice | 3G | 4C,9G | 5G | 4G |
| Sorghum | &C | 9C | 2C,8G | 2C,9G |
| Sugar beet | &C | &C | 5G | 5G |
| PREEMERGENCE | | | | |
| Morningglory | 9G | 9H | 2C,7H | 9C |
| Cocklebur | 9H | 9H | 9H | 9H |
| Sicklepod | 5C,8G | 6G,5C | 2C,7G | 8G |
| Nutsedge | 2C,5G | 0 | 5G | 2C,9G |
| Crabgrass | 1C | 0 | 2C,6H | 0 |
| Barnyardgrass | 1C | 2C | 2C,6H | 3C,8H |
| Wild Oats | 3C,9G | 5C,9G | 2C,9G | 2C,8G |
| Wheat | 2C,4G | 8G | 2G | 2C,7G |
| Corn | 3c,9G | 9G | 5G | 2C,8G |
| Soybean | 3C,7H | 3C,8H | 2C;3H | 3C,7H |
| Rice | 3C,4G | 5G | 2C,6G | 2C,6G |
| Sorghum | 5C,9H | 6C,9H | 2C,9H | 2C,9H |
| Sugar beet | 9C | &C | 8G | 2C,9G |

Table A (continued)

| | Cmpd. 14 | Cmpd. 15 |
|---|---|---|
| Rate g/ha | 50 | 50 |
| POSTEMERGENCE | | |
| Cotton | 2C,8G | 2C,8G |
| Morningglory | 2C,5G | 4C,9G |
| Cocklebur | 2C,7H | 5C,9G |
| Sicklepod | 2C,3G | 3C,6H |
| Nutsedge | &C | 3C,9G |
| Crabgrass | 3H | 0 |
| Barnyardgrass | 2C,8H | 9C |
| Wild Oats | 0 | 9C |
| Wheat | 0 | 9C |
| Corn | 1H | 3H |
| Soybean | 2C,9G | 3C,9G |
| Rice | 5G | 2C,9G |
| Sorghum | 2C,8H | 3C,9G |
| Sugar beet | 3G | 3C,9G |
| PREEMERGENCE | | |
| Morningglory | 9G | 9C |
| Cocklebur | 9H | 9H |
| Sicklepod | 7G | 3C,7G |
| Nutsedge | 5G | 0 |
| Crabgrass | 0 | 0 |
| Barnyardgrass | 2C,7G | 4G |
| Wild Oats | 2C | 2C,8G |
| Wheat | 3G | 3G |
| Corn | 2C,7H | 2C,9H |
| Soybean | 1C,1H | 3C,5H |
| Rice | 3C,6G | 7G |
| Sorghum | 3C,7H | 2C,9G |
| Sugar beet | 7G | 9G |

Table A (continued)

| | Cmpd. 16 | Cmpd. 17 | Cmpd 18 | Cmpd.19 |
|---|---|---|---|---|
| Rate g/ha | 400 | 400 | 400 | 400 |
| POST-EMERGENCE | | | | |
| Bush bean | 4C,9G,6Y | 4C,9G,6Y | 3G | 3C,4H |
| Cotton | 4C,9G | 5C,9G | 3C,7G | 0 |
| Morningglory | 3C,6G | 5C,9G | 5C,9G | 0 |
| Cocklebur | 3C,9G | 9C | 5C,9G | 1C,2H |
| Sicklepod | 3C,8H | 5C,9G | 5C,9G | 1C . |
| Nutsedge | 5G | 9C | 2C,9G | 0 |
| Crabgrass | 3C,8G | 9C | 9C | 1C,2H |
| Barnyardgrass | 3C,9H | 9C | 9C | 2C,6H |
| Wild Oats | 2C,9G | 3C,9G | 9C | 0 |
| Wheat | 2C,8G | 9G | 4U,9G | 0 |
| Corn | 5C,9G | 3U,9H | 5U,9G | 1C,5G |
| Soybean | 4C,9G | 9C | 5C,9G | 1C,1H |
| Rice | 6C,9G | 6C,9G | 5C,9G | 3C,9G |
| Sorghum | 3C,9H | 4C,9G | 5C,9G | 3C,7G |
| Sugar beet | 9C | 9C | 5C,9G | 2C,3H |
| PRE-EMERGENCE | | | | |
| Morningglory | 8H | 9G | 9C | 0 |
| Cocklebur | 9H | 9H | 9H | - |
| Sicklepod | 8G | 5C,9G | 9G | 3G |
| Nutsedge | 5G | 10E | 9G | 0 |
| Crabgrass | 2G | 5C,9G | 8G | 2G |
| Barnyardgrass | 3C,7G | 9H | 9H | 2H |
| Wild Oats | 2C,8G | 5C,9H | 2C,9G | 0 |
| Wheat | 2C,9G | 5C,9H | 3C,9G | 0 |
| Corn | 9H | 10H | 2C,9G | 1C |
| Soybean | 2C,2H | 9H | 9H | 0 |
| Rice | 10E | 10E | 10E | 2C |
| Sorghum | 6C,9H | 5C,9H | 2C,9H | 2C |
| Sugar beet | 5C,9G | 10E | 10E | 2H |

Table A (continued)

| | Cmpd. 20 | Cmpd. 21 |
|---|---|---|
| Rate g/ha | 400 | 400 |
| POST-EMERGENCE | | |
| Bush bean | 8D,9G,6Y | 2C,3H |
| Cotton | 4C,8G | 3C,7G |
| Morningglory | 3C,7G | 3C,8H |
| Cocklebur | 3C,9G | 2C,4G |
| Sicklepod | 3C,6H | 3C,6G |
| Nutsedge | 2G | 6G |
| Crabgrass | 4C,9H | 3C,9H |
| Barnyardgrass | 9C | 9C |
| Wild Oats | 4C,9G | 9C |
| Wheat | 9C | 9C |
| Corn | 9C | 9C |
| Soybean | 4C,9G | 3C,8H |
| Rice | 5C,9G | 5C,9G |
| Sorghum | 4C,9G | 5C,9G |
| Sugar beet | 9C | 9C |
| PRE-EMERGENCE | | |
| Morningglory | 0 | 3C,5H |
| Cocklebur | 2C | 2H |
| Sicklepod | 2C | 3C,8H |
| Nutsedge | 0 | 0 |
| Crabgrass | 2C,5G | 3G |
| Barnyardgrass | 5C,9H | 3C,9H |
| Wild Oats | 4C,9H | 2C,9G |
| Wheat | 2C,9H | 3C,9G |
| Corn | 3C,9H | 9H |
| Soybean | 1C,1H | 1C |
| Rice | 10E | 10E |
| Sorghum | 5C,9H | 5C,9H |
| Sugar beet | 3C,8G | 2C,8G |

Table A (continued)

| | Cmpd. 22 | Cmpd. 23 | Cmpd. 24 | Cmpd. 25 |
|---|---|---|---|---|
| Rate g/ha | 50 | 50 | 50 | 50 |
| POST-EMERGENCE | | | | |
| Bush bean | - | - | - | - |
| Cotton | 5C,9G | 9C | 5C,9H | 5C,9G |
| Morningglory | 5C,9G | 9C | 5C,9G | 6C,9G |
| Cocklebur | 10C | 9C | 5C,9H | 2C,9H |
| Sicklepod | 5C,9G | 9C | 6C,9G | 6C,9G |
| Nutsedge | 3C,8G | 9G | 3C,8G | 3G |
| Crabgrass | 3G | 2C,8G | 3C,8G | 5C,9G |
| Barnyardgrass | 4C,9H | 5C,9H | 4C,9H | 5C,9H |
| Wild Oats | 9C | 7G | 2C,9G | 8G |
| Wheat | 5G | 5G | 7G | 5G |
| Corn | 2C,4G | 1U,9G | 3C,9H | 3C,9G |
| Soybean | 3C,7G | 5C,9G | 5C,9G | 9C |
| Rice | 5C,9G | 6C,9G | 5C,9G | 6C,9G |
| Sorghum | 9G | 2U,9H | 4C,9H | 5C,9G |
| Sugar beet | 10C | 9C | 9C | 9C |
| PRE-EMERGENCE | | | | |
| Morningglory | 9G | 9G | 3C,9G | 9C |
| Cocklebur | 8H | 9H | 9H | 9H |
| Sicklepod | 8G | 9C | 5C,9G | 9G |
| Nutsedge | 2C,8G | 10E | 3C,9G | 4G |
| Crabgrass | 0 | 4G | 1C,3G | 4C,9G |
| Barnyardgrass | 2C,6G | 2C,9H | 3C,9H | 4C,9H |
| Wild Oats | 3C,8H | 2C,9G | 4C,9G | 4C,8G |
| Wheat | 3C,9H | 8G | 5C,9H | 5C,9G |
| Corn | 2C,9G | 3C,9H | 6C,9H | 10E |
| Soybean | 2C,7H | 9H | 9H | 4C,8H |
| Rice | 10E | 10E | 10E | 10E |
| Sorghum | 7C,9H | 4C,9H | 5C,9H | 6C,9H |
| Sugar beet | 5C,9G | 4C,9G | 6C,9G | 10E |
| Cotton | 2C,9G | 3C,9G | 2C,9G | 5C,9G |

Table A (continued)

| | Cmpd. 26 | Cmpd. 27 | Cmpd. 28 | Cmpd. 29 |
|---|---|---|---|---|
| Rate g/ha | 50 | 50 | 50 | 50 |
| POST-EMERGENCE | | | | |
| Bush bean | - | - | - | - |
| Cotton | 4C,9H | 10C | 9C | 9C |
| Morningglory | 5C,9H | 5C,9G | 5C,9G | 9C |
| Cocklebur | 5C,9H | 9C | 10C | 9C |
| Sicklepod | 4C,7H | 6C,9G | 9C | 5C,9G |
| Nutsedge | 2G | 4C,9G | 3C,8G | 10C |
| Crabgrass | 4C,7G | 9G | 3C,7G | 5C,9G |
| Barnyardgrass | 4C,9H | 6C,9G | 5C,9G | 9C |
| Wild Oats | 2G | 3C,9G | 2C,7G | 2C,9G |
| Wheat | 2G | 10C | 1G | 9C |
| Corn | 2U,9G | 3U,9G | 5C,9G | 5C,9G |
| Soybean | 4C,8H | 9C | 5C,9G | 6C,9G |
| Rice | 5C,9G | 6C,9G | 5C,9G | 6C,9G |
| Sorghum | 3C,9G | 5U,9C | 2U,9H | 2C,9G |
| Sugar beet | 5C,9G | 5C,9G | 5C,9H | 4C,9G |
| PRE-EMERGENCE | | | | |
| Morningglory | 9C | 9G | 9G | 9H |
| Cocklebur | 9H | 9H | 9H | 9H |
| Sicklepod | 6C,8H | 9G | 9G | 8G |
| Nutsedge | 2G | 10E | 6G | 10E |
| Crabgrass | 1C | 3C,7G | 1C | 5G |
| Barnyardgrass | 3C,9H | 3C,9H | 4C,9H | 4C,9H |
| Wild Oats | 2C,8G | 2C,8G | 3C,7G | 3C,9G |
| Wheat | 3G | 7C,9H | 5G | 10E |
| Corn | 9G | 9H | 9H | 9H |
| Soybean | 4C,6H | 9H | 9H | 9H |
| Rice | 4C,9H | 10E | 9H | 10E |
| Sorghum | 5C,9H | 4C,9H | 5C,9H | 5C,9H |
| Sugar beet | 9C | 9G | 10E | 5C,9G |
| Cotton | 3C,8H | 3C,9G | 3C,9G | 9G,3C |

Table A (continued)

| | Cmpd.30 | Cmpd. 31 | Cmpd. 32 |
|---|---|---|---|
| Rate g/ha | 50 | 50 | 50 |
| POST-EMERGENCE | | | |
| Bush bean | - | - | - |
| Cotton | 2C,9G | 3C,9H | 9C |
| Morningglory | 4C,9G | 4C,9G | 10C |
| Cocklebur | 5C,9H | 4C,9H | 10C |
| Sicklepod | 2C,3H | 1C | 4C,8G |
| Nutsedge | 4G | 2G | 3C,9G |
| Crabgrass | 4G | 0 | 2C,5G |
| Barnyardgrass | 4C,9H | 2C,7G | 3C,9H |
| Wild Oats | 3C,8G | 0 | 2C,4G |
| Wheat | 9H | 0 | 2C,6G |
| Corn | 2C,9H | 3C,9H | 2C,9G |
| Soybean | 3C,9G | 3C,9H | 3C,9G |
| Rice | 5C,9G | 5C,9H | 6C,9G |
| Sorghum | 9G | 9G | 9C |
| Sugar beet | 1C,3G | 9C | 5C,9G |
| PRE-EMERGENCE | | | |
| Morningglory | 9C | 8H | 9C |
| Cocklebur | 8H | 8H | 9C |
| Sicklepod | 7G | 5G,2C | 4C,9G |
| Nutsedge | 5G | 0 | 8G |
| Crabgrass | 0 | 0 | 2C,5G |
| Barnyardgrass | 3C,8H | 5G | 3C,9H |
| Wild Oats | 3C,8G | 2C | 9G |
| Wheat | 8G | 0 | 2C,9G |
| Corn | 2C,8G | 9G | 3C,9G |
| Soybean | 7H | 3C,4H | 9H |
| Rice | 10E | 9H | 10E |
| Sorghum | 3C,9H | 2C,9G | 5C,9H |
| Sugar beet | 8G | 5C,9G | 10E |
| Cotton | 8G | 8G | 3C,9G |

**Claims for the Contracting States: BE CH DE FR GB IT LI LU NL SE**

1. A compound of the formula:

$DSO_2NHCONA$
R

I

wherein

D is 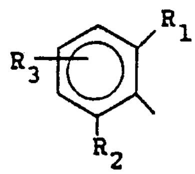 or 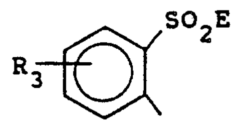 ;

D-1 D-4

R is H or $CH_3$;
$R_1$ is

$S(O)_nR_8$, $CF_3$, $NR_9R_{10}$, $CO_2R_{11}$,
$SO_2NR_{12}R_{13}$, 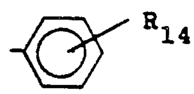 , $CHCO_2R_{11}$,
$R_{15}$
$OSO_2R_{16}$, $CH_2OR_{17}$ or Q;

$R_2$ is

$S(O)_mR_8$, $CF_3$, $NR_9R_{10}$, $CO_2R_{11}$,
$SO_2NR_{12}R_{13}$, 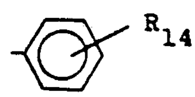 , $CHCO_2R_{11}$,
$R_{15}$
$OSO_2R_{16}$, $CH_2OR_{17}$ or Q;

$R_3$ is H, Cl, F, Br, $CH_3$, $OCH_3$ or $CF_3$;
E is

$NR_{22}R_{23}$, 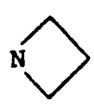, 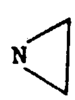 or 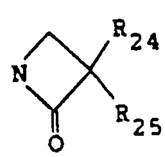 ;

$R_8$ is $C_1$—$C_3$ alkyl;
$R_9$ and $R_{10}$ are independently $C_1$—$C_2$ alkyl;
$R_{11}$ is $C_1$—$C_3$ alkyl, $CH_2CH{=}CH_2$, $CH_2CH_2Cl$ or $CH_2CH_2OCH_3$;
$R_{12}$ is $CH_3$ or $OCH_3$;
$R_{13}$ is $C_1$—$C_3$ alkyl;
$R_{14}$ is H, Cl, Br, F, $CH_3$ or $OCH_3$;
$R_{15}$ is H or $CH_3$;
$R_{16}$ is $C_1$—$C_3$ alkyl or $CF_3$;
$R_{17}$ is $C_1$—$C_3$ alkyl or $CF_2H$;
n is 0, 1 or 2;
m is 0 or 1;

Q is

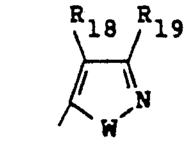 ,

Q-1

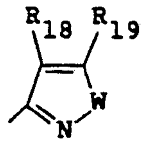 ,

Q-2

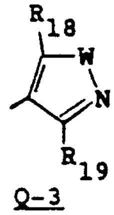 ,

Q-3

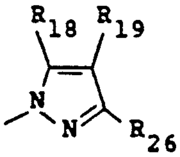 ,

Q-4

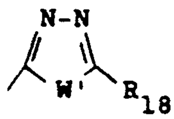 ,

Q-5

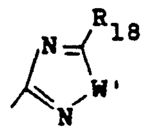 ,

Q-6

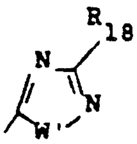 ,

Q-7

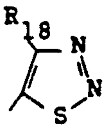 ,

Q-8

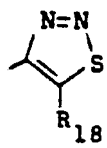 ,

Q-9

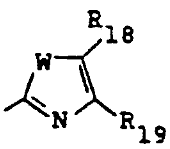 ,

Q-10

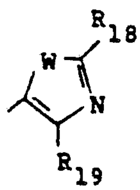 ,

Q-11

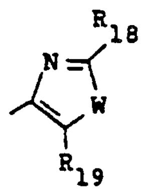 ,

Q-12

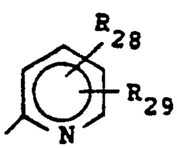 ,

Q-13

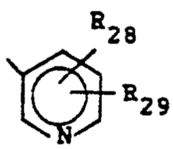 ,

Q-14

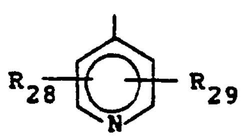 ,

Q-15

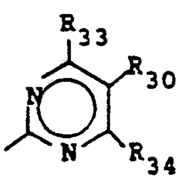 ,

Q-16

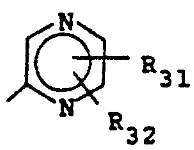 ,

Q-17

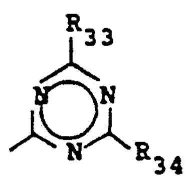 ,

Q-18

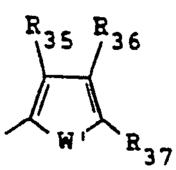

Q-19

or

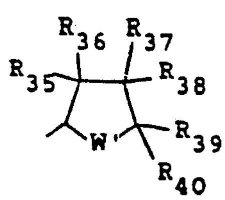 ;

Q-20

W is O, S or $NR_{27}$;

W' is O or S;

$R_{18}$, $R_{19}$, $R_{24}$, $R_{25}$, $R_{26}$, $R_{27}$, $R_{28}$, $R_{29}$, $R_{30}$, $R_{31}$, $R_{32}$, $R_{35}$, $R_{36}$, $R_{37}$, $R_{38}$, $R_{39}$ and $R_{40}$ are independently H or $CH_3$;

$R_{33}$ and $R_{34}$ are independently H, $CH_3$ or $OCH_3$;

$R_{22}$ is $C(O)R_{41}$, $C(O)NR_{42}R_{43}$, $CO_2R_{44}$, $C(O)NHR_{45}$ or $CF_2H$;

$R_{23}$ is H or $C_1$—$C_3$ alkyl;

$R_{41}$ is $CF_3$ or phenyl optionally substituted with Cl, $CH_3$, $CF_3$, $NO_2$ or $OCH_3$;

$R_{42}$ is H, $C_1$—$C_4$ alkyl or phenyl optionally substituted with Cl, $CH_3$, $CF_3$, $NO_2$ or $OCH_3$;

$R_{43}$ is H or $C_1$—$C_4$ alkyl;

$R_{42}$ and $R_{43}$ may be taken together to be $—(CH_2)_4—$, $—(CH_2)_5—$ or $—CH_2CH_2OCH_2CH_2—$;

$R_{44}$ is $C_1$—$C_4$ alkyl;

$R_{45}$ is A—1;

A is

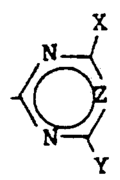 , 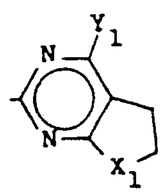 , 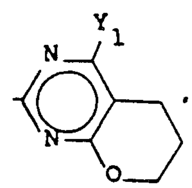 ,

A-1 A-2 A-3

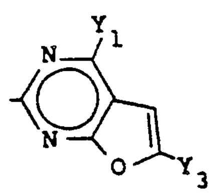 , 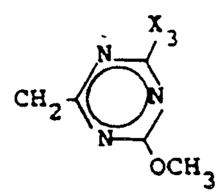 or 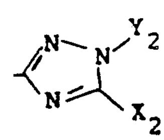 ;

A-4 A-5 A-6

X is $CH_3$, $OCH_3$, $OCF_2H$ or Cl;

Y is $CH_3$, $C_2H_5$, $OCH_3$, $OC_2H_5$, $CH_2OCH_3$, $OCH_2CF_3$, $OCF_2H$, $NHCH_3$, $N(CH_3)_2$ or $SCH_3$;

$X_1$ is $CH_2$ or O;

$Y_1$ is $CH_3$ or $OCH_3$;

$X_2$ is $CH_3$, $OCH_3$ or $SCH_3$;

$Y_2$ is $CH_3$, $C_2H_5$ or $CH_2CF_3$;

$X_3$ is $CH_3$ or $OCH_3$; and

$Y_3$ is H or $CH_3$;

Z is CH or N;

and their agriculturally suitable salts; provided that

a) when either one of $R_1$ or $R_2$ is $CF_3$, then the other is also $CF_3$, X is other than Cl when Y is $NHCH_3$, and X and Y are other than $OCF_2H$;

b) $R_1$ and $R_2$ are not simultaneously $CO_2R_{11}$;

c) when either one of $R_1$ or $R_2$ is $SO_2NR_{12}R_{13}$, then the other must not be $S(O)_nR_8$, $S(O)_mR_8$, $CH_2OR_{17}$ or $NR_9R_{10}$;

d) when $R_{12}$ is $OCH_3$, then $R_{13}$ is $CH_3$;

e) when either one of $R_1$ or $R_2$ is $CO_2R_{11}$, then the other must not be $S(O)_nR_8$, $S(O)_mR_8$ or $NR_9R_{10}$;

f) the total number of carbon atoms in $R_{35}$ to $R_{40}$ combined, is equal to or less than four;

g) when X is Cl, then Z is CH and Y is $NHCH_3$, $N(CH_3)_2$, $OCH_3$ or $OC_2H_5$;

h) when $R_1$ is $CO_2R_{11}$ and $R_2$ is $OSO_2R_{16}$, then A is not A—1;

j) when $R_3$ is H, A is A—1 and either one of $R_1$ or $R_2$ is Q and Q is not Q—4 or Q—20; then the other of $R_1$ or $R_2$ must not be $S(O)_nR_8$, $S(O)_mR_8$, $CF_3$ or $CO_2R_{11}$;

k) when A is A—6, then $R_1$ is

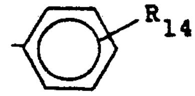 , $\underset{R_{15}}{CHCO_2R_{11}}$, $CH_2OR_{17}$ or Q;

l) when $R_{22}$ is C(O)$NHR_{45}$, then A must be A—1 and the values of X, Y and Z must be identical for both A and $R_{45}$;

m) when D is D—4, then R is H; and

n) when $R_1$ and $R_2$ are $CF_3$, then A is other than A—5.

2. The compounds of Claim 1 where A is A—1; R is H; $R_1$ and $R_2$ are not simultaneously Q; D is D—1 or D—4; and when D is other than D—1 then $R_3$ is H.

3. The compounds of Claim 2 where D is D—1 and Y is $CH_3$, $CH_2OCH_3$, $OCH_3$, $OCH_2CF_3$ or $OCF_2H$.

4. The compounds of Claim 3 where $R_3$ is in either the 3- or 5-position.

5. The compounds of Claim 4 where $R_1$ and $R_2$ are not simultaneously $SO_2NR_{12}R_{13}$ or $NR_9R_{10}$.

6. The compounds of Claim 5 where $R_3$ is H and at least one of $R_1$ or $R_2$ is

$S(O)_nR_8$, $S(O)_mR_8$, $CF_3$, $NR_9R_{10}$, $SO_2NR_{12}R_{13}$, 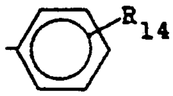, $OSO_2R_{16}$ or $CH_2OR_{17}$.

7. The compounds of Claim 6 where $R_8$ is $CH_3$ or $C_2H_5$; $R_9$ and $R_{10}$ are both $CH_3$; $R_{12}$ is $CH_3$; $R_{14}$ is H; $R_{16}$ is $CH_3$ or $C_2H_5$; and $R_{17}$ is $CH_3$.

8. The compounds of Claim 7 where X is $CH_3$, $OCH_3$ or Cl and Y is $CH_3$, $OCH_3$ or $CH_2OCH_3$.

9. The compounds of Claim 2 where D is D—4 and Y is $CH_3$, $C_2H_5$, $OCH_3$, $OC_2H_5$ or $CH_2OCH_3$.

10. The compounds of Claim 9 where E is

$NR_{22}R_{23}$ or 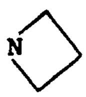 ;

and $R_{23}$ is H or $CH_3$.

11. The compounds of Claim 10 where E is

$NR_{22}R_{23}$ or 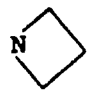 ;

and X is $CH_3$, $OCH_3$ or Cl.

12. The compound of Claim 1 which is 2-(azetidin-1-ylsulfonyl)-N-[(4-methoxy-6-methyl-1,3,5-triazin-2-yl)aminocarbonyl]benzenesulfonamide, or an agriculturally suitable salt thereof.

13. A compound of claim 1 selected from N-[(4,6-dimethoxypyrimidin-2-yl)aminocarbonyl]-3-methylsulfonyl-1,1'-biphenyl-2-sulfonamide; N,N'-bis-[(4,6-dimethoxypyrimidin-2-yl)aminocarbonyl]-1,2-benzenedisulfonamide; or an agriculturally suitable salt of any of these.

14. A compound of claim 1 which is N-[(4,6-dimethoxy-1,3,5-triazin-2-yl)aminocarbonyl]-2,6-bis(methylthio)benzenesulfonamide; or an agriculturally suitable salt thereof.

15. A compound of claim 1 selected from 2-(azetidin-1-ylsulfonyl)-N-[(4,6-dimethoxy-1,3,5-triazin-2-yl)aminocarbonyl]benzenesulfonamide; or 2-(azetidin-1-ylsulfonyl)-N-[(4-methoxy-6-methylpyrimidin-2-yl)aminocarbonyl]benzenesulfonamide; or an agriculturally suitable salt of either of these.

16. A compound of claims 1 wherein D is D—4.

17. An agriculturally suitable composition for controlling the growth of undesired vegetation comprising an effective amount of a compound of any of claims 1 to 16 and at least one of the following: surfactant, solid or liquid diluent.

18. A method for controlling the growth of undesired vegetation which comprises applying to the locus to be protected an effective amount of a compound of any of claims 1 to 16.

19. A method for regulating the growth of plants which comprises applying to the locus of such plants an effective but substantially non-phytotoxic amount of a plant growth regulant selected from compounds of any of claims 1 to 16.

20. A process for the preparation of a compound of claim 1, which comprises:

(A) reacting a sulfonamide of general formula (II)

$$DSO_2NH_2 \quad (II)$$

or a mixture of a sulfonyl chloride $DSO_2Cl$ with isocyanate anion, with a methyl carbamate of general formula (III)

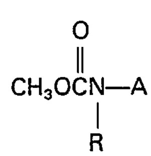

wherein:

A is as defined in claim 1,

$R_1$, $R_2$, $R_4$ and $R_5$ are not $CO_2R_{11}$; and

R is H; or

(B) reacting a sulfonyl carbamate of general formula (IV)

$$DSO_2NHCO_2C_6H_5 \qquad (IV)$$

with an appropriate amine of general formula (V)

$$\underset{\displaystyle R}{\underset{|}{HN}}\!-\!A \qquad (V)$$

wherein:

A is as defined in claim 1;

$R_1$ and $R_2$ are not $CO_2R_{11}$ or $OSO_2R_{16}$; and

R is H or $CH_3$; or

(C) reacting a sulfonamide of general formula (II) as defined above or said mixture of sulfonyl chloride and isocyanate anion, with a heterocyclic phenylcarbamate of general formula (VI)

$$C_6H_5O\overset{\displaystyle O}{\overset{\|}{C}}NHA \qquad (VI)$$

wherein A is as defined in claim 1; or

(D) reacting a benzenesulfonyl isocyanate of general formula

$$DSO_2NCO \qquad (VIII)$$

with an appropriate aminoheterocycle of general formula

$$\underset{\displaystyle R}{\underset{|}{HN}}\!-\!A \qquad (V)$$

wherein D, R and A are as defined in claim 1.

**Claims for the Contracting State: AT**

1. A process for the preparation of a compound of the formula:

$$DSO_2NHCO\underset{\displaystyle R}{\underset{|}{N}}A$$

I

wherein

D is 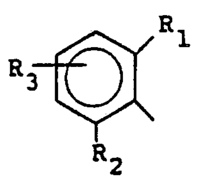 or 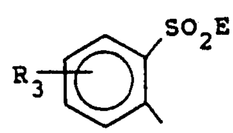 ;

D-1 D-4

R is H or $CH_3$;

$R_1$ is

$$S(O)_nR_8,\ CF_3,\ NR_9R_{10},\ CO_2R_{11},$$

$SO_2NR_{12}R_{13}$, 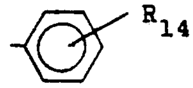 , $CHCO_2R_{11}$,
$R_{15}$

$OSO_2R_{16}$, $CH_2OR_{17}$ or Q;

$R_2$ is

$S(O)_mR_8$, $CF_3$, $NR_9R_{10}$, $CO_2R_{11}$,

$SO_2NR_{12}R_{13}$, 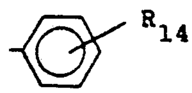 , $CHCO_2R_{11}$,
$R_{15}$

$OSO_2R_{16}$, $CH_2OR_{17}$ or Q;

$R_3$ is H, Cl, F, Br, $CH_3$, $OCH_3$ or $CF_3$;
E is

$NR_{22}R_{23}$, 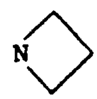 , 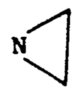 or 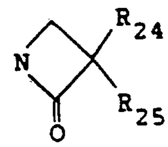 ;

$R_8$ is $C_1$—$C_3$ alkyl;
$R_9$ and $R_{10}$ are independently $C_1$—$C_2$ alkyl;
$R_{11}$ is $C_1$—$C_3$ alkyl, $CH_2CH{=}CH_2$, $CH_2CH_2Cl$ or $CH_2CH_2OCH_3$;
$R_{12}$ is $CH_3$ or $OCH_3$;
$R_{13}$ is $C_1$—$C_3$ alkyl;
$R_{14}$ is H, Cl, Br, F, $CH_3$ or $OCH_3$;
$R_{15}$ is H or $CH_3$;
$R_{16}$ is $C_1$—$C_3$ alkyl or $CF_3$;
$R_{17}$ is $C_1$—$C_3$ alkyl or $CF_2H$;
n is 0, 1 or 2;
m is 0 or 1;
Q is

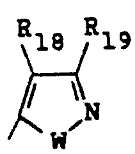 , 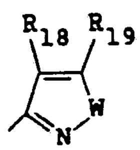 , 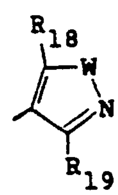 ,

Q-1 Q-2 Q-3

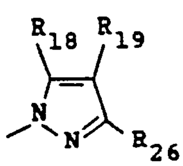 , 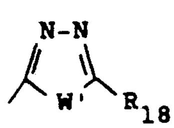 , 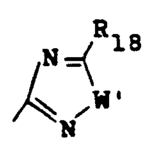 ,

Q-4 Q-5 Q-6

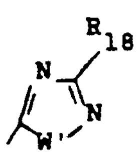 , 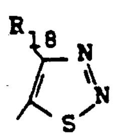 , 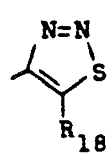 ,

Q-7 Q-8 Q-9

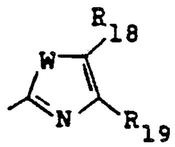

Q-10,

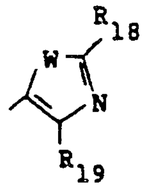

Q-11,

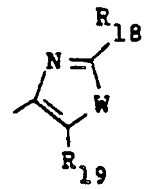

Q-12,

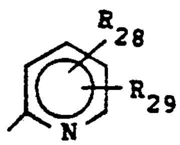

Q-13,

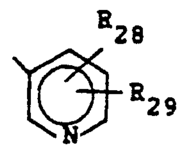

Q-14,

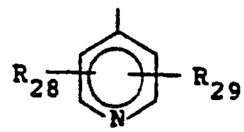

Q-15,

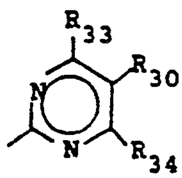

Q-16,

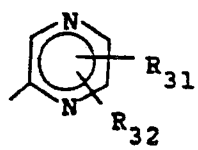

Q-17,

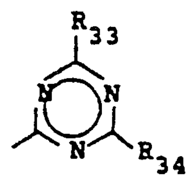

Q-18,

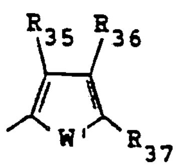

Q-19

or

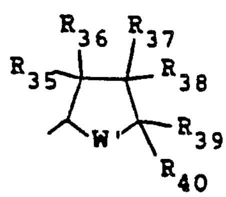

Q-20;

W is O, S or $NR_{27}$;
W' is O or S;
$R_{18}$, $R_{19}$, $R_{24}$, $R_{25}$, $R_{26}$, $R_{27}$, $R_{28}$, $R_{29}$, $R_{30}$, $R_{31}$, $R_{32}$, $R_{35}$, $R_{36}$, $R_{37}$, $R_{38}$, $R_{39}$ and $R_{40}$ are independently H or $CH_3$;
$R_{33}$ and $R_{34}$ are independently H, $CH_3$ or $OCH_3$;
$R_{22}$ is $C(O)R_{41}$, $C(O)NR_{42}R_{43}$, $CO_2R_{44}$, $C(O)NHR_{45}$ or $CF_2H$;
$R_{23}$ is H or $C_1$—$C_3$ alkyl;
$R_{41}$ is $CF_3$ or phenyl optionally substituted with Cl, $CH_3$, $CF_3$, $NO_2$ or $OCH_3$;
$R_{42}$ is H, $C_1$—$C_4$ alkyl or phenyl optionally substituted with Cl, $CH_3$, $CF_3$, $NO_2$ or $OCH_3$;
$R_{43}$ is H or $C_1$—$C_4$ alkyl;
$R_{42}$ and $R_{43}$ may be taken together to be $-(CH_2)_4-$, $-(CH_2)_5-$ or $-CH_2CH_2OCH_2CH_2-$;
$R_{44}$ is $C_1$—$C_4$ alkyl;
$R_{45}$ is A—1;
A is

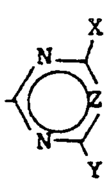

A-1,

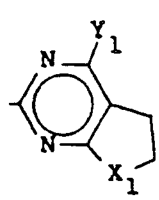

A-2,

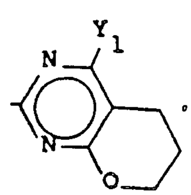

A-3.

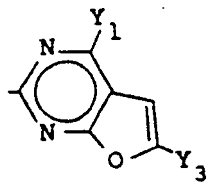 , 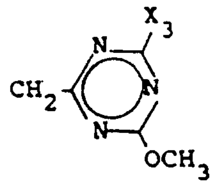 or 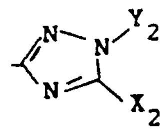 ;

A-4 A-5 A-6

X is $CH_3$, $OCH_3$, $OCF_2H$ or Cl;
Y is $CH_3$, $C_2H_5$, $OCH_3$, $OC_2H_5$, $CH_2OCH_3$, $OCH_2CF_3$, $OCF_2H$, $NHCH_3$, $N(CH_3)_2$ or $SCH_3$;
$X_1$ is $CH_2$ or O;
$Y_1$ is $CH_3$ or $OCH_3$;
$X_2$ is $CH_3$, $OCH_3$ or $SCH_3$;
$Y_2$ is $CH_3$, $C_2H_5$ or $CH_2CF_3$;
$X_3$ is $CH_3$ or $OCH_3$;
$Y_3$ is H or $CH_3$; and
Z is CH or N;

or an agriculturally suitable salt thereof; provided that

a) when either one of $R_1$ or $R_2$ is $CF_3$, then the other is also $CF_3$, X is other than Cl when Y is $NHCH_3$, and X and Y are other than $OCF_2H$;

b) $R_1$ and $R_2$ are not simultaneously $CO_2R_{11}$;

c) when either one of $R_1$ or $R_2$ is $SO_2NR_{12}R_{13}$, then the other must not be $S(O)_nR_8$, $S(O)_mR_8$, $CH_2OR_{17}$ or $NR_9R_{10}$;

d) when $R_{12}$ is $OCH_3$, then $R_{13}$ is $CH_3$;

e) when either one of $R_1$ or $R_2$ is $CO_2R_{11}$, then the other must not be $S(O)_nR_8$, $S(O)_mR_8$ or $NR_9R_{10}$;

f) the total number of carbon atoms in $R_{35}$ to $R_{40}$ combined, is equal to or less than four;

g) when X is Cl, then Z is CH and Y is $NHCH_3$, $N(CH_3)_2$, $OCH_3$ or $OC_2H_5$;

h) when $R_1$ is $CO_2R_{11}$ and $R_2$ is $OSO_2R_{16}$, then A is not A—1;

j) when $R_3$ is H, A is A—1 and either one of $R_1$ or $R_2$ is Q and Q is not Q—4 or Q—20; then the other of $R_1$ or $R_2$ must not be $S(O)_nR_8$, $S(O)_mR_8$, $CF_3$ or $CO_2R_{11}$;

k) when A is A—6, then $R_1$ is

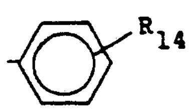 , 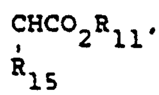 , $CH_2OR_{17}$ or Q;

l) when $R_{22}$ is $C(O)NHR_{45}$, then A must be A—1 and the values of X, Y and Z must be identical for both A and $R_{45}$;

m) when D is D—4, then R is H; and

n) when $R_1$ and $R_2$ are $CF_3$, then A is other than A—5,

which comprises:

(A) reacting a sulfonamide of general formula (II)

$$DSO_2NH_2 \qquad (II)$$

or a mixture of a sulfonyl chloride $DSO_2Cl$ with isocyanate anion, with a methyl carbamate of general formula (III)

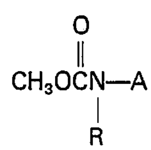 (III)

wherein:

A is as defined above;

$R_1$, $R_2$, $R_4$ and $R_5$ are not $CO_2R_{11}$; and

R is H; or

(B) reacting a sulfonyl carbamate of general formula (IV)

$$DSO_2NHCO_2C_6H_5 \qquad (IV)$$

with an appropriate amine of general formula (V)

$$HN{-}A \atop {|\atop R}$$ (V)

wherein:

A is as defined above;

$R_1$ and $R_2$ are not $CO_2R_{11}$ or $OSO_2R_{16}$; and

R is H or $CH_3$; or

(C) reacting a sulfonamide of general formula (II) as defined above or said mixture of sulfonyl chloride and isocyanate anion, with a heterocyclic phenylcarbamate of general formula (VI)

$$C_6H_5O\overset{O}{\overset{\|}{C}}NHA$$ (V)

wherein A is as defined above; or

(D) reacting a benzenesulfonyl isocyanate of general formula

$$DSO_2NCO$$ (VIII)

with an appropriate aminoheterocycle of general formula

$$HN{-}A \atop {|\atop R}$$ (V)

wherein D, R and A are as defined for formula (I).

2. The process of claim 1 where A is A—1; R is H; $R_1$ and $R_2$ are not simultaneously Q; D is D—1 or D—4; and when D is other than D—1 then $R_3$ is H.

3. The process of claim 2 where D is D—1 and Y is $CH_3$, $CH_2OCH_3$, $OCH_3$, $OCH_2CF_3$ or $OCF_2H$.

4. The process of claim 3 where $R_1$ and $R_2$ are not simultaneously $SO_2NR_{12}R_{13}$ or $NR_9R_{10}$; $R_3$ is H and at least one of $R_1$ or $R_2$ is

$S(O)_nR_8$, $S(O)_mR_8$, $CF_3$, $NR_9R_{10}$, $SO_2NR_{12}R_{13}$, 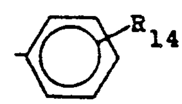, $OSO_2R_{16}$ or $CH_2OR_{17}$;

$R_8$ is $CH_3$ or $C_2H_5$; $R_9$ and $R_{10}$ are both $CH_3$; $R_{12}$ is $CH_3$;

$R_{14}$ is H; $R_{16}$ is $CH_3$ or $C_2H_5$;

$R_{17}$ is $CH_3$;

X is $CH_3$, $OCH_3$ or Cl and Y is $CH_3$, $OCH_3$ or $CH_2OCH_3$.

5. The process of claim 2 where D is D—4; Y is $CH_3$, $C_2H_5$, $OCH_3$, $OC_2H_5$ or $CH_2OCH_3$; E is

$NR_{22}R_{23}$ or 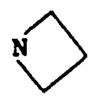 ;

$R_{22}$ and $R_{23}$ are both H; and X is $CH_3$, $OCH_3$ or Cl.

6. The process of claim 1 wherein the product is a compound selected from

2-(azetidin-1-ylsulfonyl)-N-[(4-methoxy-6-methyl-1,3,5-triazin-2-yl)aminocarbonyl]benzenesulfonamide,

N-[(4,6-dimethoxypyrimidin-2-yl)aminocarbonyl]-3-methylsulfonyl-1,1′-biphenyl-2-sulfonamide;

N,N′-bis-[(4,6-dimethoxypyrimidin-2-yl)aminocarbonyl]-1,2-benzenedisulfonamide;

N-[(4,6-dimethoxy-1,3,5-triazin-2-yl)aminocarbonyl]-2,6-bis(methylthio)benzenesulfonamide;

2-(azetidin-1-ylsulfonyl)-N-[(4,6-dimethoxy-1,3,5-triazin-2-yl)aminocarbonyl]benzenesulfonamide; or

2-(azetidin-1-ylsulfonyl)-N-[(4-methoxy-6-methylpyrimidin-2-yl)aminocarbonyl]benzenesulfonamide;

or an agriculturally suitable salt of any of these.

7. An agriculturally suitable composition for controlling the growth of undesired vegetation comprising an effective amount of a compound of formula (I) as defined in any of claims 1 to 6 and at least one of the following: surfactant, solid or liquid diluent.

8. A method for controlling the growth of undesired vegetation which comprises applying to the locus to be protected an effective amount of a compound of formula (I) as defined in any of claims 1 to 6.

9. A method for regulating the growth of plants which comprises applying to the locus of such plants an effective but substantially non-phytotoxic amount of a plant growth regulant selected from compounds of formula (I) as defined in any of claims 1 to 6.

**Patentansprüche für die Vertragsstaaten: BE CH DE FR GB IT LI LU NL SE**

1. Verbindung der Formel

$$DSO_2NHCONA$$
R

I

worin D

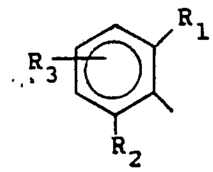

oder

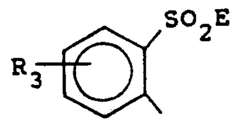

;

D-1 D-4

R H oder $CH_3$ ist;

$R_1$

$S(O)_nR_8$, $CF_3$, $NR_9R_{10}$, $CO_2R_{11}$, $SO_2NR_{12}R_{13}$, 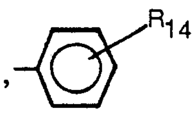

$CHCO_2R_{11}$ (mit $R_{15}$), $OSO_2R_{16}$, $CH_2OR_{17}$ oder Q ist;

$R_2$

$S(O)_mR_8$, $CF_3$, $NR_9R_{10}$, $CO_2R_{11}$, $SO_2NR_{12}R_{13}$, 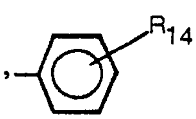

$CHCO_2R_{11}$ (mit $R_{15}$), $OSO_2R_{16}$, $CH_2OR_{17}$ oder Q ist;

$R_3$ H, Cl, F, Br, $CH_3$, $OCH_3$ oder $CF_3$ ist;

E

$NR_{22}R_{23}$, 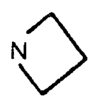 , 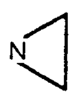 oder 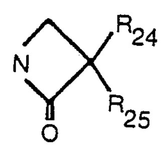 ist;

$R_8$ $C_1$-$C_3$-Alkyl ist;

$R_9$ und $R_{10}$ unabhängig $C_1$—$C_2$-Alkyl sind;

$R_{11}$ $C_1$—$C_3$-Alkyl, $CH_2CH=CH_2$, $CH_2CH_2Cl$ oder $CH_2CH_2OCH_3$ ist;

$R_{12}$ $CH_3$ oder $OCH_3$ ist;

$R_{13}$ $C_1$—$C_3$-Alkyl ist;

$R_{14}$ H, Cl, Br, F, $CH_3$ oder $OCH_3$ ist;

$R_{15}$ H oder $CH_3$ ist;

$R_{16}$ $C_1$—$C_3$-Alkyl oder $CF_3$ ist;

$R_{17}$ $C_1$—$C_{-3}$-Alkyl oder $CF_2H$ ist;

n 0, 1 oder 2 ist;

m 0 oder 1 ist;

Q

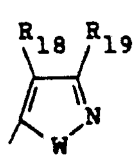 , 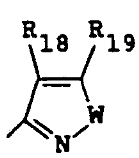 , 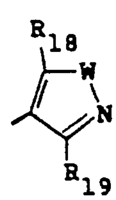 ,

Q-1 Q-2 Q-3

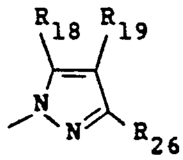

Q-4 ,

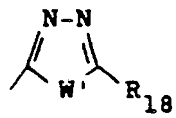

Q-5 ,

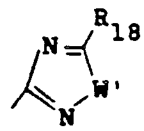

Q-6 ,

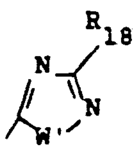

Q-7 ,

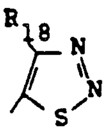

Q-8 ,

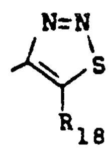

Q-9 ,

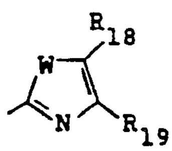

Q-10 ,

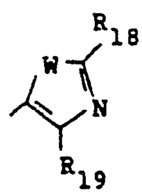

Q-11 ,

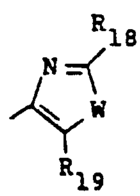

Q-12 ,

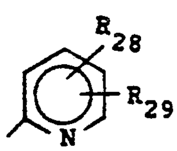

Q-13 ,

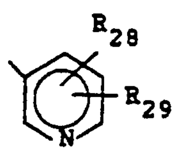

Q-14 ,

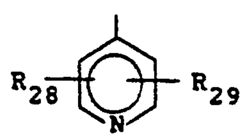

Q-15 ,

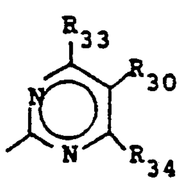

Q-16 ,

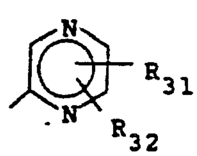

Q-17 ,

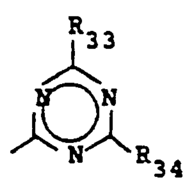

Q-18 ,

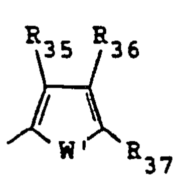

Q-19

oder

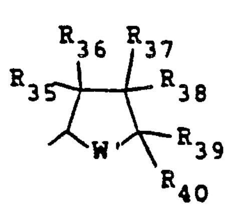

Q-20

ist;

W O, S oder $NR_{27}$ ist;

W' O oder S ist;

$R_{18}$, $R_{19}$, $R_{24}$, $R_{25}$, $R_{26}$, $R_{27}$, $R_{28}$, $R_{29}$, $R_{30}$, $R_{31}$, $R_{32}$, $R_{35}$, $R_{36}$, $R_{37}$, $R_{38}$, $R_{39}$ und $R_{40}$ unabhängig H oder $CH_3$ sind;

$R_{33}$ und $R_{34}$ unabhängig H, $CH_3$ oder $OCH_3$ sind;

$R_{22}$ $C(O)R_{41}$, $C(O)NR_{42}R_{43}$, $CO_2R_{44}$, $C(O)NHR_{45}$ oder $CF_2H$ ist;

$R_{23}$ H oder $C_1$—$C_3$-Alkyl ist;

$R_{41}$ $CF_3$ oder gegebenenfalls durch Cl, $CH_3$, $CF_3$, $NO_2$ oder $OCH_3$ substituiertes Phenyl ist;

$R_{42}$ H, $C_1$—$C_4$-Alkyl oder gegebenenfalls durch Cl, $CH_3$, $CF_3$, $NO_2$ oder $OCH_3$ substituiertes Phenyl ist;

$R_{43}$ H oder $C_1$—$C_4$-Alkyl ist;

$R_{42}$ und $R_{43}$ zusammengenommen werden können zu $-(CH_2)_4-$, $-(CH_2)_5-$ oder $-CH_2CH_2OCH_2CH_2-$;

$R_{44}$ $C_1$—$C_4$-Alkyl ist;

$R_{45}$ A—1 ist;

A

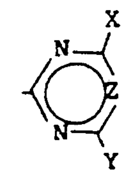 , 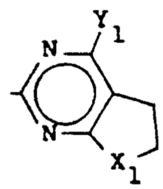 , 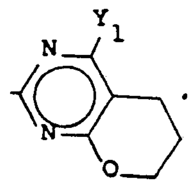 ,

A-1 A-2 A-3

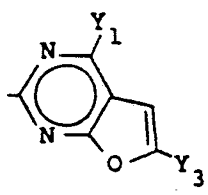 , 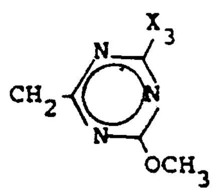 oder 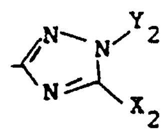 ist:

A-4 A-5 A-6

X $CH_3$, $OCH_3$, $OCF_2H$ oder Cl ist;

Y $CH_3$, $C_2H_5$, $OCH_3$, $OC_2H_5$, $CH_2OCH_3$, $OCH_2CF_3$, $OCF_2H$, $NHCH_3$, $N(CH_3)_2$ oder $SCH_3$ ist;

$X_1$ $CH_2$ oder O ist;

$Y_1$ $CH_3$ oder $OCH_3$ ist;

$X_2$ $CH_3$, $OCH_3$ oder $SCH_3$ ist;

$Y_2$ $CH_3$, $C_2H_5$ oder $CH_2CF_3$ ist;

$X_3$ $CH_3$ oder $OCH_3$ ist; und

$Y_3$ H oder $CH_3$ ist;

Z CH oder N ist;

und ihre landwirtschaftlich geeigneten Salze; mit der Massgabe, dass

a) wenn eines aus $R_1$ oder $R_2$ $CF_3$ ist, dann das andere ebenfalls $CF_3$ ist, X von Cl verschieden ist, wenn Y $NHCH_3$ ist, und X und Y von $OCF_2H$ verschieden sind;

b) $R_1$ und $R_2$ nicht gleichzeitig $CO_2R_{11}$ sind;

c) wenn eines aus $R_1$ oder $R_2$ $SO_2NR_{12}R_{13}$ ist, dann das andere nicht $S(O)_nR_8$, $S(O)_mR_8$, $CH_2OR_{17}$ oder $NR_9R_{10}$ sein darf;

d) wenn $R_{12}$ $OCH_3$ ist, dann $R_{13}$ $CH_3$ ist;

e) wenn eines aus $R_1$ oder $R_2$ $CO_2R_{11}$ ist, dann das andere nicht $S(O)_nR_8$, $S(O)_mR_8$ oder $NR_9R_{10}$ sein darf;

f) die Gesamtzahl der Kohlenstoffatome in $R_{35}$ bis $R_{40}$ zusammengenommen gleich oder weniger als vier ist;

g) wenn X Cl ist, dann Z CH ist und Y $NHCH_3$, $N(CH_3)_2$, $OCH_3$ oder $OC_2H_5$ ist;

h) wenn $R_1$ $CO_2R_{11}$ ist und $R_2$ $OSO_2R_{16}$ ist, dann A nicht A—1 ist;

j) wenn $R_3$ ist, A A—1 ist und eines aus $R_1$ oder $R_2$ Q ist und Q nicht Q—4 oder Q—20 ist, dann das andere aus $R_1$ oder $R_2$ nicht $S(O)_nR_8$, $S(O)_mR_8$ $CF_3$ oder $CO_2R_{11}$ sein darf;

k) wenn A A—6 ist, dann $R_1$

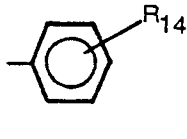 , $\underset{R_{15}}{\underset{|}{C}}HCO_2R_{11}$, $CH_2OR_{17}$ oder Q ist;

l) wenn $R_{22}$ C(O)NH$R_{45}$ ist, dann A A—1 sein muss oder die Werte von X, Y und Z für sowohl A als auch $R_{45}$ identisch sein müssen;

m) wenn D D—4 ist, dann R H ist; und

n) wenn $R_1$ und $R_2$ $CF_3$ sind, dann A von A—5 verschieden ist.

2. Verbindungen nach Anspruch 1, worin A A—1 ist; R H ist; $R_1$ und $R_2$ nicht gleichzeitig Q sind; D D—1 oder D—4 ist; und wenn D von D—1 verschieden ist, dann $R_3$ H ist.

3. Verbindungen nach Anspruch 2, worin D D—1 ist und Y $CH_3$, $CH_2OCH_3$, $OCH_3$, $OCH_2CF_3$ oder $OCF_2H$ ist.

4. Verbindungen nach Anspruch 3, worin $R_3$ entweder in der 3- oder 5-Stellung ist.

5. Verbindungen nach Anspruch 4, worin $R_1$ und $R_2$ nicht gleichzeitig $SO_2NR_{12}R_{13}$ oder $NR_9R_{10}$ sind.

6. Verbindungen nach Anspruch 5, worin $R_3$ H ist und wenigstens eines aus $R_1$ oder $R_2$

$S(O)_nR_8$, $S(O)_mR_8$, $CF_3$, $NR_9R_{10}$, $SO_2NR_{12}R_{13}$, 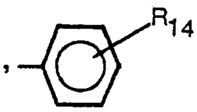 ,
$OSO_2R_{16}$ oder $CH_2OR_{17}$ ist.

7. Verbindungen nach Anspruch 6, worin $R_8$ $CH_3$ oder $C_2H_5$ ist; $R_9$ und $R_{10}$ beide $CH_3$ sind; $R_{12}$ $CH_3$ ist; $R_{14}$ H ist; $R_{16}$ $CH_3$ oder $C_2H_5$ ist; und $R_{17}$ $CH_3$ ist.

8. Verbindungen nach Anspruch 7, worin X $CH_3$, $OCH_3$ oder Cl ist und Y $CH_3$, $OCH_3$ oder $CH_2OCH_3$ ist.

9. Verbindungen nach Anspruch 2, worin D D—4 ist und Y $CH_3$, $C_2H_5$, $OCH_3$, $OC_2H_5$ oder $CH_2OCH_3$ ist.

10. Verbindungen nach Anspruch 9, worin E

$NR_{22}R_{23}$ oder 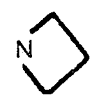 ist;

und $R_{23}$ H oder $CH_3$ ist.

11. Verbindungen nach Anspruch 10, worin E

$NR_{22}R_{23}$ oder 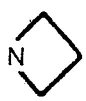 ist;

und X $CH_3$, $OCH_3$ oder Cl ist.

12. Verbindung nach Anspruch 1, welche 2 - (Azetidin - 1 - ylsulfonyl) - N - [(4 - methoxy - 6 - methyl - 2,3,5 - triazin - 2 - yl)aminocarbonyl]benzolsulfonamid oder ein landwirtschaftlich geeignetes Salz davon ist.

13. Verbindung nach Anspruch 1, ausgewählt aus N - [(4,6 - Dimethoxypyrimidin - 2 - yl)aminocarbonyl] - 3 - methylsulfonyl - 1,1' - biphenyl - 2 - sulfonamid; N,N' - Bis - [(4,6 - dimethoxypyrimidin - 2 - yl)aminocarbonyl] - 1,2 - benzoldisulfonamid; oder ein landwirtschaftlich geeignetes Salz von diesen ist.

14. Verbindung nach Anspruch 1, welche N - [(4,6 - Dimethoxy - 1,3,5 - triazin - 2 - yl)aminocarbonyl] - 2,6 - bis(methylthio)benzolsulfonamid oder ein landwirtschaftlich geeignetes Salz davon ist.

15. Verbindung nach Anspruch 1, ausgewählt aus 2 - (Azetidin - 1 - ylsulfonyl) - N - [(4,6 - dimethoxy - 1,3,5 - triazin - 2 - yl)aminocarbonyl]benzolsulfonamid, oder 2 - (Azetidin - 1 - ylsulfonyl) - N - [(4 - methoxy - 6 - methylpyrimidin - 2 - yl)aminocarbonyl]benzolsulfonamid oder ein landwirtschaftlich geeignetes Salz von diesen ist.

16. Verbindung nach Anspruch 1, worin D D—4 ist.

17. Landwirtschaftlich geeignete Zusammensetzung zur Bekämpfung des Wachstums unerwünschter Vegetation, welche eine wirksame Menge einer Verbindung nach einem der Ansprüche 1 bis 16 und wenigstens eines der folgenden: oberflächenaktives Mittel, festes oder flüssiges Verdünnungsmittel umfasst.

18. Verfahren zur Bekämpfung des Wachstums unerwünschter Vegetation, welches die Anwendung einer wirksamen Menge einer Verbindung nach einem der Ansprüche 1 bis 16 auf den zu schützenden Ort umfasst.

19. Verfahren zur Regulierung des Wachstums von Pflanzen, welches die Anwendung einer wirksamen, jedoch im wesentlichen nichtphytotoxischen Menge eines Pflanzenwachstumsreglers, ausgewählt aus den Verbindungen nach einem der Ansprüche 1 bis 16, auf den Ort solcher Pflanzen umfasst.

20. Verfahren zur Herstellung einer Verbindung nach Anspruch 1 durch

(A) Reagieren eines Sulfonamids der allgemeinen Formel (II)

$$DSO_2N_2 \quad (II)$$

oder einer Mischung eines Sulfonylchlorids $DSO_2Cl$ mit Isocyanatanion mit einem Methylcarbamat der allgemeinen Formel (III)

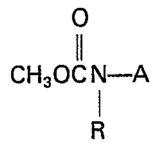

(III)

worin
A wie in Anspruch 1 definiert ist,
$R_1$, $R_2$, $R_4$, und $R_5$ nicht $CO_2R_{11}$ sind; und
R H ist; oder
(B) Reagieren eines Sulfonylcarbamats der allgemeinen Formel (IV)

$$DSO_2NHCO_2C_6H_5 \quad (IV)$$

mit einem geeigneten Amin der allgemeinen Formel (V)

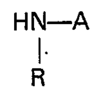

(V)

worin
A wie in Anspruch 1 definiert ist;
$R_1$ und $R_2$ nicht $CO_2R_{11}$ oder $OSO_2R_{16}$ sind; und
R H oder $CH_3$ ist; oder
(C) Reagieren eines Sulfonamids der allgemeinen Formel (II), wie oben definiert, oder der Mischung aus Sulfonylchlorid und Isocyanatanion, mit einem heterocyclischen Phenylcarbamat der allgemeinen Formel (VI)

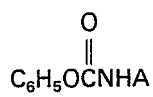

(VI)

worin A wie in Anspruch 1 definiert ist; oder
(D) Reagieren eines Benzolsulfonylisocyanats der allgemeinen Formel

$$DSO_2NCO \quad (VIII)$$

mit einem geeigneten Aminoheterozyklus der allgemeinen Formel

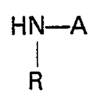

(V)

worin D, R und A wie in Anspruch 1 definiert sind.

**Patentansprüche für den Vertragsstaat: AT**

1. Verfahren zur Herstellung einer Verbindung der Formel

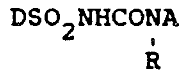

I

worin D

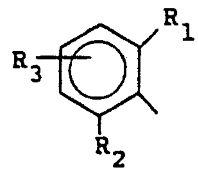

D-1

oder

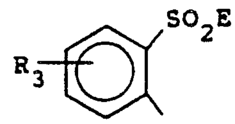

D-4

ist:

R H oder $CH_3$ ist;

$R_1$

$S(O)_nR_8$, $CF_3$, $NR_9R_{10}$, $CO_2R_{11}$, $SO_2NR_{12}R_{13}$, 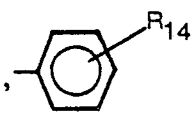

$CHCO_2R_{11}$, $OSO_2R_{16}$, $CH_2OR_{17}$ oder Q ist;
$R_{15}$

$R_2$

$S(O)_mR_8$, $CF_3$, $NR_9R_{10}$, $CO_2R_{11}$, $SO_2NR_{12}R_{13}$, 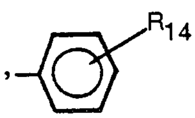

$CHCO_2R_{11}$, $OSO_2R_{16}$, $CH_2OR_{17}$ oder Q ist;
$R_{15}$

$R_3$ H, Cl, F, Br, $CH_3$, $OCH_3$ oder $CF_3$ ist;

E

$NR_{22}R_{23}$, 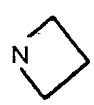, 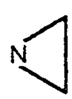 oder 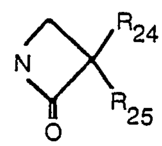 ist;

$R_8$ $C_1$—$C_3$-Alkyl ist;
$R_9$ und $R_{10}$ unabhängig $C_1$—$C_2$-Alkyl sind;
$R_{11}$ $C_1$—$C_3$-Alkyl, $CH_2CH=CH_2$, $CH_2CH_2Cl$ oder $CH_2CH_2OCH_3$ ist;
$R_{12}$ $CH_3$ oder $OCH_3$ ist;
$R_{13}$ $C_1$—$C_3$-Alkyl ist;
$R_{14}$ H, Cl, Br, F, $CH_3$ oder $OCH_3$ ist;
$R_{15}$ H oder $CH_3$ ist;
$R_{16}$ $C_1$—$C_3$-Alkyl oder $CF_3$ ist;
$R_{17}$ $C_1$—$C_{-3}$-Alkyl oder $CF_2H$ ist;
n 0, 1 oder 2 ist;
m 0 oder 1 ist;
Q

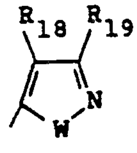

Q-1

,

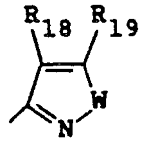

Q-2

,

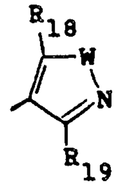

Q-3

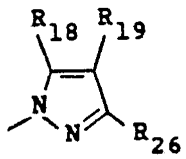 , 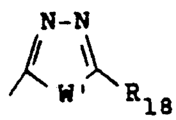 , 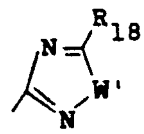 ,

Q-4 Q-5 Q-6

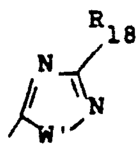 , 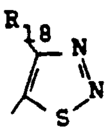 , 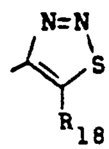 ,

Q-7 Q-8 Q-9

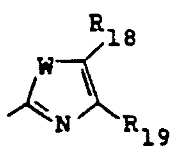 , 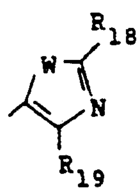 , 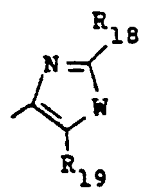 ,

Q-10 Q-11 Q-12

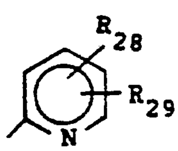 , 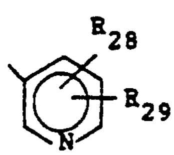 , 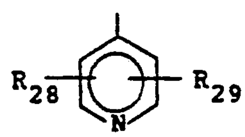 ,

Q-13 Q-14 Q-15

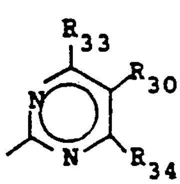 , 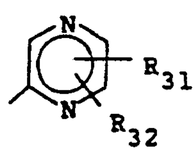 , 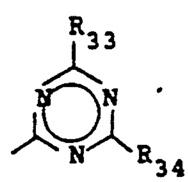 ,

Q-16 Q-17 Q-18

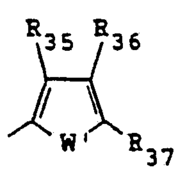 oder 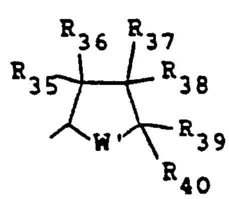 ist;

Q-19 Q-20

W O, S oder $NR_{27}$ ist;

W' O oder S ist;

$R_{18}$, $R_{19}$, $R_{24}$, $R_{25}$, $R_{26}$, $R_{27}$, $R_{28}$, $R_{29}$, $R_{30}$, $R_{31}$, $R_{32}$, $R_{35}$, $R_{36}$, $R_{37}$, $R_{38}$, $R_{39}$ und $R_{40}$ unabhängig H oder $CH_3$ sind;

$R_{33}$ und $R_{34}$ unabhängig H, $CH_3$ oder $OCH_3$ sind;

$R_{22}$ $C(O)R_{41}$, $C(O)NR_{42}R_{43}$, $CO_2R_{44}$, $C(O)NHR_{45}$ oder $CF_2H$ ist;

$R_{23}$ H oder $C_1$—$C_3$-Alkyl ist;

$R_{41}$ $CF_3$ oder gegebenenfalls durch Cl, $CH_3$, $CF_3$, $NO_2$ oder $OCH_3$ substituiertes Phenyl ist;

$R_{42}$ H, $C_1$—$C_4$-Alkyl oder gegebenenfalls durch Cl, $CH_3$, $CF_3$, $NO_2$ oder $OCH_3$ substituiertes Phenyl ist;

$R_{43}$ H oder $C_1$—$C_4$-Alkyl ist;

$R_{42}$ und $R_{43}$ zusammengenommen werden können zu $-(CH_2)_4-$, $-(CH_2)_5-$ oder $-CH_2CH_2OCH_2CH_2-$;

$R_{44}$ $C_1$—$C_4$-Alkyl ist;

$R_{45}$ A—1 ist;

A

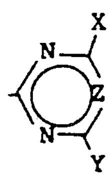 , 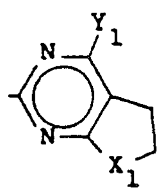 , 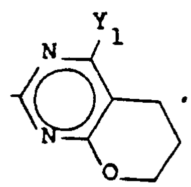 ,

A-1 A-2 A-3

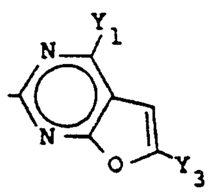 , 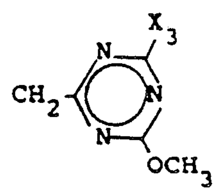 oder 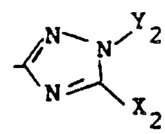 ist;

A-4 A-5 A-6

X $CH_3$, $OCH_3$, $OCF_2H$ oder Cl ist;

Y $CH_3$, $C_2H_5$, $OCH_3$, $OC_2H_5$, $CH_2OCH_3$, $OCH_2CF_3$, $OCF_2H$, $NHCH_3$, $N(CH_3)_2$ oder $SCH_3$ ist;

$X_1$ $CH_2$ oder O ist;

$Y_1$ $CH_3$ oder $OCH_3$ ist;

$X_2$ $CH_3$, $OCH_3$ oder $SCH_3$ ist;

$Y_2$ $CH_3$, $C_2H_5$ oder $CH_2CF_3$ ist;

$X_3$ $CH_3$ oder $OCH_3$ ist; und

$Y_3$ H oder $CH_3$ ist;

Z CH oder N ist;

und ihre landwirtschaftlich geeigneten Salze; mit der Massgabe, dass

a) wenn eines aus $R_1$ oder $R_2$ $CF_3$ ist, dann das andere ebenfalls $CF_3$ ist, X von Cl verschieden ist, wenn Y $NHCH_3$ ist, und X und Y von $OCF_2H$ verschieden sind;

b) $R_1$ und $R_2$ nicht gleichzeitig $CO_2R_{11}$ sind;

c) wenn eines aus $R_1$ oder $R_2$ $SO_2NR_{12}R_{13}$ ist, dann das andere nicht $S(O)_nR_8$, $S(O)_mR_8$, $CH_2OR_{17}$ oder $NR_9R_{10}$ sein darf;

d) wenn $R_{12}$ $OCH_3$ ist, dann $R_{13}$ $CH_3$ ist;

e) wenn eines aus $R_1$ oder $R_2$ $CO_2R_{11}$ ist, dann das andere nicht $S(O)_nR_8$, $S(O)_mR_8$ oder $NR_9R_{10}$ sein darf;

f) die Gesamtzahl der Kohlenstoffatome in $R_{35}$ bis $R_{40}$ zusammengenommen gleich oder weniger als vier ist;

g) wenn X Cl ist, dann Z CH ist und Y $NHCH_3$, $N(CH_3)_2$, $OCH_3$ oder $OC_2H_5$ ist;

h) wenn $R_1$ $CO_2R_{11}$ ist und $R_2$ $OSO_2R_{16}$ ist, dann A nicht A—1 ist;

j) wenn $R_3$ ist, A A—1 ist und eines aus $R_1$ oder $R_2$ Q ist und Q nicht Q—4 oder Q—20 ist, dann das andere aus $R_1$ oder $R_2$ nicht $S(O)_nR_8$, $S(O)_mR_8$ $CF_3$ oder $CO_2R_{11}$ sein darf;

k) wenn A A—6 ist, dann $R_1$

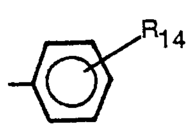 , $\underset{|\atop R_{15}}{CHCO_2R_{11}}$, $CH_2OR_{17}$ oder Q ist;

l) wenn $R_{22}$ C(O)NH$R_{45}$ ist, dann A A—1 sein muss oder die Werte von X, Y und Z für sowohl A als auch $R_{45}$ identisch sein müssen;

m) wenn D D—4 ist, dann R H ist; und

n) wenn $R_1$ und $R_2$ $CF_3$ sind, dann A von A—5 verschieden ist, durch

(A) Reagieren eines Sulfonamids der allgemeinen Formel (II)

$DSO_2N_2$ (II)

oder einer Mischung eines Sulfonylchlorids $DSO_2Cl$ mit Isocyanatanion mit einem Methylcarbamat der allgemeinen Formel (III)

O
‖
$CH_3OC$N—A (III)
|
R

worin

A wie oben definiert ist,

$R_1$, $R_2$, $R_4$, und $R_5$ nicht $CO_2R_{11}$ sind; und

R H ist; oder

(B) Reagieren eines Sulfonylcarbamats der allgemeinen Formel (IV)

$DSO_2NHCO_2C_6H_5$ (IV)

mit einem geeigneten Amin der allgemeinen Formel (V)

HN—A
|
R (V)

worin

A wie oben definiert ist;

$R_1$ und $R_2$ nicht $CO_2R_{11}$ oder $OSO_2R_{16}$ sind; und

R H oder $CH_3$ ist; oder

(C) Reagieren eines Sulfonamids der allgemeinen Formel (II), wie oben definiert, oder der Mischung aus Sulfonylchlorid und Isocyanatanion, mit einem heterocyclischen Phenylcarbamat der allgemeinen Formel (VI)

O
‖
$C_6H_5OCNHA$ (VI)

worin A wie oben definiert ist; oder

(D) Reagieren eines Benzolsulfonylisocyanats der allgemeinen Formel

$DSO_2NCO$ (VIII)

mit einem geeigneten Aminoheterozyklus der allgemeinen Formel

HN—A
|
R (V)

worin D, R und A wie für Formel (I) definiert sind.

2. Verfahren nach Anspruch 1, worin A A—1 ist; R H ist; $R_1$ und $R_2$ nicht gleichzeitig Q sind; D D—1 oder D—4 ist; und, wenn D von D—1 verschieden ist, dann $R_3$ H ist.

3. Verfahren nach Anspruch 2, worin D D—1 ist und Y $CH_3$, $CH_2OCH_3$, $OCH_3$, $OCH_2CF_3$ oder $OCF_2H$ ist.

4. Verfahren nach Anspruch 4, worin $R_1$ und $R_2$ nicht gleichzeitig $SO_2NR_{12}R_{13}$ oder $NR_9R_{10}$ sind; $R_3$ H ist und wenigstens eines aus $R_1$ oder $R_2$

$S(O)_nR_8$, $S(O)_mR_8$, $CF_3$, $NR_9R_{10}$, $SO_2NR_{12}R_{13}$, 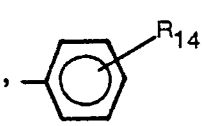 ,

$OSO_2R_{16}$ oder $CH_2OR_{17}$ ist;

$R_8$ $CH_3$ oder $C_2H_5$ ist; $R_9$ und $R_{10}$ beide $CH_3$ sind; $R_{12}$ $CH_3$ ist; $R_{14}$ H ist; $R_{16}$ $CH_3$ oder $C_2H_5$ ist; und $R_{17}$ $CH_3$ ist; X $CH_3$, $OCH_3$ oder Cl ist und Y $CH_3$, $OCH_3$ oder $CH_2OCH_3$ ist.

5. Verfahren nach Anspruch 2, worin D D—4 ist; und Y $CH_3$, $C_2H_5$, $OCH_3$, $OC_2H_5$ oder $CH_2OCH_3$ ist; E

$NR_{22}R_{23}$ oder 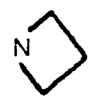 ist;

$R_{22}$ und $R_{23}$ beide H sind und X $CH_3$, $OCH_3$ oder Cl ist.

6. Verfahren nach Anspruch 1, worin das Produkt eine Verbindung ist, ausgewählt aus 2 - (Azetidin - 1 - ylsulfonyl) - N - [(4 - methoxy - 6 - methyl - 2,3,5 - triazin - 2 - yl)aminocarbonyl]benzolsulfonamid, N - [(4,6 - Dimethoxypyrimidin - 2 - yl)aminocarbonyl] - 3 - methylsulfonyl - 1,1′ - biphenyl - 2 - sulfonamid; N,N′ - Bis - [(4,6 - dimethoxypyrimidin - 2 - yl)aminocarbonyl] - 1,2 - benzoldisulfonamid, N - [(4,6 - Dimethoxy - 1,3,5 - triazin - 2 - yl)aminocarbonyl] - 2,6 - bis(methylthio)benzolsulfonamid, 2 - (Azetidin - 1 - ylsulfonyl) - N - [(4,6 - dimethoxy - 1,3,5 - triazin - 2 - yl)aminocarbonyl]benzolsulfonamid oder 2 - (Azetidin - 1 - ylsulfonyl) - N - [(4 - methoxy - 6 - methylpyrimidin - 2 - yl)aminocarbonyl]benzolsulfonamid oder ein landwirtschaftlich geeignetes Salz davon.

7. Landwirtschaftlich geeignete Zusammensetzung zur Bekämpfung des Wachstums unerwünschter Vegetation, welche eine wirksame Menge einer Verbindung der Formel (I), wie in einem der Ansprüche 1 bis 6 definiert, und wenigstens eines der folgenden: oberflächenaktives Mittel, festes oder flüssiges Verdünnungsmittel umfasst.

8. Verfahren zur Bekämpfung des Wachstums unerwünschter Vegetation, welches die Anwendung einer wirksamen Menge einer Verbindung der Formel (I), wie in einem der Ansprüche 1 bis 6 definiert, auf den zu schützenden Ort umfasst.

9. Verfahren zur Regulierung des Wachstums von Pflanzen, welches die Anwendung einer wirksamen, jedoch im wesentlichen nichtphytotoxischen Menge eines Pflanzenwachstumsreglers, ausgewählt aus den Verbindungen der Formel (I), wie in einem der Ansprüche 1 bis 6 definiert, auf den Ort solcher Pflanzen umfasst.

**Revendications pour les Etats contractants: BE CH DE FR GB IT LI LU NL SE**

1. Un composé de la formule:

$$\underset{\displaystyle R}{DSO_2NHCONA}$$

I

dans laquelle

D est 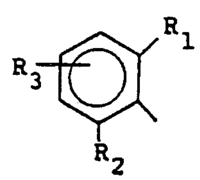 ou 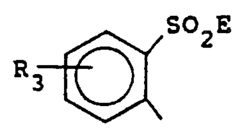

D-1 D-4

R est H ou $CH_3$;

$R_1$ est

$S(O)_nR_8$, $CF_3$, $NR_9R_{10}$, $CO_2R_{11}$,

$SO_2NR_{12}R_{13}$, 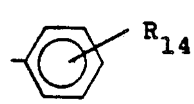 , $\underset{R_{15}}{CHCO_2R_{11}}$,

$OSO_2R_{16}$, $CH_2OR_{17}$ ou Q;

$R_2$ est

$S(O)_mR_8$, $CF_3$, $NR_9R_{10}$, $CO_2R_{11}$,

$SO_2NR_{12}R_{13}$, 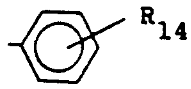 , 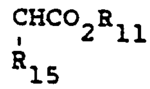,

$OSO_2R_{16}$, $CH_2OR_{17}$ ou Q;

$R_3$ est H, Cl, F, Br, $CH_3$, $OCH_3$ ou $CF_3$;
E est

$NR_{22}R_{23}$, 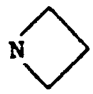, 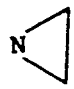 ou 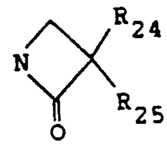 ;

$R_8$ est un groupe alkyle en $C_1$—$C_3$;
$R_9$ et $R_{10}$ sont indépendamment un groupe alkyle en $C_1$—$C_2$;
$R_{11}$ est un groupe alkyle en $C_1$—$C_3$, $CH_2CH{=}CH_2$, $CH_2CH_2Cl$ ou $CH_2CH_2OCH_3$;
$R_{12}$ est $CH_3$ ou $OCH_3$;
$R_{13}$ est un groupe alkyle en $C_1$—$C_3$;
$R_{14}$ est H, Cl, Br, F, $CH_3$ ou $OCH_3$;
$R_{15}$ est H ou $CH_3$;
$R_{16}$ est un groupe alkyle en $C_1$—$C_3$ ou $CF_3$;
$R_{17}$ est un groupe alkyle en $C_1$—$C_{-3}$ ou $CF_2H$;
n est 0, 1 ou 2;
m est 0 ou 1;
Q est

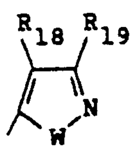 , 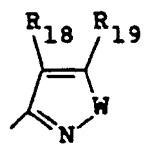 , 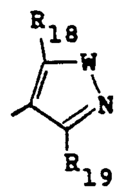 ,

Q-1 Q-2 Q-3

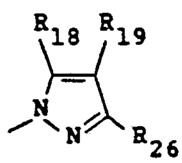 , 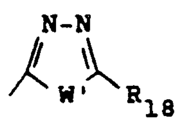 , 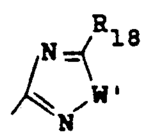 ,

Q-4 Q-5 Q-6

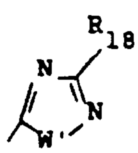 , 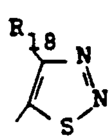 , 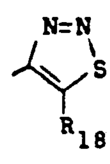 ,

Q-7 Q-8 Q-9

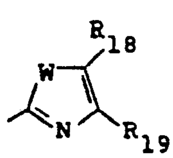 , 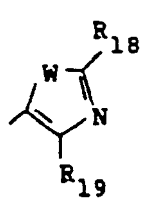 , 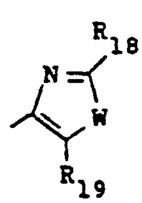 ,

Q-10 Q-11 Q-12

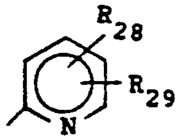 , 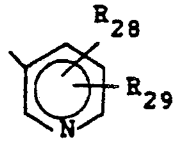 , 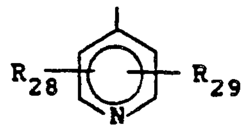 ,

Q-13 Q-14 Q-15

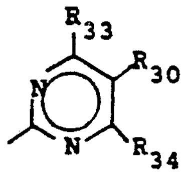 , 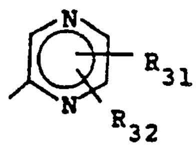 , 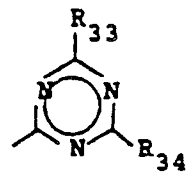 ,

Q-16 Q-17 Q-18

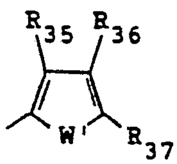 or 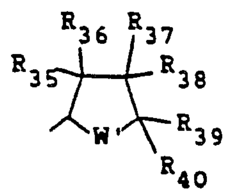 ;

Q-19 Q-20

W est O, S ou $NR_{27}$;

W' est O ou S

$R_{18}$, $R_{19}$, $R_{24}$, $R_{25}$, $R_{26}$, $R_{27}$, $R_{28}$, $R_{29}$, $R_{30}$, $R_{31}$, $R_{32}$, $R_{35}$, $R_{36}$, $R_{37}$, $R_{38}$, $R_{39}$ et $R_{40}$ sont indépendamment H ou $CH_3$;

$R_{33}$ et $R_{34}$ sont indépendamment H, $CH_3$ ou $OCH_3$;

$R_{22}$ est $C(O)R_{41}$, $C(O)NR_{42}R_{43}$, $CO_2R_{44}$, $C(O)NHR_{45}$ ou $CF_2H$;

$R_{23}$ est H ou un groupe alkyle en $C_1$—$C_3$;

$R_{41}$ est $CF_3$ ou un groupe phényle facultativement substitué par Cl, $CH_3$, $CF_3$, $NO_2$ ou $OCH_3$;

$R_{42}$ est H, un groupe alkyle en $C_1$—$C_4$ ou un groupe phényle facultativement substitué par Cl, $CH_3$, $CF_3$, $NO_2$ ou $OCH_3$;

$R_{43}$ est H ou un groupe alkyle en $C_1$—$C_4$;

$R_{42}$ et $R_{43}$ peuvent être pris ensemble pour former $—(CH_2)_4—$, $—(CH_2)_5—$ ou $—CH_2CH_2OCH_2CH_2—$;

$R_{44}$ est un groupe alkyle en $C_1$—$C_4$;

$R_{45}$ est A—1;

A est

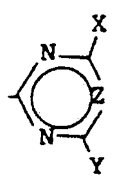 , 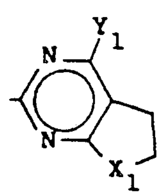 , 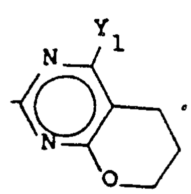 ,

A-1 A-2 A-3

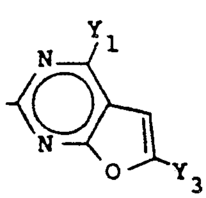 , 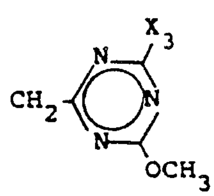 ou 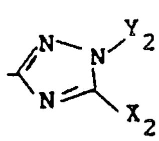 ;

A-4 A-5 A-6

X est $CH_3$, $OCH_3$, $OCF_2H$ ou Cl;
Y est $CH_3$, $C_2H_5$, $OCH_3$, $OC_2H_5$, $CH_2OCH_3$, $OCH_2CF_3$, $OCF_2H$, $NHCH_3$, $N(CH_3)_2$ ou $SCH_3$;
$X_1$ est $CH_2$ ou O;
$Y_1$ est $CH_3$ ou $OCH_3$;
$X_2$ est $CH_3$, $OCH_3$ ou $SCH_3$;
$Y_2$ est $CH_3$, $C_2H_5$ ou $CH_2CF_3$;
$X_3$ est $CH_3$ ou $OCH_3$;
$Y_3$ est H ou $CH_3$; et
Z est CH ou N;

et ses sels utilisables en agriculture; avec les conditions suivantes

a) si l'un de $R_1$ et $R_2$ est $CF_3$, alors l'autre est également $CF_3$, X est autre chose que Cl lorsque Y est $NHCH_3$, et X et Y sont autre chose que $OCF_2H$;

b) $R_1$ et $R_2$ ne sont pas simultanément $CO_2R_{11}$;

c) si l'un de $R_1$ et $R_2$ est $SO_2NR_{12}R_{13}$, alors l'autre ne doit pas être $S(O)_nR_8$, $S(O)_mR_8$, $CH_2OR_{17}$ ou $NR_9R_{10}$;

d) si $R_{12}$ est $OCH_3$, alors $R_{13}$ est $CH_3$;

e) si l'un de $R_1$ et $R_2$ est $CO_2R_{11}$, alors l'autre ne doit pas être $S(O)_nR_8$, $S(O)_mR_8$ ou $NR_9R_{10}$;

f) le nombre total d'atomes de carbone dans $R_{35}$ à $R_{40}$ combinés est égal ou inférieur à quatre;

g) si X est Cl, alors Z est CH et Y est $NHCH_3$, $N(CH_3)_2$, $OCH_3$ ou $OC_2H_5$;

h) si $R_1$ est $CO_2R_{11}$ et $R_2$ est $OSO_2R_{16}$, alors A n'est pas A—1;

j) si $R_3$ est H, A est A—1 et l'un de $R_1$ et $R_2$ est Q et Q n'est pas Q—4 ou Q—20, alors l'autre de $R_1$ et $R_2$ ne doit pas être $S(O)_nR_8$, $S(O)_mR_8$, $CF_3$ ou $CO_2R_{11}$;

k) si A est A—6, alors $R_1$ est

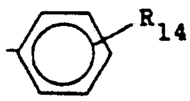, $\underset{R_{15}}{CHCO_2R_{11}}$, $CH_2OR_{17}$ ou Q;

l) si $R_{22}$ est $C(O)NHR_{45}$, alors A doit être A—1 et les significations de X, Y et Z doivent être identiques pour A et $R_{45}$;

m) si D est D—4, alors R est H; et

n) si $R_1$ et $R_2$ sont $CF_3$, alors A est autre chose que A—5.

2. Les composés de la revendication 1, où A est A—1; R est H; $R_1$ et $R_2$ ne sont pas simultanément Q; D est D—1 ou D—4; et si D est autre chose que D—1, alors $R_3$ est H.

3. Les composés de la revendication 2, où D est D—1 et Y est $CH_3$, $CH_2OCH_3$, $OCH_3$, $OCH_2CF_3$ ou $OCF_2H$.

4. Les composés de la revendication 3, où $R_3$ est à l'une ou l'autre des positions 3 ou 5.

5. Les composés de la revendication 4, où $R_1$ et $R_2$ ne sont pas simultanément $SO_2NR_{12}R_{13}$ ou $NR_9R_{10}$.

6. Les composés de la revendication 5, où $R_3$ est H et l'un au moins de $R_1$ ou $R_2$ est

$S(O)_nR_8$, $S(O)_mR_8$, $CF_3$,

$NR_9R_{10}$, $SO_2NR_{12}R_{13}$, 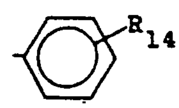, $OSO_2R_{16}$ ou $CH_2OR_{17}$.

7. Les composés de la revendication 6, où $R_8$ est $CH_3$ ou $C_2H_5$; $R_9$ et $R_{10}$ sont tous deux $CH_3$; $R_{12}$ est $CH_3$; $R_{14}$ est H, $R_{16}$ est $CH_3$ ou $C_2H_5$; et $R_{17}$ est $CH_3$.

8. Les composés de la revendication 7, où X est $CH_3$, $OCH_3$ ou Cl, et Y est $CH_3$, $OCH_3$ ou $CH_2OCH_3$.

9. Les composés de la revendication 2, où D est D—4 et Y est $CH_3$, $C_2H_5$, $OCH_3$, $OC_2H_5$ ou $CH_2OCH_3$.

10. Les composés de la revendication 9, où E est

$NR_{22}R_{23}$ ou 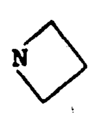 ;

et $R_{23}$ est H ou $CH_3$.

11. Les composés de la revendication 10, où E est

$NR_{22}R_{23}$ ou 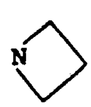 ;

et X est $CH_3$, $OCH_3$ ou Cl.

12. Le composé de la revendication 1, qui est le 2 - (azétidine - 1 - ylsulfonyl) - N - [(4 - méthoxy - 6 - méthyl - 1,3,5 - triazine - 2 - yl)aminocarbonyl]benzènesulfonamide, ou un sel utilisable en agriculture de celui-ci.

13. Un composé de la revendication 1, choisi parmi le N - [(4,6 - diméthoxypyrimidine - 2 - yl)aminocarbonyl] - 3 - méthylsulfonyl - 1,1′ - biphényle - 2 - sulfonamide, le N,N′ - bis - [(4,6 - diméthoxypyrimidine - 2 - yl)aminocarbonyl] - 1,2 - benzènedisulfonamide, et un sel utilisable en agriculture de l'un quelconque de ceux-ci.

14. Un composé de la revendication 1, qui est le N - [(4,6 - diméthoxy - 1,3,5 - triazine - 2 - yl)aminocarbonyl] - 2,6 - bis(méthylthio)benzènesulfonamide ou un sel utilisable en agriculture de celui-ci.

15. Un composé de la revendication 1, choisi parmi le 2 - (azétidine - 1 - ylsulfonyl) - N - [(4,6 - diméthoxy - 1,3,5 - triazine - 2 - yl)aminocarbonyl]benzènesulfonamide, le 2 - (azétidine - 1 - ylsulfonyl) - N - [(4 - méthoxy - 6 - méthylpyrimidine - 2 - yl)aminocarbonyl]benzènesulfonamide, et un sel utilisable en agriculture de l'un ou l'autre de ceux-ci.

16. Un composé de la revendication 1, où D est D—4.

17. Une composition utilisable en agriculture pour lutter contre la croissance de végétation indésirable, comprenant une quantité efficace d'un composé de l'une quelconque des revendications 1 à 16 et l'un au moins des ingrédients suivants: agent tensioactif, diluant solide ou diluant liquide.

18. Un procédé pour lutter contre la croissance de végétation indésirable, qui consiste à appliquer au lieu à protéger une quantité efficace d'un composé de l'une quelconque des revendications 1 à 16.

19. Un procédé pour réguler la croissance des plantes, qui consiste à appliquer au lieu où se trouvent ces plantes une quantité efficace mais sensiblement non phytotoxique d'un régulateur de croissance des plantes choisi parmi les composés de l'une quelconque des revendications 1 à 16.

20. Un procédé pour la préparation d'un composé de la revendication 1, qui consiste:

(A) à faire réagir un sulfonamide de formule générale (II)

$$DSO_2NH_2 \qquad (II)$$

ou un mélange d'un chlorure de sulfonyle $DSO_2Cl$ avec un anion isocyanate, avec un carbamate de méthyle de formule générale (III)

```
      O
      ‖
CH3OC N—A
        |
        R
```

dans laquelle:

A est tel que défini dans la revendication 1;
$R_1$, $R_2$, $R_4$ et $R_5$ ne sont pas $CO_2R_{11}$; et
R est H; ou

(B) à faire réagir un sulfonylcarbamate de formule générale (IV)

$$DSO_2NHCO_2C_6H_5 \qquad (IV)$$

avec une amine appropriée de formule générale V)

```
HN—A
|
R          (V)
```

dans laquelle:

A est tel que défini dans la revendication 1;
$R_1$ et $R_2$ ne sont pas $CO_2R_{11}$ ni $OSO_2R_{16}$; et
R est H ou $CH_3$; ou

(C) à faire réagir un sulfonamide de formule générale (II) comme définie ci-dessus, ou ledit mélange de chlorure de sulfonyle et d'anion isocyanate, avec un phénylcarbamate hétérocyclique de formule générale (VI)

```
       O
       ‖
C6H5OCNHA          (VI)
```

dans laquelle A est tel que défini dans la revendication 1; ou

(D) à faire réagir un isocyanate de benzènesulfonyle de formule générale

$$DSO_2NCO \qquad (VIII)$$

avec un aminohétérocycle approprié de formule générale

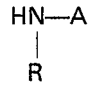 (V)

dans laquelle D, R et A sont tels que définis dans la revendication 1.

**Revendications pour l'Etat contractant: AT**

1. Un procédé pour la préparation d'un composé de la formule:

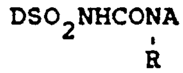

I

dans laquelle

D est 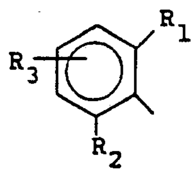 ou 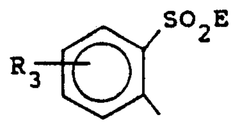

D-1 D-4

R est H ou $CH_3$;
$R_1$ est

$S(O)_nR_8$, $CF_3$, $NR_9R_{10}$, $CO_2R_{11}$,

$SO_2NR_{12}R_{13}$, 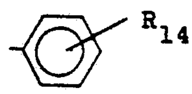, 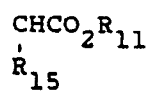,

$OSO_2R_{16}$, $CH_2OR_{17}$ ou Q;

$R_2$ est

$S(O)_mR_8$, $CF_3$, $NR_9R_{10}$, $CO_2R_{11}$,

$SO_2NR_{12}R_{13}$, 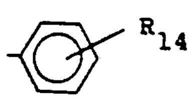, 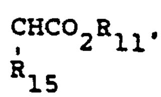,

$OSO_2R_{16}$, $CH_2OR_{17}$ ou Q;

$R_3$ est H, Cl, F, Br, $CH_3$, $OCH_3$ ou $CF_3$;
E est

$NR_{22}R_{23}$, 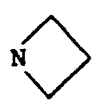, 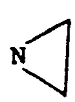 ou 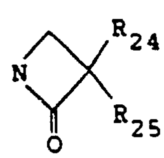;

$R_8$ est un groupe alkyle en $C_1$—$C_3$;
$R_9$ et $R_{10}$ sont indépendamment un groupe alkyle en $C_1$—$C_2$;
$R_{11}$ est un groupe alkyle en $C_1$—$C_3$, $CH_2CH{=}CH_2$, $CH_2CH_2Cl$ ou $CH_2CH_2OCH_3$;
$R_{12}$ est $CH_3$ ou $OCH_3$;
$R_{13}$ est un groupe alkyle en $C_1$—$C_3$;
$R_{14}$ est H, Cl, Br, F, $CH_3$ ou $OCH_3$;

$R_{15}$ est H ou $CH_3$;
$R_{16}$ est un groupe alkyle en $C_1$—$C_3$ ou $CF_3$;
$R_{17}$ est un groupe alkyle en $C_1$—$C_3$ ou $CF_2H$;
n est 0, 1 ou 2;
m est 0 ou 1;
Q est

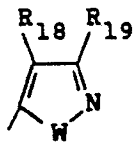 ,
Q-1

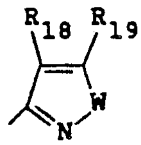 ,
Q-2

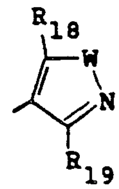 ,
Q-3

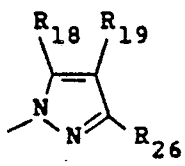 ,
Q-4

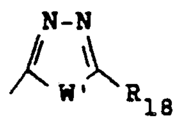 ,
Q-5

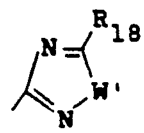 ,
Q-6

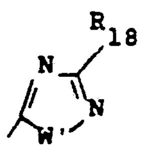 ,
Q-7

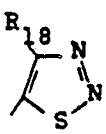 ,
Q-8

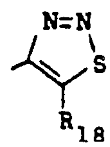 ,
Q-9

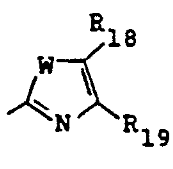 ,
Q-10

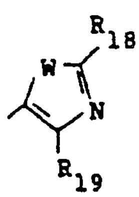 ,
Q-11

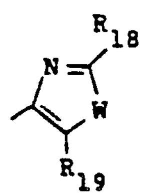 ,
Q-12

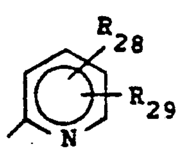 ,
Q-13

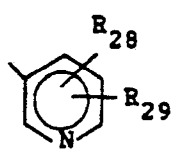 ,
Q-14

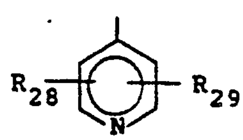 ,
Q-15

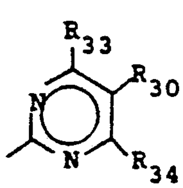 ,
Q-16

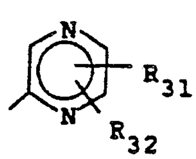 ,
Q-17

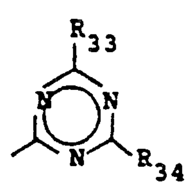 ,
Q-18

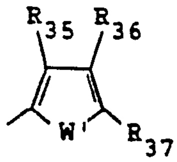 ou 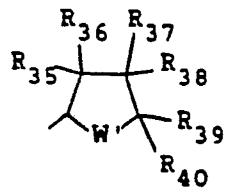 ;

Q-19 Q-20

W est O, S ou $NR_{27}$;

W' est O ou S

$R_{18}$, $R_{19}$, $R_{24}$, $R_{25}$, $R_{26}$, $R_{27}$, $R_{28}$, $R_{29}$, $R_{30}$, $R_{31}$, $R_{32}$, $R_{35}$, $R_{36}$, $R_{37}$, $R_{38}$, $R_{39}$ et $R_{40}$ sont indépendamment H ou $CH_3$;

$R_{33}$ et $R_{34}$ sont indépendamment H, $CH_3$ ou $OCH_3$;

$R_{22}$ est $C(O)R_{41}$, $C(O)NR_{42}R_{43}$, $CO_2R_{44}$, $C(O)NHR_{45}$ ou $CF_2H$;

$R_{23}$ est H ou un groupe alkyle en $C_1$—$C_3$;

$R_{41}$ est $CF_3$ ou un groupe phényle facultativement substitué par Cl, $CH_3$, $CF_3$, $NO_2$ ou $OCH_3$;

$R_{42}$ est H, un groupe alkyle en $C_1$—$C_4$ ou un groupe phényle facultativement substitué par Cl, $CH_3$, $CF_3$, $NO_2$ ou $OCH_3$;

$R_{43}$ est H ou un groupe alkyle en $C_1$—$C_4$;

$R_{42}$ et $R_{43}$ peuvent être pris ensemble pour former $-(CH_2)_4-$, $-(CH_2)_5-$ ou $-CH_2CH_2OCH_2CH_2-$;

$R_{44}$ est un groupe alkyle en $C_1$—$C_4$;

$R_{45}$ est A—1;

A est

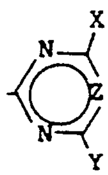 , 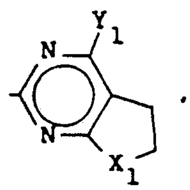 , 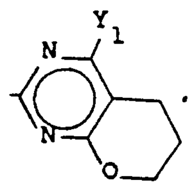 ,

A-1 A-2 A-3

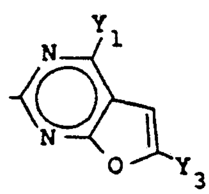 , 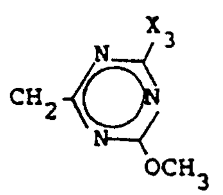 ou 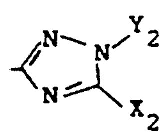 ;

A-4 A-5 A-6

X est $CH_3$, $OCH_3$, $OCF_2H$ ou Cl;

Y est $CH_3$, $C_2H_5$, $OCH_3$, $OC_2H_5$, $CH_2OCH_3$, $OCH_2CF_3$, $OCF_2H$, $NHCH_3$, $N(CH_3)_2$ ou $SCH_3$;

$X_1$ est $CH_2$ ou O;

$Y_1$ est $CH_3$ ou $OCH_3$;

$X_2$ est $CH_3$, $OCH_3$ ou $SCH_3$;

$Y_2$ est $CH_3$, $C_2H_5$ ou $CH_2CF_3$;

$X_3$ est $CH_3$ ou $OCH_3$; et

$Y_3$ est H ou $CH_3$;

Z est CH ou N;

ou un sel utilisable en agriculture de celui-ci; avec les conditions suivantes

a) si l'un de $R_1$ et $R_2$ est $CF_3$, alors l'autre est également $CF_3$, X est autre chose que Cl lorsque Y est $NHCH_3$, et X et Y sont autre chose que $OCF_2H$;

b) $R_1$ et $R_2$ ne sont pas simultanément $CO_2R_{11}$;

c) si l'un de $R_1$ et $R_2$ est $SO_2NR_{12}R_{13}$, alors l'autre ne doit pas être $S(O)_nR_8$, $S(O)_mR_8$, $CH_2OR_{17}$ ou $NR_9R_{10}$;

d) si $R_{12}$ est $OCH_3$, alors $R_{13}$ est $CH_3$;

e) si l'un de $R_1$ et $R_2$ est $CO_2R_{11}$, alors l'autre ne doit pas être $S(O)_nR_8$, $S(O)_mR_8$ ou $NR_9R_{10}$;

f) le nombre total d'atomes de carbone dans $R_{35}$ à $R_{40}$ combinés est égal ou inférieur à quatre;

g) si X est Cl, alors Z est CH et Y est $NHCH_3$, $N(CH_3)_2$, $OCH_3$ ou $OC_2H_5$;

h) si $R_1$ est $CO_2R_{11}$ et $R_2$ est $OSO_2R_{16}$, alors A n'est pas A—1;

j) si $R_3$ est H, A est A—1 et l'un de $R_1$ et $R_2$ est Q et Q n'est pas Q—4 ou Q—20, alors l'autre de $R_1$ et $R_2$ ne doit pas être $S(O)_nR_8$, $S(O)_mR_8$, $CF_3$ ou $CO_2R_{11}$;

k) si A est A—6, alors $R_1$ est

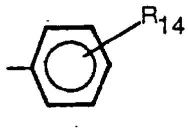 , $\underset{R_{15}}{\underset{|}{CHCO_2R_{11}}}$, $CH_2OR_{17}$ ou Q ;

l) si $R_{22}$ est $C(O)NHR_{45}$, alors A doit être A—1 et les significations de X, Y et Z doivent être identiques pour A et $R_{45}$;

m) si D est D—4, alors R est H; et

n) si $R_1$ et $R_2$ sont $CF_3$, alors A est autre chose que A—5.

qui consiste

(A) à faire réagir un sulfonamide de formule générale (II)

$$DSO_2NH_2 \qquad (II)$$

ou un mélange d'un chlorure de sulfonyle $DSO_2Cl$ avec un anion isocyanate, avec un carbamate de méthyle de formule générale (III)

$$CH_3O\overset{O}{\overset{\|}{C}}\underset{R}{\underset{|}{N}}—A \qquad (III)$$

dans laquelle:

A est tel que défini dans la revendication 1;

$R_1$, $R_2$, $R_4$ et $R_5$ ne sònt pas $CO_2R_{11}$; et

R est H; ou

(B) à faire réagir un sulfonylcarbamate de formule générale (IV)

$$DSO_2NHCO_2C_6H_5 \qquad (IV)$$

avec une amine appropriée de formule générale V)

$$\underset{R}{\underset{|}{HN}}—A \qquad (V)$$

dans laquelle:

A est tel que défini ci dessus;

$R_1$ et $R_2$ ne sont pas $CO_2R_{11}$ ou $OSO_2R_{16}$; et

R est H ou $CH_3$; ou

(C) à faire réagir un sulfonamide de formule générale (II) comme définie ci-dessus, ou ledit mélange de chlorure de sulfonyle et d'anion isocyanate, avec un phénylcarbamate hétérocyclique de formule générale (VI)

$$C_6H_5O\overset{O}{\overset{\|}{C}}NHA \qquad (VI)$$

dans laquelle A est tel que défini ci dessus; ou

(D) à faire réagir un isocyanate de benzènesulfonyle de formule générale

$$DSO_2NCO \qquad (VIII)$$

avec un aminohétérocycle approprié de formule générale

$$\underset{R}{\underset{|}{HN}}—A \qquad (V)$$

dans laquelle D, R et A sont tels que définis pour la formule (I).

2. Le procédé de la revendication 1, où A est A—1; R est H; $R_1$ et $R_2$ ne sont pas simultanément Q; D est D—1 ou D—4; et si D est autre chose que D—1, alors $R_3$ est H.

3. Le procédé de la revendication 2, où D est D—1 et Y est $CH_3$, $CH_2OCH_3$, $OCH_3$, $OCH_2CF_3$ ou $OCF_2H$.

4. Le procédé de la revendication 3, où $R_1$ et $R_2$ ne sont pas simultanément $SO_2NR_{12}R_{13}$ ou $NR_9R_{10}$; $R_3$ est H et l'un au moins de $R_1$ et $R_2$ est

$S(O)_nR_8$, $S(O)_mR_8$, $CF_3$,

$NR_9R_{10}$, $SO_2NR_{12}R_{13}$, 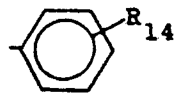, $OSO_2R_{16}$ ou $CH_2OR_{17}$;

$R_8$ est $CH_3$ ou $C_2H_5$; $R_9$ et $R_{10}$ sont tous deux $CH_3$; $R_{12}$ est $CH_3$;
$R_{14}$ est H; $R_{16}$ est $CH_3$ ou $C_2H_5$;
$R_{17}$ est $CH_3$;
X est $CH_3$, $OCH_3$ ou Cl, et Y est $CH_3$, $OCH_3$ ou $CH_2OCH_3$.

5. Le procédé de la revendication 2, où D est D—4; Y est $CH_3$, $C_2H_5$, $OCH_3$, $OC_2H_5$ ou $CH_2OCH_3$; E est

$NR_{22}R_{23}$ ou 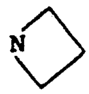 ;

$R_{22}$ et $R_{23}$ sont tous deux H; et X est $CH_3$, $OCH_3$ ou Cl.

6. Le procédé de la revendication 1, dans lequel le produit est un composé choisi parmi

le 2-(azétidine-1-ylsulfonyl)-N-[(4-méthoxy-6-méthyl-1,3,5-triazine-2-yl)aminocarbonyl]benzène-sulfonamide;

le N-[(4,6-diméthoxypyrimidine-2-yl)aminocarbonyl]-3-méthylsulfonyl-1,1'-biphényle-2-sulfonamide;

le N,N'-bis[(4,6-diméthoxypyrimidine-2-yl)aminocarbonyl]-1,2-benzènedisulfonamide;

le N-[(4,6-diméthoxy-1,3,5-triazine-2-yl)aminocarbonyl]-2,6-bis(méthylthio)benzènesulfonamide;

le 2-(azétidine-1-ylsulfonyl)-N-[(4,6-diméthoxy-1,3,5-triazine-2-yl)amionocarbonyl]benzène-sulfonamide; ou

le 2-(azétidine-1-ylsulfonyl)-N-[(4-méthoxy-6-méthylpyrimidine-2-yl)-aminocarbonyl]benzène-sulfonamide;

et un sel utilisable en agriculture de l'un quelconque de ceux-ci.

7. Une composition utilisable en agriculture pour lutter contre la croissance de végétation indésirable, comprenant une quantité efficace d'un composé de formule (I) tel que défini dans l'une quelconque des revendications 1 à 6, et l'un au moins des ingrédients suivants: agent tensioactif, diluant solide ou diluant liquide.

8. Un procédé pour lutter contre la croissance de végétation indésirable, qui consiste à appliquer au lieu à protéger une quantité efficace d'un composé de formule (I) tel que défini dans l'une quelconque des revendications 1 à 6.

9. Un procédé pour réguler la croissance de plantes, qui consiste à appliquer au lieu où se trouvent ces plantes une quantité efficace mais sensiblement non phytotoxique d'un régulateur de croissance des plantes choisi parmi les composés de formule (I) tels que définis dans l'une quelconque des revendications 1 à 6.